# EUROPEAN PATENT SPECIFICATION

(11) **EP 4 200 015 B1**
(45) Date of publication and mention of the grant of the patent: **29.04.2026**
(21) Application number: 21769573.3
(22) Date of filing: 18.08.2021
(51) Int. Cl.: A61P 1/16, C07D 471/04, C07D 519/00, A61K 31/437

(54) **SUBSTITUTED HETEROARYL COMPOUNDS USEFUL AS INHIBITORS OF TLR9**
SUBSTITUIERTE HETEROARYLVERBINDUNGEN ALS TLR9-HEMMER
COMPOSÉS HÉTÉROARYLES SUBSTITUÉS UTILES EN TANT QU'INHIBITEURS DE TLR9

(30) Priority: 19.08.2020 US 202063067389 P
(43) Date of publication of application: 28.06.2023
(73) Proprietor: Bristol-Myers Squibb Company, Princeton, NJ 08543 (US)
(72) Inventor: LIU, Chunjian, Princeton, New Jersey 08543 (US); REGUEIRO-REN, Alicia, Princeton, New Jersey 08543 (US)
(74) Representative: Mathys & Squire
(86) International application number: PCT/US2021/046475
(87) International publication number: WO 2022/040293

(56) References cited:
- WO-A1-2013/092467
- WO-A1-2019/125977
- WO-A1-2019/126082
- WO-A1-2019/126253

## Description

The present invention generally relates to substituted heteroaryl compounds useful as inhibitors of signaling through Toll-like receptor 9 (TLR9). Provided herein are substituted heteroaryl compounds, compositions comprising such compounds, and methods of their use. The invention further pertains to pharmaceutical compositions containing at least one compound according to the invention that are useful for the treatment of conditions related to TLR9 modulation, such as inflammatory and autoimmune diseases, and methods of inhibiting the activity of TLR9 in a mammal.

Toll-like receptors (TLRs) are transmembrane proteins having the ability to initiate an inflammatory response upon recognition of pattern-associated molecular patterns (PAMPs) or microbe-associated molecular patterns (MAMPs). A total of 10 human TLRs have been identified and can be located in the cell surface or, as in the case of TLR7, 8 and 9, in the endolysosomes. TLR9 recognizes unmethylated singlestranded DNA containing cytosine-phosphate-guanine (CpG) motifs that are typically found in bacterial and mitochondrial DNA (mtDNA). TLR9 may contribute to fibrogenesis by promoting inflammation via the MyD88-dependent signalling pathway that ultimately mediates activation of IL-6, IFN-α, IL-1β, and TNF-α among others cytokines. (Barton GM, Kagan JC (2009) Nat. Rev. Immunol. 9(8), 535-42; Li X, Jiang S, Tapping RI (2010) Cytokine 49(1), 1-9). International patent publications WO 2019/125977 A1 and WO 2019/126082 A1 describe 4-azaindole compounds and 6-azaindole compounds, respectively, that are useful as inhibitors of signaling through TLR7, TLR8 or TLR9. International patent publication No. WO 2019/126253 A1 describes aryl and heteroaryl substituted indole compounds that are useful as inhibitors of signaling through TLR7, TLR8 or TLR9.

TLR9 levels are higher in lung biopsies of rapid idiopathic pulmonary fibrosis (IPF) progressors than in the healthy or stable IPF progressors (Sci. Transl. Med. 2010, 2(57):57ra82). Circulating mtDNA, the ligand for TLR9 has recently been identified as a mechanism-based prognostic biomarker of IPF (Am J. Resp. and Crit. Care Med. 2017, 196(12), 1502). In addition, it has been observed that TLR9 is upregulated in human and murine non-alcoholic steatohepatitis (NASH) (Clin. Sci. 2017, 131(16), 2145), while hepatocyte mitochondrial DNA drives NASH via activation of TLR9 (J. Clin. Inv. 2016, 126(3), 859. Accordingly, inhibitors/antagonists of TLR9 are predicted to have efficacy as novel therapeutic agents to treat fibrotic diseases.

TLR9 inhibition has been recognized as a potential route to therapies for fibrotic diseases including idiopathic pulmonary fibrosis (Trujillo et al. Sci. Transl. Med. 2010, 2(57):57ra82; Yoshizaki et al. Ann Rheum Dis. 2016 Oct;75(10):1858-65), non-alcoholic steatohepatitis (Garcia-Martinez et al. J Clin Invest 2016, 126: 859-864; Gabele et al. Biochem Biophys Res Commun. 2008;376:271-276), hepatic injury (Shaker et al. Biochem Pharmacol. 2016. 112:90-101; Hoeque et al. J. Immun. 2013, 190:4297-304), and scleroderma (systemic sclerosis or SSc) (Yoshizaki et al. Ann Rheum Dis . 2016 Oct;75(10):1858-65); as well as heart failure (Oka et al. Nature 485, pages251-255(2012)), and hypertension (McCarthy et al. Cardiovascular Research, 2015, Pages 119-130).

There remains a need for compounds useful as inhibitors of TLR9. Additionally, there remains a need for compounds useful as inhibitors of TLR9 that have selectivity over TLR7 or TLR8.

In view of the conditions that may benefit by treatment involving modulation of Toll-like receptors, it is immediately apparent that new compounds capable of inhibiting TLR9 and methods of using these compounds could provide substantial therapeutic benefits to a wide variety of patients.

Applicants have found potent compounds that have activity as TLR9 inhibitors. Further, applicants have found compounds that have activity as TLR9 inhibitors and are selective over TLR7 or TLR8. These compounds are provided to be useful as pharmaceuticals with desirable stability, bioavailability, therapeutic index, and toxicity values that are important to their drugability.

### SUMMARY

The present disclosure relates to a new class of substituted heteroaryl compounds found to be effective inhibitors of signaling through TLR9. These compounds are provided to be useful as pharmaceuticals with desirable stability, bioavailability, therapeutic index, and toxicity values that are important to their drugability. The invention is defined in the claims.

The present disclosure relates to compounds of Formula (I) that are useful as inhibitors of signaling through Toll-like receptor 9 and are useful for the treatment of fibrotic diseases, or stereoisomers, N-oxides, tautomers, pharmaceutically acceptable salts, solvates or prodrugs thereof.

The present invention provides a compound of Formula (Ia-2) or Formula (IIb) or stereoisomers, tautomer, solvates or salts thereof, in accordance with the appended claims.

The present invention also provides a pharmaceutical composition comprising a pharmaceutically acceptable carrier or diluent and one or more of the compounds of the present invention or stereoisomers, tautomer, salts, or solvates thereof.

The present invention also provides a compound, or stereoisomers, tautomers, solvates or pharmaceutically-acceptable salts thereof of the present invention, or a pharmaceutical composition of the present invention, for use in treating pathological fibrosis.

The present invention also provides a compound, or stereoisomers, tautomers, solvates or pharmaceutically-acceptable salts thereof of the present invention, or a pharmaceutical composition of the present invention, for use in treating nonalcoholic steatohepatitis (NASH), non-alcoholic fatty liver disease (NAFLD), chronic kidney disease, diabetic kidney disease, primary sclerosing cholangitis (PSC), or primary biliary cirrhosis (PBC).

The present invention also provides a compound, or stereoisomers, tautomers, solvates or pharmaceutically-acceptable salts thereof of the present invention, or a pharmaceutical composition of the present invention, for use in treating idiopathic pulmonary fibrosis (IPF).

These and other features of the invention will be set forth in expanded form as the disclosure continues.

### DETAILED DESCRIPTION

The present disclosure relates to compounds of Formula (I) and Formula (II): 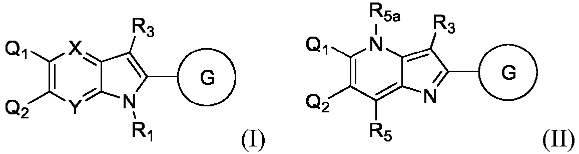 or stereoisomers, tautomer, solvates or salts thereof, wherein:
one of X and Y is N and the other of X and Y is CR₅;
one of Q₁ and Q₂ is A and the other of Q₁ and Q₂ is R₅;
G is:
   (i) phenyl substituted with 1 to 3 substituents independently selected from F, Cl, Br, -CN, C₁₋₂ alkoxy, C₁₋₂ fluoroalkoxy, C₃₋₄ cycloalkyl -C(O)NR_{y}R_{y}, -S(O)₂CH₃, -S(O)₂(phenyl), -S(O)₂(cyclopropyl), -S(O)₂NRₓRₓ, and -S(O)(NH)NRₓRₓ;
   (ii) 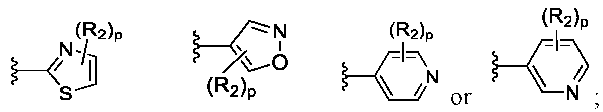
   (iii) 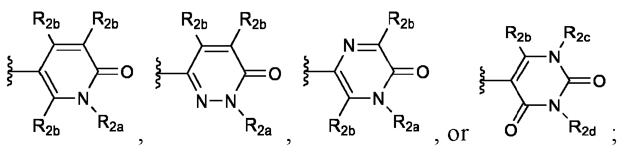
   (iv) 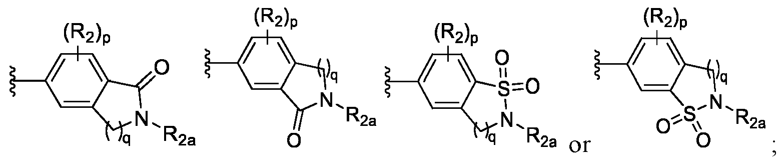
   (v) a 9-membered heterocyclic ring selected from: 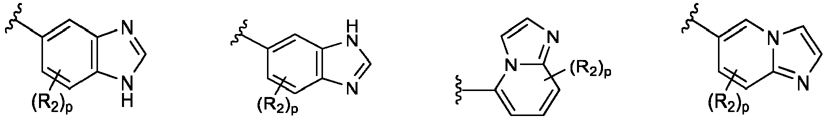 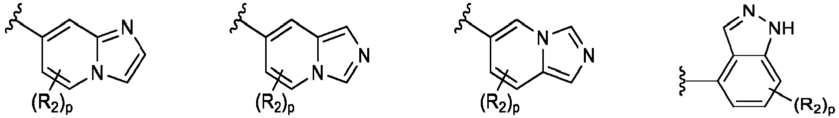 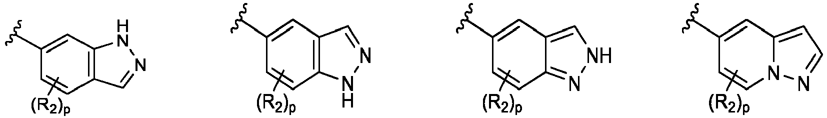 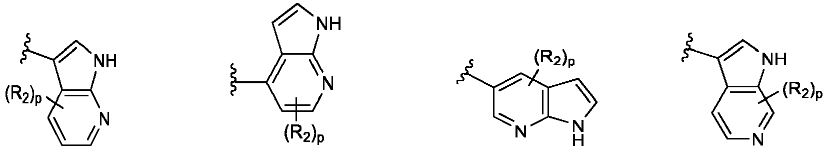 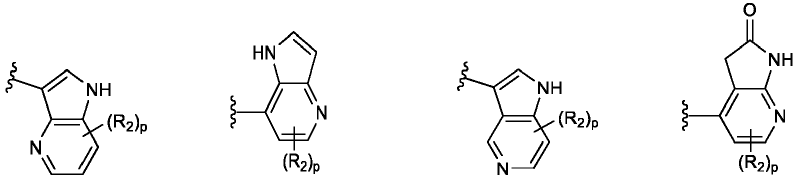 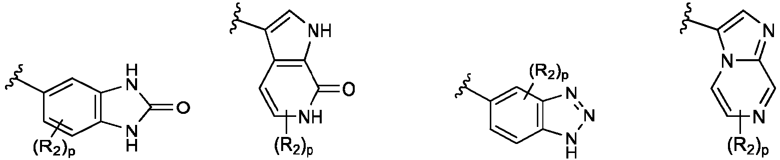 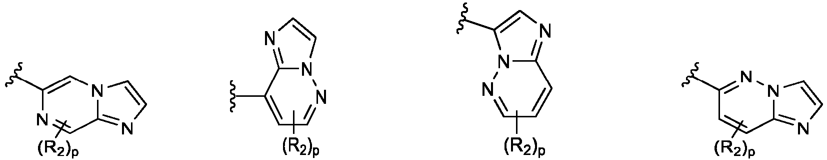 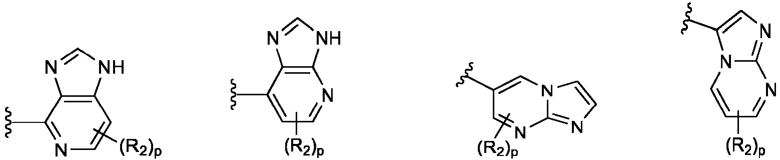 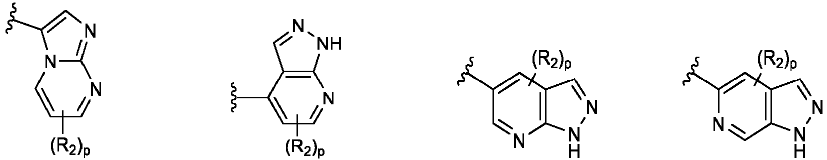 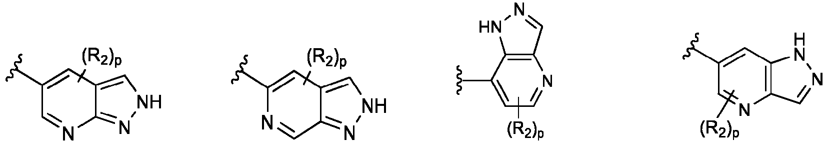 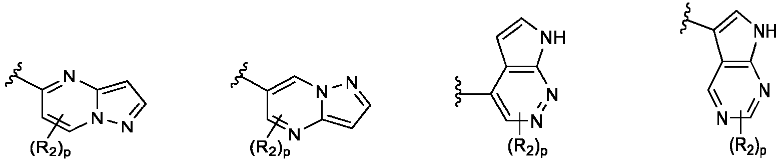 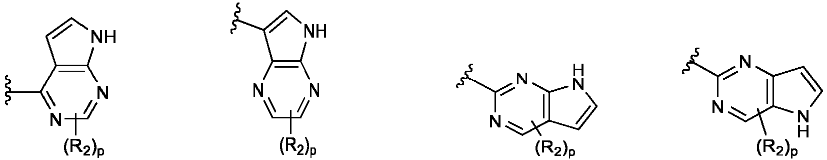 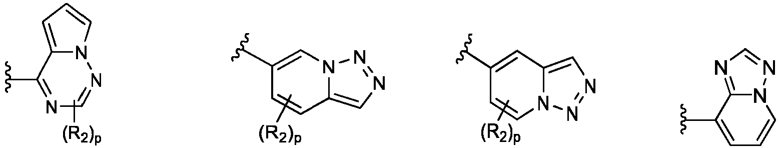 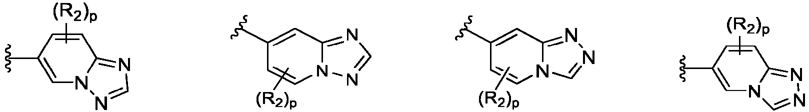 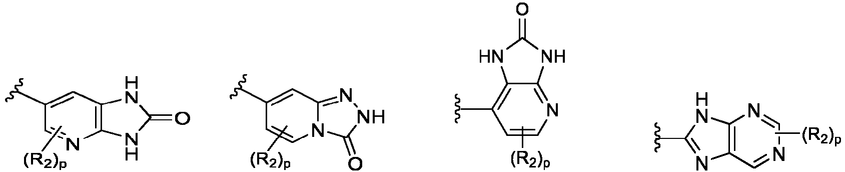 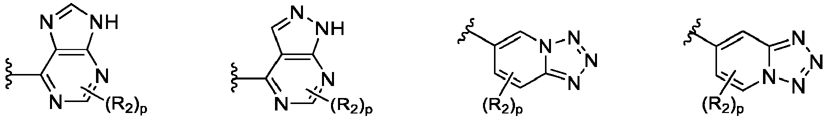 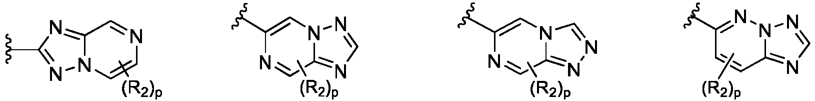 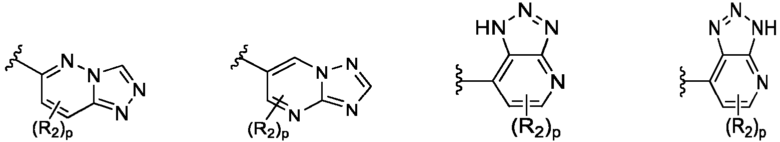 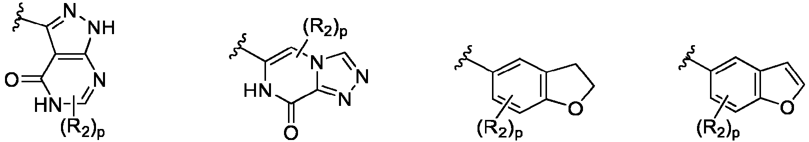 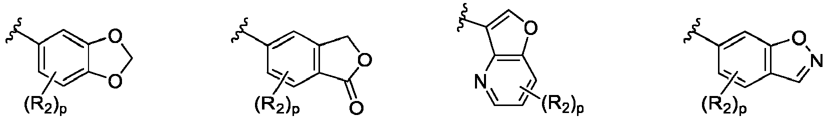 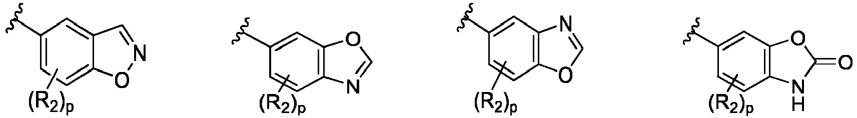 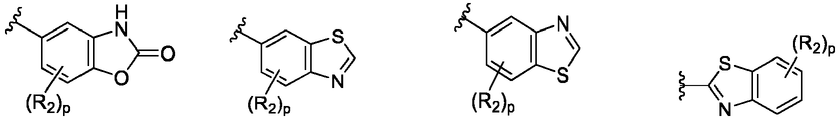 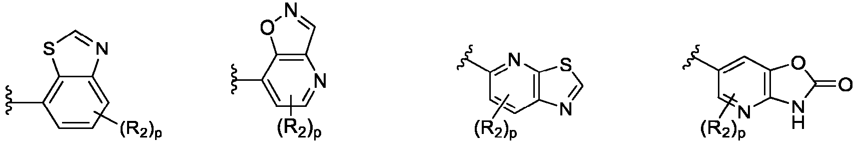 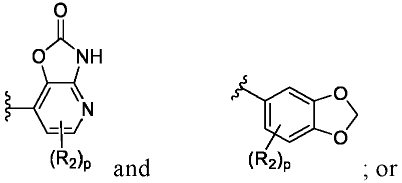
   (vi) 10-membered heterocyclic ring selected from: 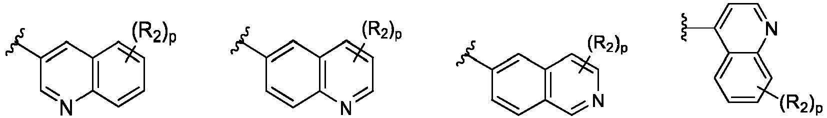 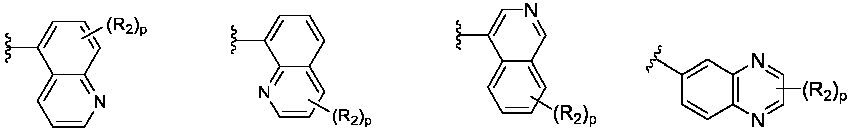 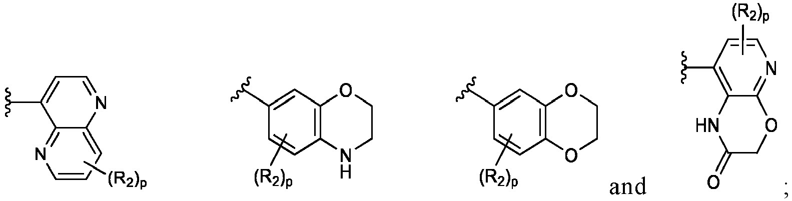
A is piperidinyl, phenyl, pyridinyl, pyrimidinyl, 6-azabicyclo[3.2.1]octanyl, or azabicyclo[3.2.1]octanyl, each substituted with -L-R₄ and zero to 1 R_{4b};
L is a bond, -CRₓRₓ- or -C(O)(CRₓRₓ)₀₋₂-;
R₁ is hydrogen, C₁₋₃ alkyl, C₁₋₂ fluoroalkyl, or C₃₋₄ cycloalkyl;
each R₂ is independently halo, -CN, -OH, -NO₂, C₁₋₄ alkyl, C₁₋₂ fluoroalkyl, C₁₋₂ cyanoalkyl, C₁₋₃ hydroxyalkyl, C₁₋₃ aminoalkyl, -O(CH₂)₁₋₂OH, -(CH₂)₀₋₄O(C₁₋₄ alkyl), C₁₋₃ fluoroalkoxy, -(CH₂)₁₋₄O(C₁₋₃ alkyl), -O(CH₂)₁₋₂OC(O)(C₁₋₃ alkyl), -O(CH₂)₁₋₂NRₓRₓ, -C(O)O(C₁₋₃ alkyl), -(CH₂)₀₋₂C(O)NR_{y}R_{y}, -C(O)NRₓ(C₁₋₅ hydroxyalkyl), -C(O)NRₓ(C₂₋₆ alkoxyalkyl), -C(O)NRₓ(C₃₋₆ cycloalkyl), -NR_{y}R_{y}, -NR_{y}(C₁₋₃ fluoroalkyl), -NR_{y}(C₁₋₄ hydroxyalkyl), -NRₓCH₂(phenyl), -NRₓS(O)₂(C₃₋₆ cycloalkyl), -NRₓC(O)(C₁₋₃ alkyl), -NRₓCH₂(C₃₋₆ cycloalkyl), -S(O)₂(C₁₋₃ alkyl), -S(O)₂N(C₁₋₃ alkyl)₂, -S(O)(NH)N(C₁₋₃ alkyl)₂, -(CH₂)₀₋₂(C₃₋₆ cycloalkyl), -(CH₂)₀₋₂(phenyl), morpholinyl, dioxothiomorpholinyl, dimethyl pyrazolyl, methylpiperidinyl, methylpiperazinyl, amino-oxadiazolyl, imidazolyl, triazolyl, or -C(O)(thiazolyl);
R₂ₐ is C₁₋₆ alkyl, C₁₋₃ fluoroalkyl, C₁₋₆ hydroxyalkyl, C₁₋₃ aminoalkyl, -(CH₂)₀₋₄O(C₁₋₃ alkyl), C₃₋₆ cycloalkyl, -(CH₂)₁₋₃C(O)NRₓRₓ, -CH₂(C₃₋₆ cycloalkyl), -CH₂(phenyl), tetrahydrofuranyl, tetrahydropyranyl, or phenyl;
each R_{2b} is independently hydrogen, halo, -CN, -NRₓRₓ, C₁₋₆ alkyl, C₁₋₃ fluoroalkyl, C₁₋₃ hydroxyalkyl, C₁₋₃ fluoroalkoxy, -(CH₂)₀₋₂O(C₁₋₃ alkyl), -(CH₂)₀₋₃C(O)NRₓRₓ, -(CH₂)₁₋₃(C₃₋₆ cycloalkyl), -C(O)O(C₁₋₃ alkyl), -C(O)NRₓ(C₁₋₃ alkyl), -CRₓ=CRₓRₓ, or -CRₓ=CH(C₃₋₆ cycloalkyl);
R_{2c} is R₂ₐ or R_{2b};
R₂ₐ is R₂ₐ or R_{2b}; provided that one of R_{2c} and R_{2d} is R₂ₐ, and the other of R_{2c} and R_{2d} is R_{2b};
R₃ is hydrogen, F, Cl, C₁₋₃ alkyl, C₁₋₂ fluoroalkyl, or C₃₋₄ cycloalkyl;
R₄ is:
   (i) -N(CH₃)₂;
   (ii) pyrrolidinyl, piperidinyl, piperazinyl, pyridinyl, azaspiro[3.3]heptanyl, azabicyclo[3 .2.1]octanyl, or diazabicyclo[3.2.1]octanyl, each substituted with zero to 3 R₄ₐ and zero to 2 -CH₃; or
   (iii) 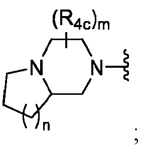
each R₄ₐ is independently -OH, C₁₋₆ alkyl, C₁₋₃ fluoroalkyl, C₁₋₆ hydroxyalkyl, C₃₋₆ cycloalkyl, -CH₂(C₃₋₆ cycloalkyl), -C(O)(C₁₋₄ alkyl), -C(O)(C₃₋₆ cycloalkyl), -C(O)(phenyl), -C(O)CH₂(C₃₋₆ cycloalkyl), -C(O)CH₂(phenyl), or -C(O)O(C₁₋₄ alkyl);
R_{4b} is F, Cl, or -CH₃;
each R_{4c} is independently C₁₋₆ alkyl, C₁₋₃ fluoroalkyl, -CH₂(C₃₋₆ cycloalkyl), -C(O)(C₁₋₄ alkyl), -C(O)(phenyl), -C(O)CH₂(phenyl), -C(O)OCH₂CH₃, or C₃₋₆ cycloalkyl;
each R₅ is independently hydrogen, F, Cl, C₁₋₂ alkyl, C₁₋₂ fluoroalkyl, or cyclopropyl;
R₅ₐ is hydrogen, C₁₋₂ alkyl, C₁₋₂ fluoroalkyl, or cyclopropyl;
each Rₓ is independently hydrogen or -CH₃;
each R_{y} is independently hydrogen or C₁₋₆ alkyl;
m is zero, 1, or 2;
n is zero, 1, or 2;
p is zero, 1, 2, 3, or 4; and
q is 1 or 2.

The present disclosure also relates to compounds of Formula (I) and Formula (II): 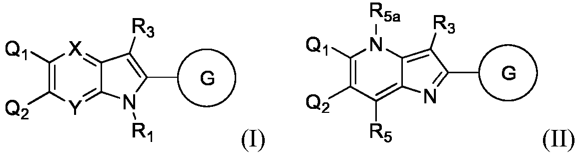 or a salt thereof, wherein:
one of X and Y is N and the other of X and Y is CR₅;
one of Q₁ and Q₂ is A and the other of Q₁ and Q₂ is R₅;
G is:
   (i) phenyl substituted with 1 to 3 substituents independently selected from F, Cl, Br, C₁₋₂ alkoxy, C₁₋₂ fluoroalkoxy, C₃₋₄ cycloalkyl -C(O)NR_{y}R_{y}, -S(O)₂CH₃, -S(O)₂(phenyl), -S(O)₂NRₓRₓ, and -S(O)(NH)NRₓRₓ;
   (ii) 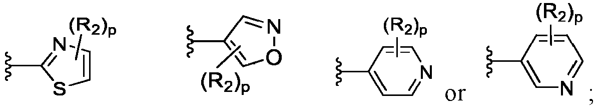
   (iii) 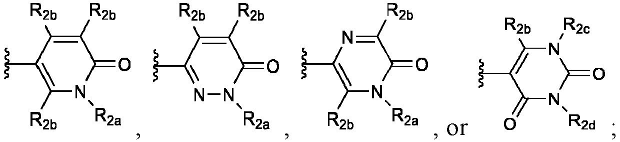
   (iv) 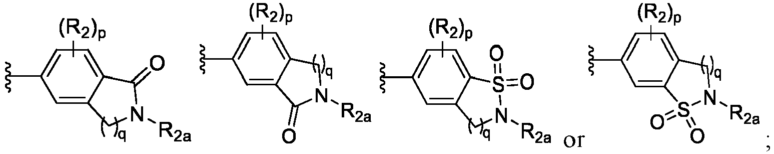
   (v) a 9-membered heterocyclic ring selected from: 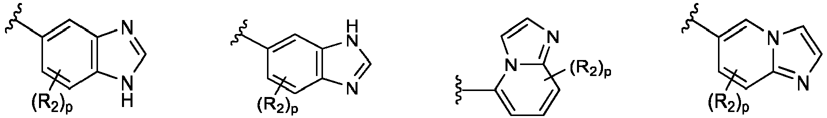 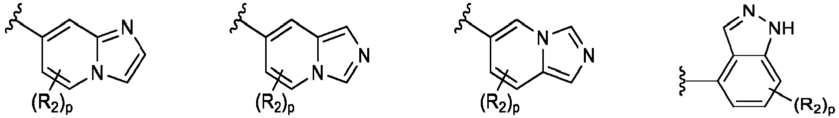 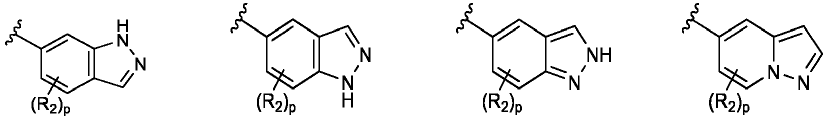 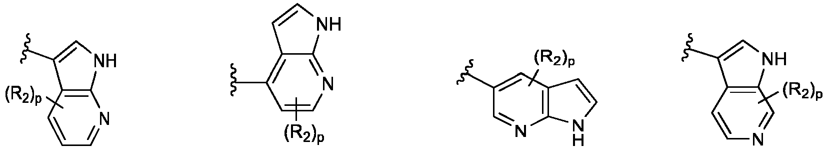 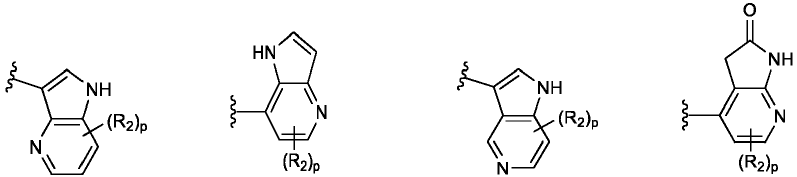 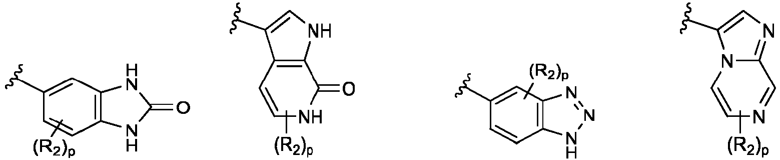 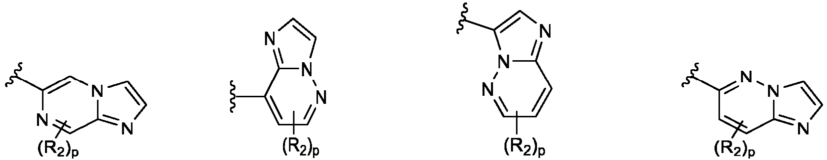 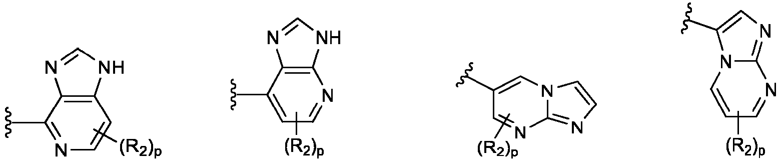 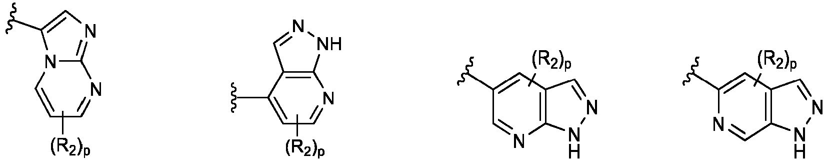 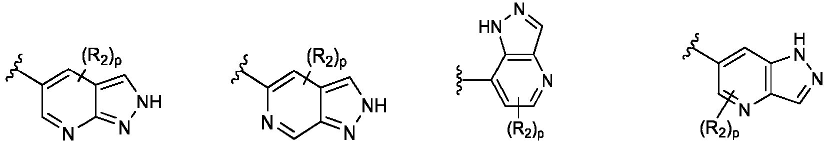 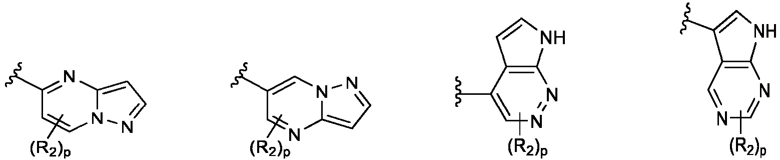 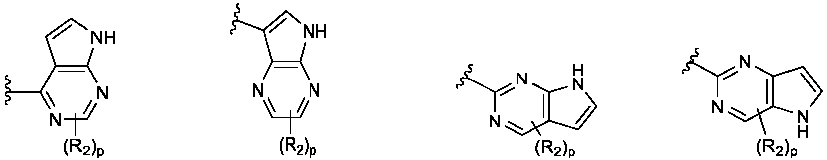 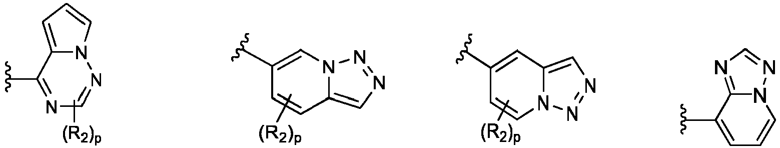 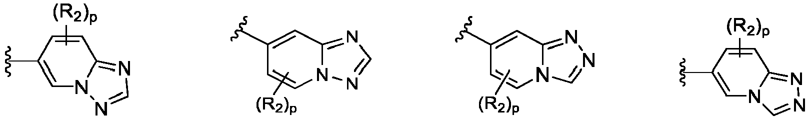 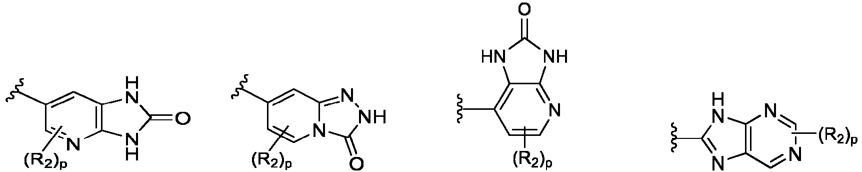 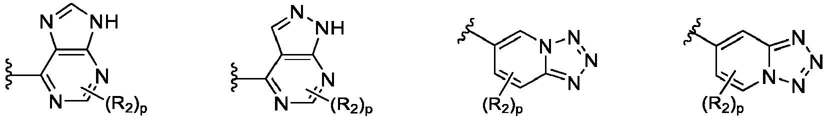 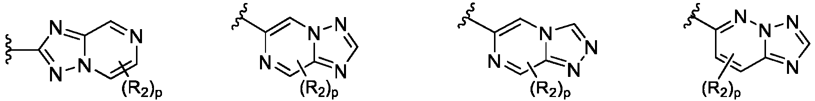 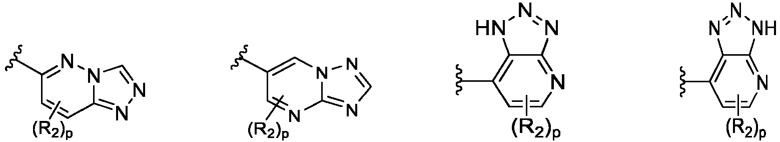 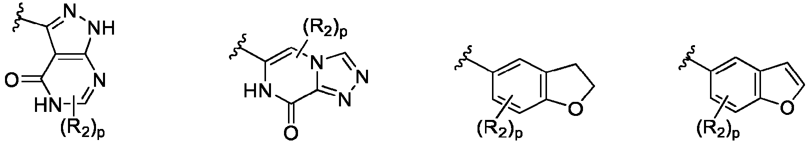 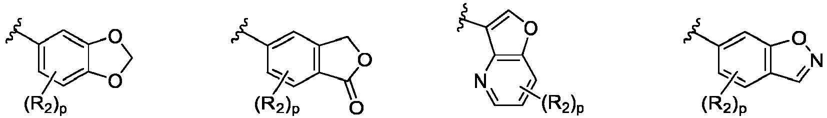 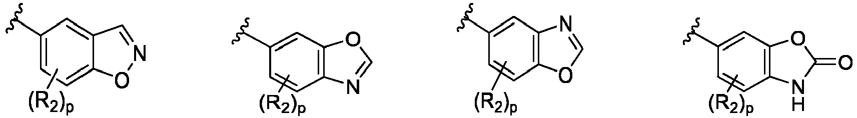 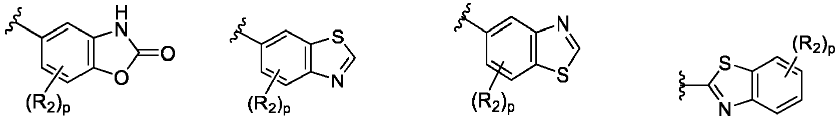 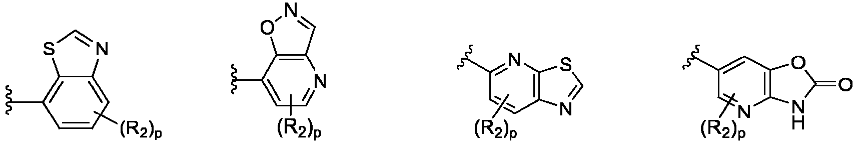 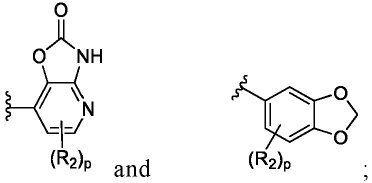 or
   (vi) 10-membered heterocyclic ring selected from: 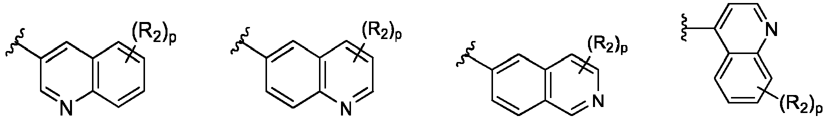 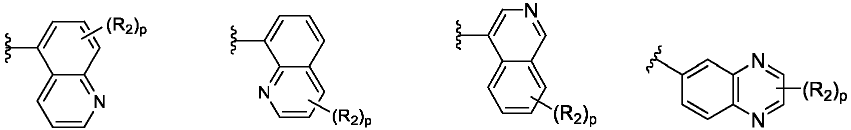 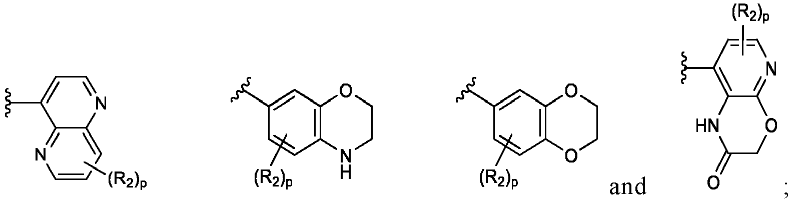
A is piperidinyl, phenyl, pyridinyl, pyrimidinyl, 6-azabicyclo[3.2.1]octanyl, or azabicyclo[3.2.1]octanyl, each substituted with -L-R₄ and zero to 1 R_{4b};
L is a bond, -CRₓRₓ- or -C(O)(CRₓRₓ)₀₋₂-;
R₁ is hydrogen, C₁₋₃ alkyl, C₁₋₂ fluoroalkyl, or C₃₋₄ cycloalkyl;
each R₂ is independently halo, -CN, -OH, -NO₂, C₁₋₄ alkyl, C₁₋₂ fluoroalkyl, C₁₋₂ cyanoalkyl, C₁₋₃ hydroxyalkyl, C₁₋₃ aminoalkyl, -O(CH₂)₁₋₂OH, -(CH₂)₀₋₄O(C₁₋₄ alkyl), C₁₋₃ fluoroalkoxy, -(CH₂)₁₋₄O(C₁₋₃ alkyl), -O(CH₂)₁₋₂OC(O)(C₁₋₃ alkyl), -O(CH₂)₁₋₂NRₓRₓ, -C(O)O(C₁₋₃ alkyl), -(CH₂)₀₋₂C(O)NR_{y}R_{y}, -C(O)NRₓ(C₁₋₅ hydroxyalkyl), -C(O)NRₓ(C₂₋₆ alkoxyalkyl), -C(O)NRₓ(C₃₋₆ cycloalkyl), -NR_{y}R_{y}, -NR_{y}(C₁₋₃ fluoroalkyl), -NR_{y}(C₁₋₄ hydroxyalkyl), -NRₓCH₂(phenyl), -NRₓS(O)₂(C₃₋₆ cycloalkyl), -NRₓC(O)(C₁₋₃ alkyl), -NRₓCH₂(C₃₋₆ cycloalkyl), -S(O)₂(C₁₋₃ alkyl), -S(O)₂N(C₁₋₃ alkyl)₂, -S(O)(NH)N(C₁₋₃ alkyl)₂, -(CH₂)₀₋₂(C₃₋₆ cycloalkyl), -(CH₂)₀₋₂(phenyl), morpholinyl, dioxothiomorpholinyl, dimethyl pyrazolyl, methylpiperidinyl, methylpiperazinyl, amino-oxadiazolyl, imidazolyl, triazolyl, or -C(O)(thiazolyl);
R₂ₐ is C₁₋₆ alkyl, C₁₋₃ fluoroalkyl, C₁₋₆ hydroxyalkyl, C₁₋₃ aminoalkyl, -(CH₂)₀₋₄O(C₁₋₃ alkyl), C₃₋₆ cycloalkyl, -(CH₂)₁₋₃C(O)NRₓRₓ, -CH₂(C₃₋₆ cycloalkyl), -CH₂(phenyl), tetrahydrofuranyl, tetrahydropyranyl, or phenyl;
each R_{2b} is independently hydrogen, halo, -CN, -NRₓRₓ, C₁₋₆ alkyl, C₁₋₃ fluoroalkyl, C₁₋₃ hydroxyalkyl, C₁₋₃ fluoroalkoxy, -(CH₂)₀₋₂O(C₁₋₃ alkyl), -(CH₂)₀₋₃C(O)NRₓRₓ, -(CH₂)₁₋₃(C₃₋₆ cycloalkyl), -C(O)O(C₁₋₃ alkyl), -C(O)NRₓ(C₁₋₃ alkyl), -CRₓ=CRₓRₓ, or -CRₓ=CH(C₃₋₆ cycloalkyl);
R_{2c} is R₂ₐ or R_{2b};
R_{2d} is R₂ₐ or R_{2b}; provided that one of R_{2c} and R_{2d} is R₂ₐ, and the other of R_{2c} and R_{2d} is R_{2b};
R₃ is hydrogen, F, Cl, C₁₋₃ alkyl, C₁₋₂ fluoroalkyl, or C₃₋₄ cycloalkyl;
R₄ is:
   (i) -N(CH₃)₂;
   (ii) pyrrolidinyl, piperidinyl, piperazinyl, pyridinyl, azaspiro[3.3]heptanyl, or azabicyclo[3.2.1]octanyl, each substituted with zero to 2 R₄ₐ; or
   (iii) 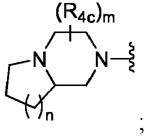
each R₄ₐ is independently C₁₋₆ alkyl, C₁₋₃ fluoroalkyl, C₃₋₆ cycloalkyl, -CH₂(C₃₋₆ cycloalkyl), -C(O)(C₁₋₄ alkyl), -C(O)(C₃₋₆ cycloalkyl), -C(O)(phenyl), -C(O)CH₂(C₃₋₆ cycloalkyl), -C(O)CH₂(phenyl), or -C(O)O(C₁₋₄ alkyl);
R_{4b} is F, Cl, or -CH₃;
each R_{4c} is independently C₁₋₆ alkyl, C₁₋₃ fluoroalkyl, -CH₂(C₃₋₆ cycloalkyl), -C(O)(C₁₋₄ alkyl), -C(O)(phenyl), -C(O)CH₂(phenyl), -C(O)OCH₂CH₃, or C₃₋₆ cycloalkyl;
each R₅ is independently hydrogen, F, Cl, C₁₋₂ alkyl, C₁₋₂ fluoroalkyl, or cyclopropyl;
R₅ₐ is hydrogen, C₁₋₂ alkyl, C₁₋₂ fluoroalkyl, or cyclopropyl;
each Rₓ is independently hydrogen or -CH₃;
each R_{y} is independently hydrogen or C₁₋₆ alkyl;
m is zero, 1, or 2;
n is zero, 1, or 2;
p is zero, 1, 2, 3, or 4; and
q is 1 or 2.

Compounds of Formula (I) or a salt thereof or stereoisomers, tautomer, solvates or salts thereof are disclosed wherein X is N and Y is CR₅. Compounds of this instance (which are not claimed as such) have the structure of Formula (Ia):

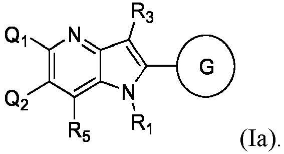

Compounds of Formula (I) or stereoisomers, tautomer, solvates or salts thereof are also disclosed wherein X is CR₅ and Y is N. Compounds of this instance (which are not claimed as such) have the structure of Formula (Ib):

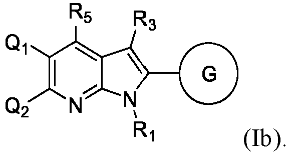

In one instance (which is not claimed as such), a compound of Formula (I) or stereoisomers, tautomer, solvates or salts thereof is disclosed wherein X is N; Y is CR₅; Q₁ is A; and Q₂ is R₅. Such compounds have the structure of Formula (Ia-1):

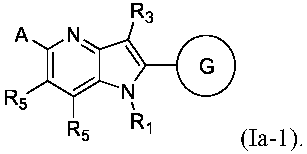

In one aspect, the present invention provides a compound of Formula (I) or stereoisomers, tautomer, solvates or salts thereof wherein X is N; Y is CR₅; Q₁ is R₅; and Q₂ is A. Such compounds have the structure of Formula (Ia-2): 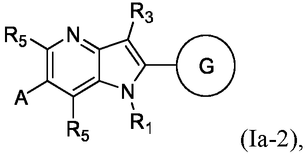 wherein G, A, R₁, R₃ and R₅ are each as defined in the appended claims.

Thus, in one embodiment the present invention provides a compound of Formula (Ia-2) or stereoisomers, tautomer, solvates or salts thereof.

Compounds of Formula (I) or a salt thereof or stereoisomers, tautomer, solvates or salts thereof are also disclosed wherein X is CR₅; Y is N; Q₁ is A; and Q₂ is R₅. Compounds of this instance (which are not claimed as such) have the structure of Formula (Ib-1):

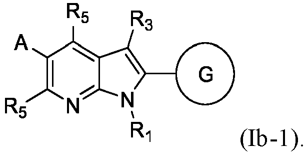

Compounds of Formula (I) or a salt thereof or stereoisomers, tautomer, solvates or salts thereof are also disclosed wherein X is CR₅; Y is N; Q₁ is R₅; and Q₂ is A. Compounds of this instance (which are not claimed as such) have the structure of Formula (Ib-2):

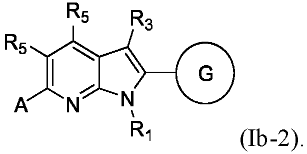

Compounds of Formula (II) or stereoisomers, tautomer, solvates or salts thereof are disclosed wherein Q₁ is A and Q₂ is R₅. Compounds of this instance (which are not claimed as such) have the structure of Formula (IIa):

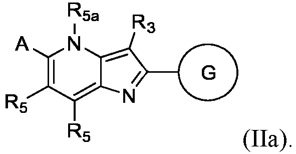

In one aspect, the present invention provides a compound of Formula (II) or stereoisomers, tautomer, solvates or salts thereof wherein Q₁ is R₅ and Q₂ is A. Such compounds have the structure of Formula (IIb): 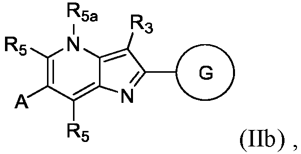 wherein G, A, R₃, R₅ and R₅ₐ are each as defined in the appended claims.

In one embodiment, a compound of Formula (I) or Formula (II), or stereoisomers, tautomer, solvates or salts thereof is provided wherein G is phenyl substituted with 1 to 3 substituents independently selected from F, Cl, Br, -CN, C₁₋₂ alkoxy, C₁₋₂ fluoroalkoxy, C₃₋₄ cycloalkyl -C(O)NR_{y}R_{y}, -S(O)₂CH₃, -S(O)₂(phenyl), -S(O)₂(cyclopropyl), -S(O)₂NRₓRₓ, and -S(O)(NH)NRₓRₓ. Included in this embodiment are compounds in which G is phenyl substituted with 1 to 2 substituents independently selected from F, -CN, -OCH₃, -S(O)₂CH₃, -S(O)₂(cyclopropyl), or -S(O)₂N(CH₃)₂. Also included in this embodiment are compounds in which G is: 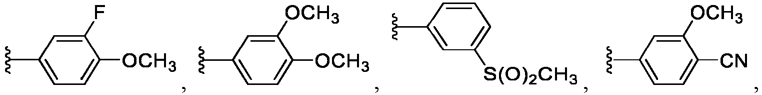 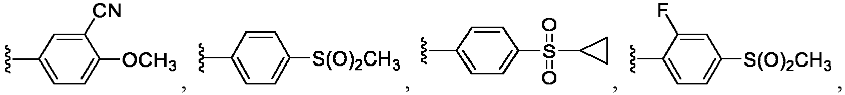 or

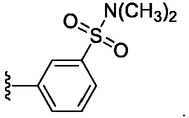

In one embodiment, a compound of Formula (I) or Formula (II), or stereoisomers, tautomer, solvates or salts thereof is provided wherein G is phenyl substituted with 1 to 2 substituents independently selected from F, -OCH₃, -S(O)₂CH₃, -S(O)₂N(CH₃)₂, and -S(O)(NH)N(CH₃)₂. Included in this embodiment are compounds in which G is phenyl substituted with 1 to 2 substituents independently selected from F, -OCH₃ and -S(O)₂CH₃. Also included in this embodiment are compounds in which G is:

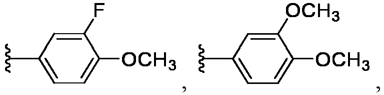

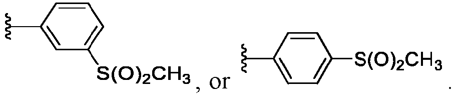

In one embodiment, a compound of Formula (I) or Formula (II), or stereoisomers, tautomer, solvates or salts thereof is provided wherein G is 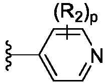 or 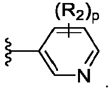 Included in this embodiment are compounds in which each R₂ is independently F, Cl, Br, -CN, -OH, -CH₃, -CH₂CH₃, -CF₃, -CH₂OH, -C(CH₃)₂OH, -CH₂NH₂, -OCH₃, -OCH₂CH₃, -OCH(CH₃)₂, -OCH₂CH₂OCH₃, -OCH₂CH₂N(CH₃)₂, -OCHF₂, -C(O)OCH₃, -C(O)NH₂, -C(O)NH(CH₂CH₃), -C(O)(thiazolyl), -NH₂, -NH(CH₃), -NH(CH₂CH₃), -N(CH₃)₂, -NHC(O)CH₃, -NHC(O)C(CH₃)₃, -NH(CH₂-cyclopropyl), cyclopropyl, methylpiperidinyl, methylpiperazinyl, amino-oxadiazolyl, imidazolyl, or triazolyl. Also included in this embodiment are compounds in which each R₂ is independently F, Cl, -CN, -CH₃, -OCH₃, -NH₂, or cyclopropyl. Additionally, included in this embodiment are compounds in which p is 2; one R₂ is -CH₃; and the other R₂ is F, Cl, -CN, -CH₃, -OCH₃, -NH₂, or cyclopropyl.

In a compound of Formula (I) or Formula (II), or stereoisomers, tautomer, solvates or salts thereof of the disclosure, G may be a 9-membered heterocyclic ring selected from: 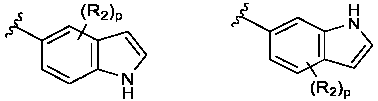 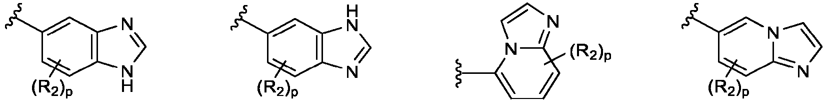 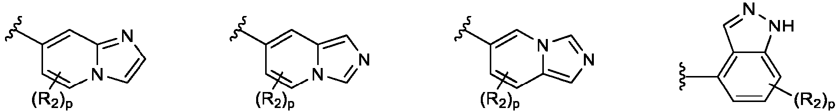 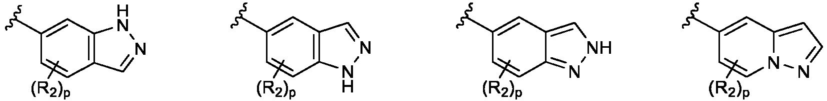 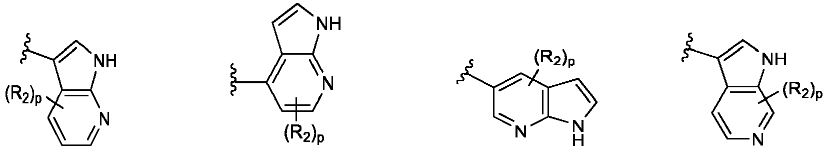 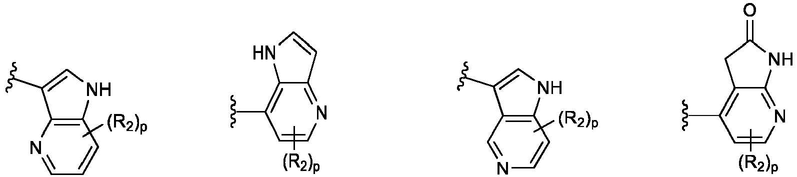 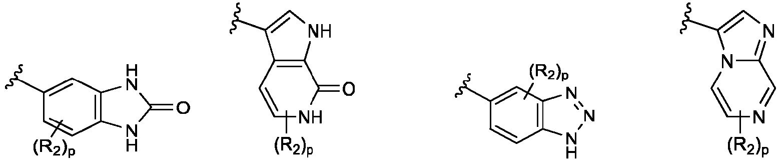 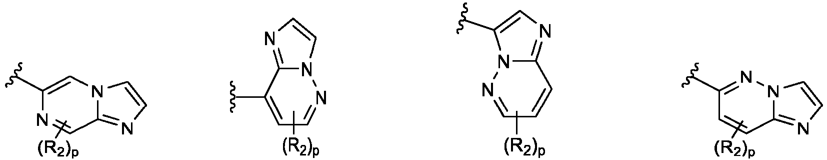 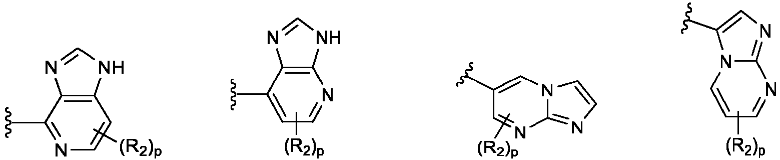 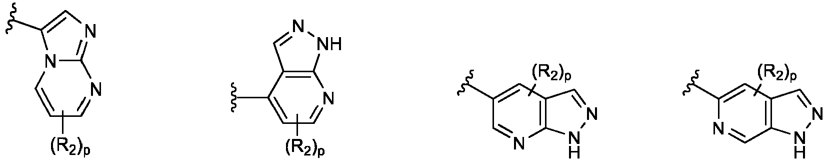 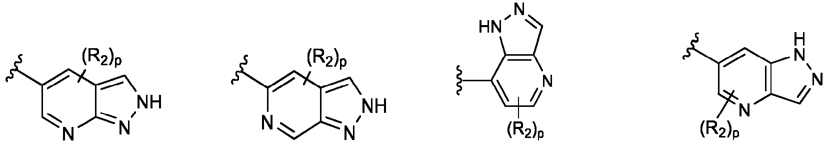 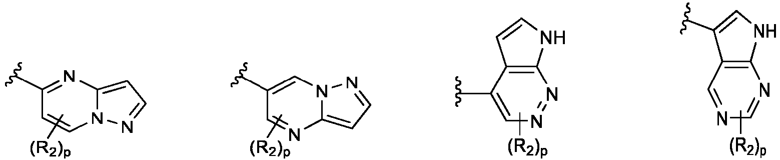 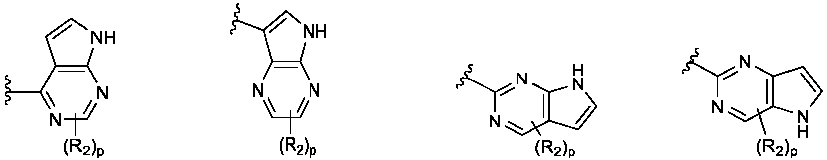 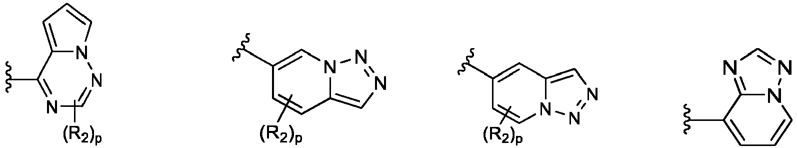 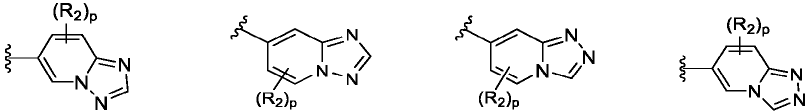 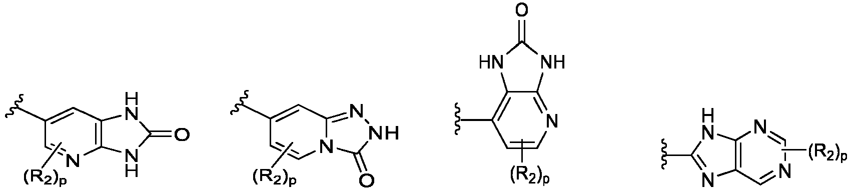 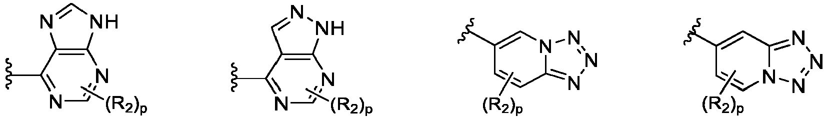 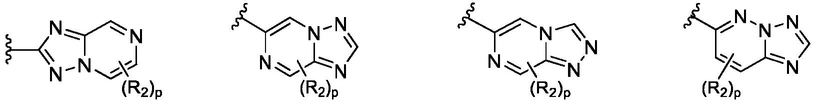 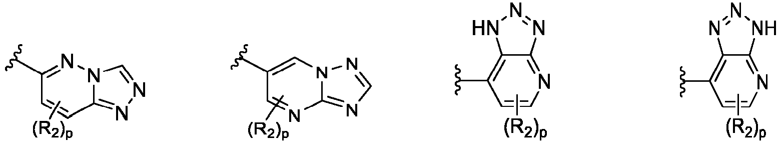 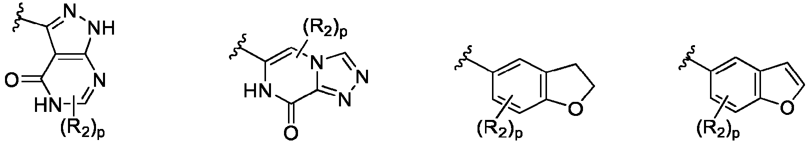 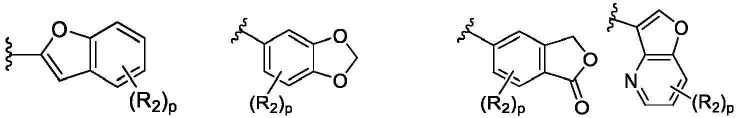 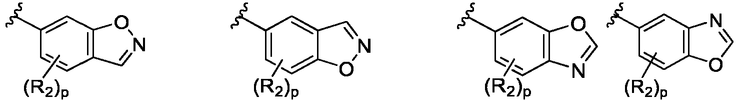 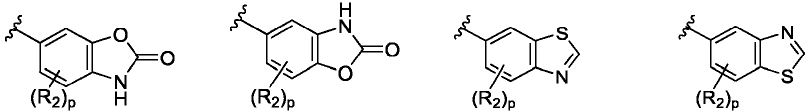 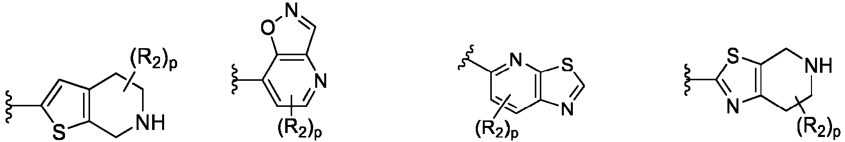 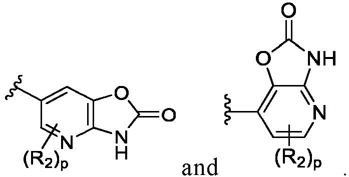 For example, G may be:

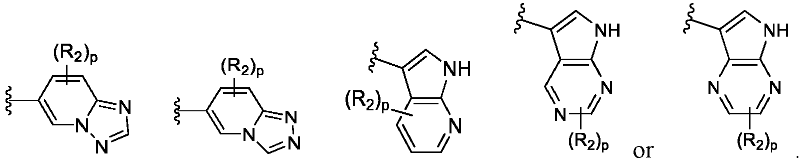

In a compound of Formula (I) or Formula (II), or stereoisomers, tautomer, solvates or salts thereof of the disclosure, G may be a 10-membered heterocyclic ring selected from: 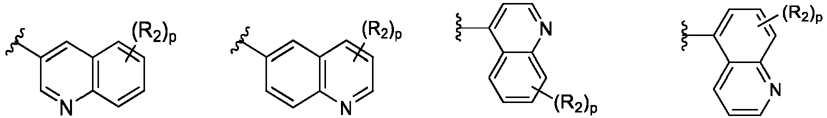 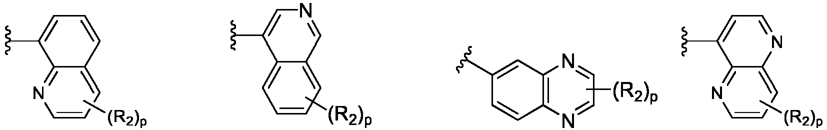 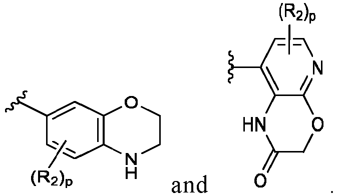 For example, G may be:

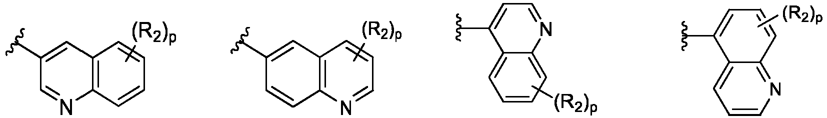

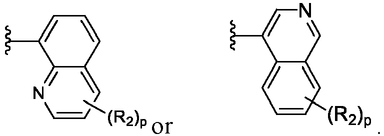

In a compound of Formula (I) or Formula (II), or stereoisomers, tautomer, solvates or salts thereof of the disclosure, G may be:
(i) phenyl substituted with 1 to 2 substituents independently selected from F, -CN, -OCH₃, -S(O)₂CH₃, -S(O)₂(cyclopropyl), or -S(O)₂N(CH₃)₂;
(ii) 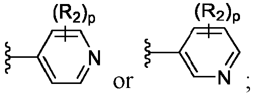
(iii) 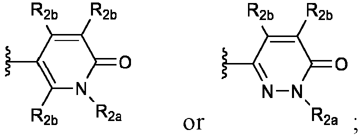 or
(iv) 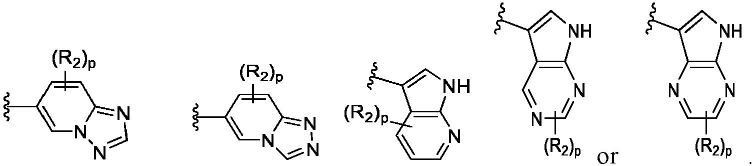 Included in this instance are compounds in which each R₂ is independently Cl, -CH₃, -CH₂CH₃, -CH₂OH, -CH₂CH₂OH, -CH₂CN, -OCH₃, -CH₂OCH₃, or -CH₂CH₂S(O)₂CH₃.

In a compound of Formula (I) or Formula (II), or stereoisomers, tautomer, solvates or salts thereof of the disclosure, G may be:
(i) phenyl substituted with 1 to 2 substituents independently selected from F, -OCH₃, -S(O)₂CH₃, -S(O)₂N(CH₃)₂, and -S(O)(NH)N(CH₃)₂;
(ii) 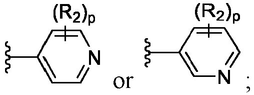
(iii) 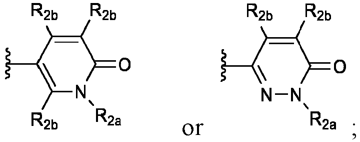 or
(iv) 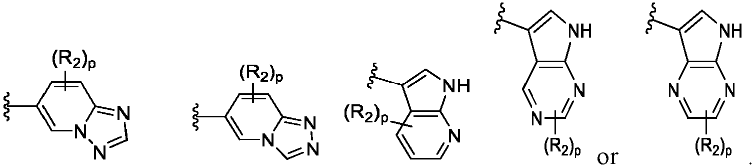 Included in this instance are compounds in which each R₂ is independently Cl, -CH₃, -CH₂CH₃, -CH₂OH, -CH₂CH₂OH, -CH₂CN, -OCH₃, -CH₂OCH₃, or -CH₂CH₂S(O)₂CH₃.

In a compound of Formula (I) or Formula (II), or stereoisomers, tautomer, solvates or salts thereof of the disclosure, p may be zero, 1, 2, or 3. Included in this instance are compounds in which p is 1 or 2.

In the compounds of the present invention, G is:
(i) phenyl substituted with 1 to 3 substituents independently selected from F, Cl, Br, -CN, C₁₋₂ alkoxy, C₁₋₂ fluoroalkoxy, C₃₋₄ cycloalkyl -C(O)NR_{y}R_{y}, -S(O)₂CH₃, -S(O)₂(phenyl), -S(O)₂(cyclopropyl), -S(O)₂NRₓRₓ, and -S(O)(NH)NRₓRₓ;
(ii) 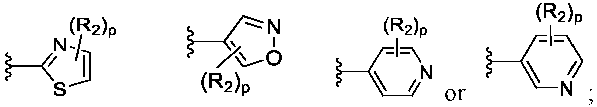
(iii) 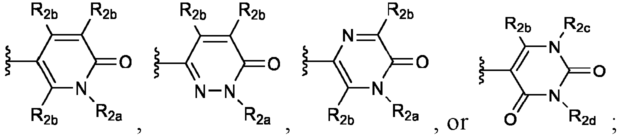
(iv) 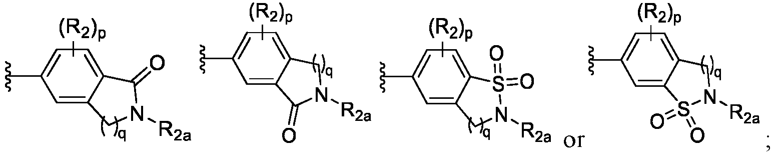
(v) a 9-membered heterocyclic ring selected from: 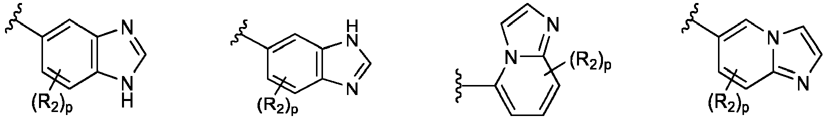 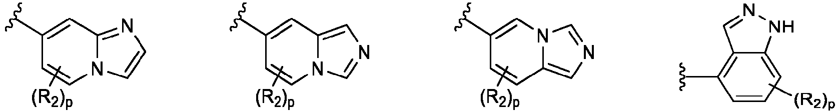 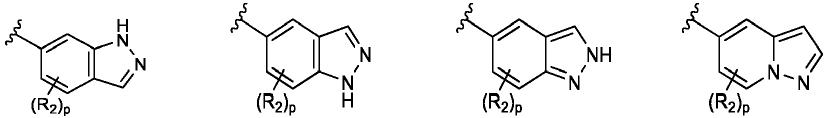 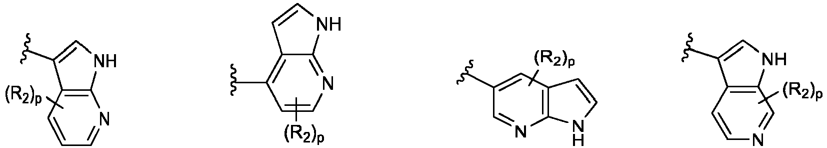 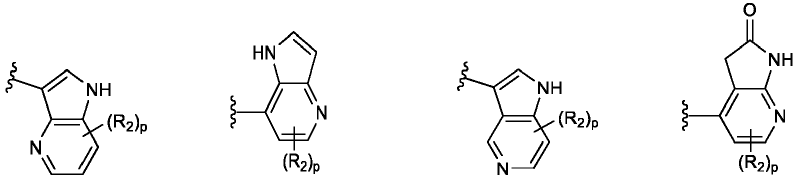 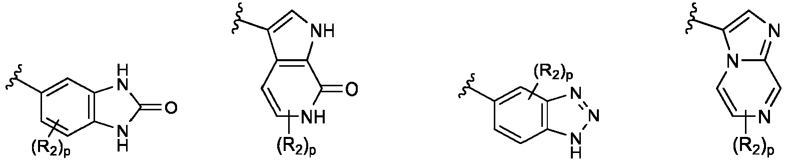 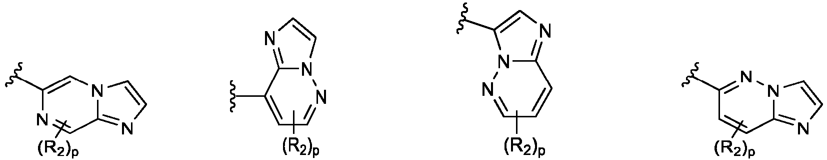 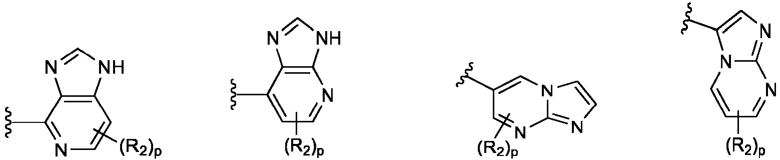 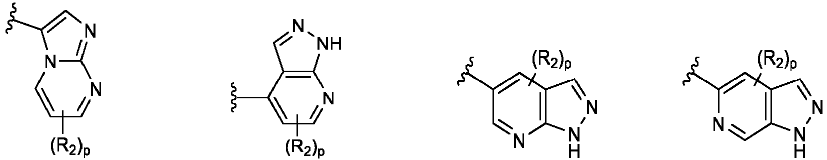 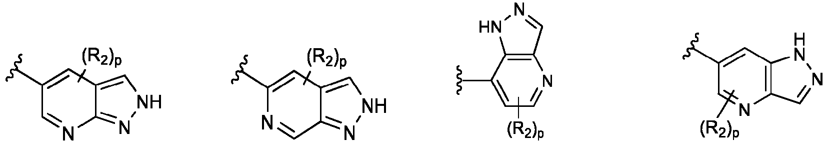 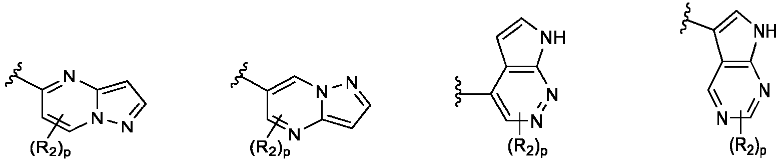 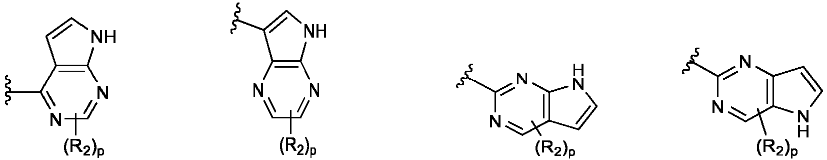 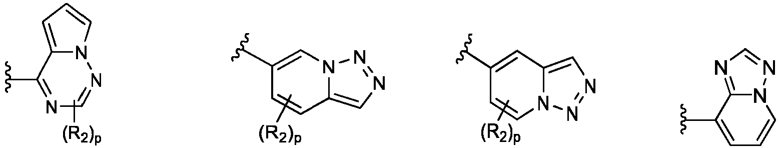 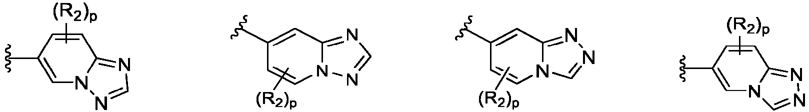 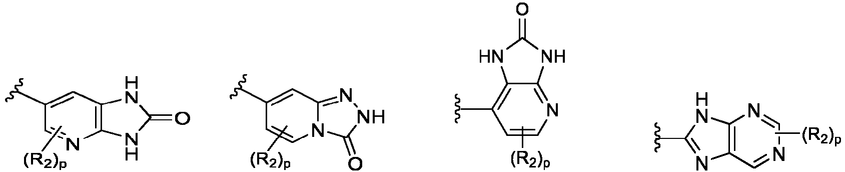 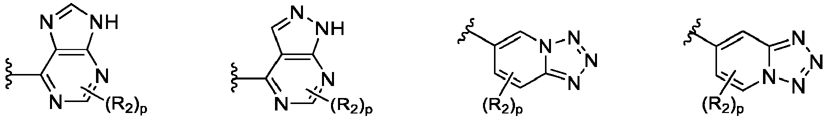 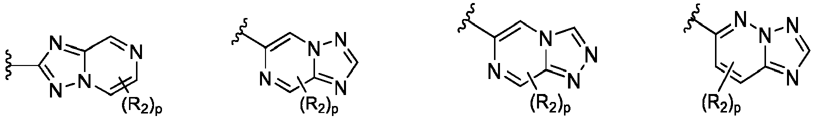 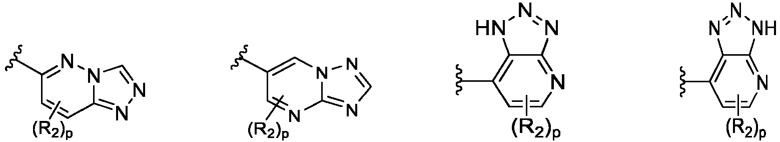 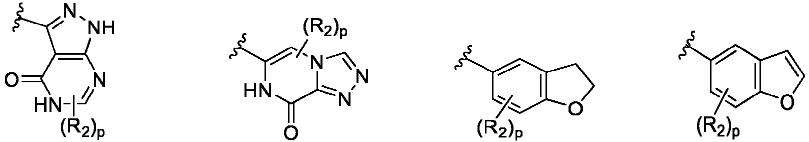 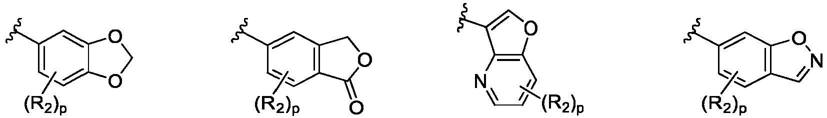 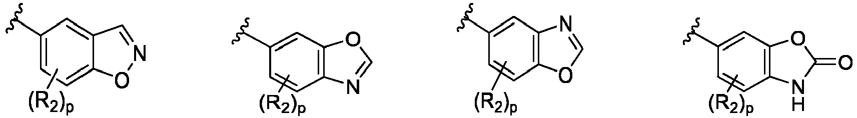 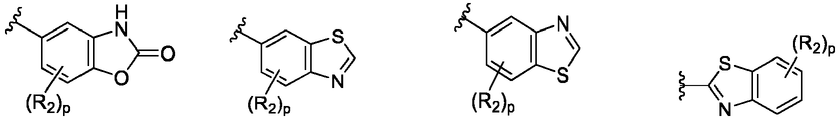 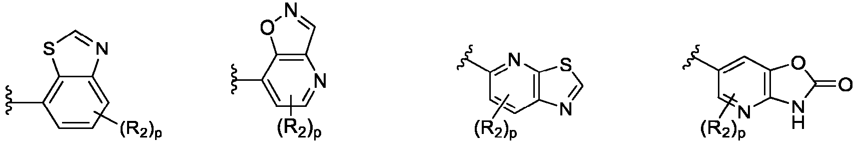 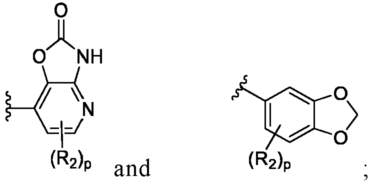 or
(vi) 10-membered heterocyclic ring selected from: 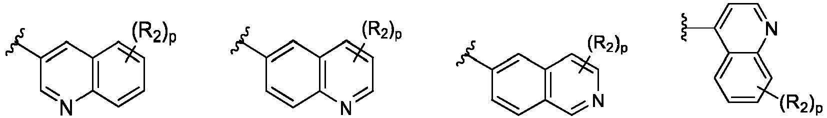 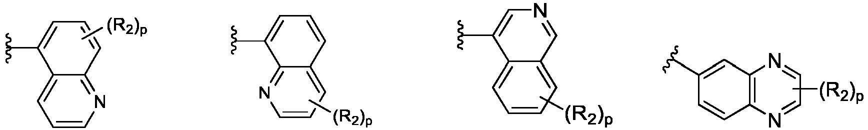 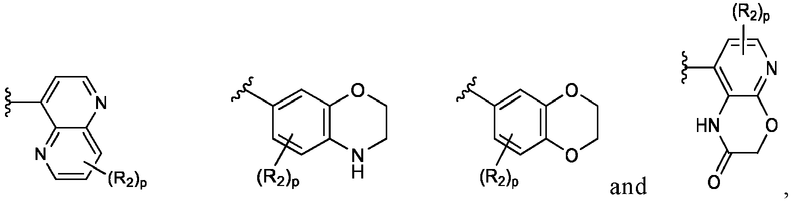 wherein R₂, R₂ₐ, R_{2b}, R_{2c}, R₂ₐ, p and q are as defined in the claims.

In a compound of Formula (I) or Formula (II), or stereoisomers, tautomer, solvates or salts thereof of the disclosure, A may be piperidinyl, phenyl, pyridinyl, pyrimidinyl, 6-azabicyclo[3.2.1]octanyl, or azabicyclo[3.2.1]octanyl, each substituted with -L-R₄ and zero to 1 R_{4b}. Included in this instance are compounds in which A is piperidinyl, phenyl, or pyridinyl, each substituted with -L-R₄ and zero to 1 R_{4b}. Also, included in this instance are compounds in which A is piperidinyl or phenyl, each substituted with -L-R₄ and zero to 1 R_{4b}. Additionally, included in this instance are compounds in which A is phenyl or pyridinyl, each substituted with -L-R₄ and zero to 1 R_{4b}.

In a compound of Formula (I) or Formula (II), or stereoisomers, tautomer, solvates or salts thereof of the disclosure, A may be piperidinyl, phenyl, pyridinyl, or pyrimidinyl, each substituted with -L-R₄ and zero to 1 R_{4b}; and L is a bond or -C(O)-. Included in this instance are compounds in which A is phenyl, or pyridinyl, each substituted with -L-R₄ and zero to 1 R_{4b}; and L is a bond.

In a compound of Formula (I) or Formula (II), or stereoisomers, tautomer, solvates or salts thereof of the disclosure, L may be a bond, -CRₓRₓ- or -C(O)(CRₓRₓ)₀₋₁-. Included in this instance are compounds in which L is a bond, -CH₂- or -C(O)(CH₂)₀₋₁-. Also included in this instance are compounds in which L is -CRₓRₓ- or -C(O)(CRₓRₓ)₀₋₁-. Additionally, included in this instance are compounds in which L is -C(O)CH₂-.

In a compound of Formula (I) or Formula (II), or stereoisomers, tautomer, solvates or salts thereof of the disclosure, L may be a bond, -CH₂- or -C(O)-.

In a compound of Formula (I) or Formula (II), or stereoisomers, tautomer, solvates or salts thereof of the disclosure, L may be a bond, -CH₂- or -C(O)(CH₂)₀₋₂-. Included in this instance are compounds in which L is -CH₂-. Also included in this instance are compounds in which L is -C(O)(CH₂)₀₋₂-.

In a compound of Formula (I) or Formula (II), or stereoisomers, tautomer, solvates or salts thereof of the disclosure, L may be a bond.

In a compound of Formula (I) or Formula (II), or stereoisomers, tautomer, solvates or salts thereof of the disclosure, L may be a -CH₂-.

In a compound of Formula (I) or Formula (II), or stereoisomers, tautomer, solvates or salts thereof of the disclosure, L may be a -C(O)-.

In a compound of Formula (I) or stereoisomers, tautomer, solvates or salts thereof of the disclosure, R₄ may be -N(CH₃)₂.

In a compound of Formula (I) or stereoisomers, tautomer, solvates or salts thereof of the disclosure, R₄ may be: (i) piperidinyl, piperazinyl, pyridinyl, azabicyclo[3.2.1]octanyl, or diazabicyclo[3.2.1]octanyl, each substituted with zero to 1 R₄ₐ and zero to 2 -CH₃; or (ii)

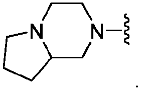

In a compound of Formula (I) or stereoisomers, tautomer, solvates or salts thereof of the disclosure, R₄ may be pyrrolidinyl, piperidinyl, piperazinyl, pyridinyl, azaspiro[3.3]heptanyl, or azabicyclo[3.2.1]octanyl, each substituted with zero to 2 R₄ₐ.

In a compound of Formula (I) or stereoisomers, tautomer, solvates or salts thereof of the disclosure, R₄ may be pyrrolidinyl, piperidinyl, piperazinyl, or pyridinyl, each substituted with zero to 2 R₄ₐ. Included in this instance are compounds in which R₄ is piperidinyl, piperazinyl, or pyridinyl. Also included in this instance are compounds in which R₄ is piperidinyl or piperazinyl. Additionally, included in this instance are compounds in which R₄ is piperazinyl substituted with zero or 1 R₄ₐ.

In a compound of Formula (I) or stereoisomers, tautomer, solvates or salts thereof of the disclosure, R₄ may be 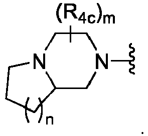 Included in this instance are compounds in which n is 1 or 2. Also included in this instance are compounds in which n is 1. Additionally, included in this instance are compounds in which n is 2.

In a compound of Formula (I) or stereoisomers, tautomer, solvates or salts thereof of the disclosure, R₄ may be pyrrolidinyl, piperidinyl, piperazinyl, or pyridinyl, each substituted with zero to 2 R₄ₐ; or

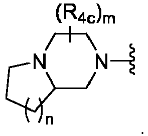

In a compound of Formula (I) or stereoisomers, tautomer, solvates or salts thereof of the disclosure, R₄ may be

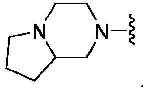

In a compound of Formula (I) or stereoisomers, tautomer, solvates or salts thereof of the disclosure, R_{4b} may be F or Cl. Included in this instance are compounds in which R_{4b} is F.

In a compound of Formula (I) or stereoisomers, tautomer, solvates or salts thereof of the disclosure, each R₄, may be independently C₁₋₄ alkyl, C₁₋₂ fluoroalkyl, -CH₂(C₃₋₆ cycloalkyl), -C(O)(C₁₋₃ alkyl), -C(O)(phenyl), -C(O)CH₂(phenyl), -C(O)OCH₂CH₃, or C₃₋₆ cycloalkyl. Included in this instance are compounds in which each R_{4c} is independently C₁₋₃ alkyl, C₁₋₂ fluoroalkyl, -CH₂(C₃₋₄ cycloalkyl), -C(O)(C₁₋₂ alkyl), -C(O)(phenyl), -C(O)CH₂(phenyl), -C(O)OCH₂CH₃, or C₃₋₄ cycloalkyl.

In a compound of Formula (I) or Formula (II), or stereoisomers, tautomer, solvates or salts thereof of the disclosure, R₁ may be hydrogen, C₁₋₃ alkyl, -CHF₂, -CF₃, or C₃₋₄ cycloalkyl. Included in this instance are compounds in which R₁ is hydrogen, -CH₃, -CH₂CH₃, -CHF₂, -CF₃, or cyclopropyl. Also, included in this instance are compounds in which R₁ is hydrogen or -CH₃.

In a compound of Formula (I) or Formula (II), or stereoisomers, tautomer, solvates or salts thereof of the disclosure, each R₂ may be independently F, Cl, -CN, -OH, C₁₋₃ alkyl, C₁₋₂ fluoroalkyl, C₁₋₂ cyanoalkyl, C₁₋₃ hydroxyalkyl, C₁₋₂ aminoalkyl, -(CH₂)₀₋₂O(C₁₋₃ alkyl), C₃₋₆ cycloalkyl, -NRₓRₓ, -(CH₂)₀₋₂C(O)NRₓRₓ, -CH₂(C₃₋₆ cycloalkyl), -CH₂(phenyl), or phenyl. Included in this instance are compounds in which each R₂ is independently Cl, -CH₃, -CH₂CH₃, -CH₂OH, -CH₂CH₂OH, -CH₂CN, -OCH₃, -CH₂OCH₃, or -CH₂CH₂S(O)₂CH₃. Also, included in this instance are compounds in which each R₂ is independently Cl, -CH₃, -CH₂OH, or -OCH₃.

In a compound of Formula (I) or Formula (II), or stereoisomers, tautomer, solvates or salts thereof of the disclosure, R₂ₐ may be C₁₋₄ alkyl, C₁₋₂ fluoroalkyl, C₁₋₄ hydroxyalkyl, -(CH₂)₁₋₃OCH₃, C₃₋₆ cycloalkyl, -CH₂C(O)NRₓRₓ, -CH₂(C₃₋₆ cycloalkyl), -CH₂(phenyl), tetrahydrofuranyl, or phenyl; and each R_{2b} is independently H, F, Cl, -CN, -NRₓRₓ, C₁₋₆ alkyl, C₁₋₂ fluoroalkyl, C₁₋₃ hydroxyalkyl, -(CH₂)₀₋₂O(C₁₋₂ alkyl), -(CH₂)₀₋₂C(O)NRₓRₓ, -(CH₂)₁₋₃(cyclopropyl), -C(O)O(C₁₋₂ alkyl), -C(O)NRₓ(C₁₋₃ alkyl), -CRₓ=CH₂, or -CH=CH(C₃₋₆ cycloalkyl). Included in this instance are compounds in which R₂ₐ is -CH₃; and each R_{2b} is independently H, Cl, or -CH₃.

In a compound of Formula (I) or Formula (II), or stereoisomers, tautomer, solvates or salts thereof of the disclosure, R₃ may be hydrogen, F, Cl, C₁₋₃ alkyl, -CHF₂, -CF₃, or C₃₋₄ cycloalkyl. Included in this instance are compounds in which R₃ is hydrogen, F, -CH₃, -CH₂CH₃, alkyl, -CHF₂, -CF₃, or cyclopropyl. Also included are compounds in which R₃ is hydrogen or -CH₃. Additionally, included are compounds in which R₃ is hydrogen.

In a compound of Formula (I) or stereoisomers, tautomer, solvates or salts thereof of the disclosure, each R₅ may be independently hydrogen, F, Cl, -CH₃, or cyclopropyl. Included in this instance are compounds in which each R₅ is independently hydrogen, -CH₃, or cyclopropyl. Also included are compounds in which each R₅ is hydrogen or -CH₃.

In a compound of Formula (II) or stereoisomers, tautomer, solvates or salts thereof of the disclosure, R₅ₐ may be hydrogen, C₁₋₂ alkyl, -CHF₂, -CF₃, or cyclopropyl. Included in this instance are compounds in which R₅ₐ is hydrogen, -CH₃, -CHF₂, -CF₃, or cyclopropyl.

In one instance, a compound of Formula (I) or stereoisomers, tautomer, solvates or salts thereof is disclosed wherein: R₁ is hydrogen or -CH₃; each R₅ is hydrogen; G is phenyl substituted with 1 to 2 substituents independently selected from F, -CN, -OCH₃, -S(O)₂CH₃, -S(O)₂(cyclopropyl), or -S(O)₂N(CH₃)₂; A is piperidinyl, phenyl or pyridinyl, each substituted with -L-R₄; L is a bond, -CH₂-, or -C(O)-; R₃ is hydrogen; R₄ is: (i) piperidinyl, piperazinyl, pyridinyl, azabicyclo[3.2.1]octanyl, or diazabicyclo[3.2.1]octanyl, each substituted with zero to 1 R₄ₐ and zero to 2 -CH₃; or (ii) 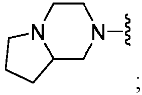 R₄ₐ is -OH, -CH₃, -CH₂CH₃, -CH(CH₃)₂, -CH₂CH(CH₃)₂, -CH₂C(CH₃)₂OH, -CH₂CH₂C(CH₃)₂OH, -CH₂(cyclopropyl), or cyclopropyl; and each R₅ is hydrogen or -CH₃.

In one instance, a compound of Formula (I) or stereoisomers, tautomer, solvates or salts thereof is disclosed wherein: R₁ is hydrogen or -CH₃; each R₅ is hydrogen; G is phenyl substituted with 1 to 2 substituents independently selected from F, -OCH₃, or -S(O)₂CH₃; A is phenyl or pyridinyl, each substituted with -L-R₄; L is a bond or -C(O)-; R₃ is hydrogen; R₄ is piperazinyl substituted with zero or 1 R₄ₐ; R₄ₐ is -CH(CH₃)₂, -CH₂CH(CH₃)₂, -CH₂C(CH₃)₂OH, -CH₂CH₂C(CH₃)₂OH, or -CH₂(cyclopropyl); and each R₅ is hydrogen or -CH₃. Included in this instance are compounds in which X is N and Y is CH. Also included in this instance are compounds in which X is CH and Y is N.

In one instance, a compound of Formula (II) or stereoisomers, tautomer, solvates or salts thereof is disclosed wherein X is N(CH₃); Y is CH; Q₁ is hydrogen; Q₂ is A; A is phenyl; L is a bond; R₁ is hydrogen; R₃ is hydrogen; R₄ is piperazinyl; and R₄ₐ is -CH(CH₃)₂ or -CH₂CH(CH₃)₂.

One embodiment provides a compound of Formula (I) or stereoisomers, tautomer, solvates or salts thereof, wherein said compound is: 2-(3,4-dimethoxyphenyl)-6-(4-(4-isopropylpiperazin-1-yl)phenyl)-1-methyl-1H-pyrrolo[3,2-b]pyridine (1); 6-(4-(4-isopropylpiperazin-1-yl)phenyl)-1-methyl-2-(4-(methylsulfonyl)phenyl)-1H-pyrrolo[3,2-b]pyridine (2); 1-(4-(4-(2-(3,4-dimethoxyphenyl)-1-methyl-1H-pyrrolo[3,2-b]pyridin-6-yl)phenyl) piperazin-1-yl)-2-methylpropan-2-ol (4); (4-(2-(3,4-dimethoxyphenyl)-1-methyl-1H-pyrrolo[3,2-b]pyridin-6-yl)phenyl)(4-isopropylpiperazin-1-yl)methanone (5); 6-(4-(4-isobutylpiperazin-1-yl) phenyl)-1-methyl-2-(4-(methylsulfonyl)phenyl)-1H-pyrrolo[3,2-b]pyridine (6); 6-(4-(4-(cyclopropylmethyl)piperazin-1-yl)phenyl)-2-(3,4-dimethoxyphenyl)-1-methyl-1H-pyrrolo[3,2-b]pyridine (8); 2-(3,4-dimethoxyphenyl)-6-(6-(4-isopropylpiperazin-1-yl) pyridin-3-yl)-1-methyl-1H-pyrrolo[3,2-b]pyridine (9); 2-(3-fluoro-4-methoxyphenyl)-6-(4-(4-isopropylpiperazin-1-yl)phenyl)-1-methyl-1H-pyrrolo[3,2-b]pyridine (10); 6-(4-(4-isopropylpiperazin-1-yl)phenyl)-2-(4-(methylsulfonyl)phenyl)-1H-pyrrolo[3,2-b]pyridine (11); 4-(4-(4-(2-(3,4-dimethoxyphenyl)-1-methyl-1H-pyrrolo[3,2-b]pyridin-6-yl)phenyl) piperazin-1-yl)-2-methylbutan-2-ol (12); 2-(3,4-dimethoxyphenyl)-1-methyl-6-(4-(piperazin-1-yl)phenyl)-1H-pyrrolo[3,2-b] pyridine (13); 6-(4-(4-isobutylpiperazin-1-yl)phenyl)-2-(4-(methylsulfonyl)phenyl)-1H-pyrrolo[3,2-b]pyridine (14); 2-methyl-1-(4-(4-(1-methyl-2-(4-(methylsulfonyl)phenyl)-1H-pyrrolo[3,2-b]pyridin-6-yl)phenyl)piperazin-1-yl)propan-2-ol (15); -methyl-4-(4-(4-(1-methyl-2-(4-(methylsulfonyl)phenyl)-1H-pyrrolo[3,2-b]pyridin-6-yl)phenyl)piperazin-1-yl)butan-2-ol (16); or 6-(4-(4-isopropylpiperazin-1-yl)phenyl)-1-methyl-2-(3-(methylsulfonyl)phenyl)-1H-pyrrolo[3,2-b]pyridine (18).

One instance of the disclosure (which is not recited in the claims) provides a compound of Formula (I) or stereoisomers, tautomer, solvates or salts thereof, wherein said compound is 2-(3,4-dimethoxyphenyl)-6-(4-(4-isopropylpiperazin-1-yl)phenyl)-1-methyl-1H-pyrrolo[2,3-b]pyridine (7).

One embodiment provides a compound of Formula (I) or stereoisomers, tautomer, solvates or salts thereof is provided, wherein said compound is: 6-(4-(4-isopropylpiperazin-1-yl)phenyl)-4-methyl-2-(4-(methylsulfonyl)phenyl)-4H-pyrrolo[3,2-b]pyridine (3); or 6-(4-(4-isobutylpiperazin-1-yl)phenyl)-4-methyl-2-(4-(methylsulfonyl)phenyl)-4H-pyrrolo[3,2-b]pyridine (17).

The present disclosure describes compounds of the Formula (I) having TLR9 IC₅₀ values of ≤ 0.6 µM. Compounds of the Formula (I) may have TLR9 IC₅₀ values of ≤ 0.1 µM. Compounds of the Formula (I) may have TLR9 IC₅₀ values of ≤ 0.05 µM. Compounds of the Formula (I) may have TLR9 IC₅₀ values of ≤ 0.025 µM. Compounds of the Formula (I) may have TLR9 IC₅₀ values of ≤ 0.015 µM. Compounds of the Formula (I) may have TLR9 IC₅₀ values of ≤ 0.01 µM.

The present disclosure describes a composition comprising at least one of the compounds of the present disclosure, or a stereoisomer, a tautomer, or a pharmaceutically acceptable salt or a solvate thereof.

The present disclosure also describes a pharmaceutical composition comprising a pharmaceutically acceptable carrier and at least one of the compounds of the present disclosure or a stereoisomer, a tautomer, or a pharmaceutically acceptable salt or a solvate thereof.

The pharmaceutical composition may comprise a pharmaceutically acceptable carrier and a therapeutically effective amount of at least one of the compounds of the present disclosure or a stereoisomer, a tautomer, or a pharmaceutically acceptable salt or a solvate thereof.

The present disclosure further describes a process for making a compound of the present disclosure.

The present disclosure also describes an intermediate for making a compound of the present disclosure.

The present disclosure also describes a pharmaceutical composition as defined above further comprising one or more additional therapeutic agents.

### DEFINITIONS

The features and advantages of the disclosure and invention may be more readily understood by those of ordinary skill in the art upon reading the following detailed description. It is to be appreciated that certain features of the disclosure and invention that are, for clarity reasons, described above and below in the context of separate instances or embodiments, may also be combined to form a single instance or embodiment. Conversely, various features of the disclosure and invention that are, for brevity reasons, described in the context of a single instance or embodiment, may also be combined so as to form sub-combinations thereof. Instances and embodiments identified herein as exemplary or preferred are intended to be illustrative and not limiting.

Unless specifically stated otherwise herein, references made in the singular may also include the plural. For example, "a" and "an" may refer to either one, or one or more.

As used herein, the phase "compounds" refers to at least one compound. For example, a compound of Formula (I) includes a compound of Formula (I) and two or more compounds of Formula (I).

Unless otherwise indicated, any heteroatom with unsatisfied valences is assumed to have hydrogen atoms sufficient to satisfy the valences.

The definitions set forth herein take precedence over definitions set forth in any patent, patent application, and/or patent application publication referenced herein.

Listed below are definitions of various terms used to describe the present disclosure and invention. These definitions apply to the terms as they are used throughout the specification (unless they are otherwise limited in specific instances) either individually or as part of a larger group.

Throughout the specification, groups and substituents thereof may be chosen by one skilled in the field to provide stable moieties and compounds.

In accordance with a convention used in the art, 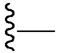 is used in structural formulas herein to depict the bond that is the point of attachment of the moiety or substituent to the core or backbone structure.

The terms "halo" and "halogen," as used herein, refer to F, Cl, Br, and I.

The term "cyano" refers to the group -CN.

The term "amino" refers to the group -NH₂.

The term "oxo" refers to the group =O.

The term "alkyl" as used herein, refers to both branched and straight-chain saturated aliphatic hydrocarbon groups containing, for example, from 1 to 12 carbon atoms, from 1 to 6 carbon atoms, and from 1 to 4 carbon atoms. Examples of alkyl groups include, but are not limited to, methyl (Me), ethyl (Et), propyl (*e.g.*, n-propyl and i-propyl), butyl (*e.g.,* n-butyl, i-butyl, sec-butyl, and *t*-butyl)*,* and pentyl (*e.g*., n-pentyl, isopentyl, neopentyl), n-hexyl, 2-methylpentyl, 2-ethylbutyl, 3-methylpentyl, and 4-methylpentyl. When numbers appear in a subscript after the symbol "C", the subscript defines with more specificity the number of carbon atoms that a particular group may contain. For example, "C₁₋₆ alkyl" denotes straight and branched chain alkyl groups with one to six carbon atoms.

The term "fluoroalkyl" as used herein is intended to include both branched and straight-chain saturated aliphatic hydrocarbon groups substituted with one or more fluorine atoms. For example, "C₁₋₄ fluoroalkyl" is intended to include C₁, C₂, C₃, and C₄ alkyl groups substituted with one or more fluorine atoms. Representative examples of fluoroalkyl groups include, but are not limited to, -CF₃ and -CH₂CF₃.

The term "hydroxyalkyl" includes both branched and straight-chain saturated alkyl groups substituted with one or more hydroxyl groups. For example, "hydroxyalkyl" includes -CH₂OH, -CH₂CH₂OH, and C₁₋₄ hydroxyalkyl.

The term "aminoalkyl" includes both branched and straight-chain saturated alkyl groups substituted with one or more amine groups. For example, "aminoalkyl" includes -CH₂NH₂, -CH₂CH₂NH₂, and C₁₋₄ aminoalkyl.

The term "cyanoalkyl" includes both branched and straight-chain saturated alkyl groups substituted with one or more cyano groups. For example, "aminoalkyl" includes -CH₂CN, -CH₂CH₂CN, and C₁₋₄ cyanoalkyl.

The term "alkoxy," as used herein, refers to an alkyl group attached to the parent molecular moiety through an oxygen atom, for example, methoxy group (-OCH₃). For example, "C₁₋₃ alkoxy" denotes alkoxy groups with one to three carbon atoms.

The terms "fluoroalkoxy" and "-O(fluoroalkyl)" represent a fluoroalkyl group as defined above attached through an oxygen linkage (-O-). For example, "C₁₋₄ fluoroalkoxy" is intended to include C₁, C₂, C₃, and C₄ fluoroalkoxy groups.

The term "alkoxyalkyl," as used herein, refers to an alkoxy group attached through its oxygen atom to an alkyl group, which is attached to the parent molecular moiety through a carbon atom, for example, methoxymethyl group (-CH₂OCH₃). For example, "C₂₋₄ alkoxyalkyl" denotes alkoxyalkyl groups with two to four carbon atoms, such as -CH₂OCH₃, -CH₂CH₂OCH₃, -CH₂OCH₂CH₃, and -CH₂CH₂OCH₂CH₃.

The term "cycloalkyl," as used herein, refers to a group derived from a nonaromatic monocyclic or polycyclic hydrocarbon molecule by removal of one hydrogen atom from a saturated ring carbon atom. Representative examples of cycloalkyl groups include, but are not limited to, cyclopropyl, cyclopentyl, and cyclohexyl. When numbers appear in a subscript after the symbol "C", the subscript defines with more specificity the number of carbon atoms that a particular cycloalkyl group may contain. For example, "C₃₋₆ cycloalkyl" denotes cycloalkyl groups with three to six carbon atoms.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compounds of Formula (I) and Formula (II) can be provided as amorphous solids or crystalline solids. Lyophilization can be employed to provide the compounds of Formula (I) and Formula (II) as amorphous solids.

It should further be understood that solvates (e.g., hydrates) of the compounds of Formula (I) and Formula (II) are also within the scope of the present disclosure. The term "solvate" means a physical association of a compound of Formula (I) or a compound of Formula (II) with one or more solvent molecules, whether organic or inorganic. This physical association includes hydrogen bonding. In certain instances the solvate will be capable of isolation, for example when one or more solvent molecules are incorporated in the crystal lattice of the crystalline solid. "Solvate" encompasses both solution-phase and isolable solvates. Exemplary solvates include hydrates, ethanolates, methanolates, isopropanolates, acetonitrile solvates, and ethyl acetate solvates. Methods of solvation are known in the art.

Various forms of prodrugs are well known in the art and are described in Rautio, J. et al., Nature Review Drug Discovery, 17, 559-587 (2018).

In addition, compounds of Formula (I) and Formula (II), subsequent to their preparation, can be isolated and purified to obtain a composition containing an amount by weight equal to or greater than 99% of a compound of Formula (I) and Formula (II), respectively ("substantially pure"), which is then used or formulated as described herein. Such "substantially pure" compounds of Formula (I) and "substantially pure" compounds of Formula (II) are also contemplated herein as part of the present disclosure.

"Stable compound" and "stable structure" are meant to indicate a compound that is sufficiently robust to survive isolation to a useful degree of purity from a reaction mixture, and formulation into an efficacious therapeutic agent. The present disclosure and invention is intended to embody stable compounds.

"Therapeutically effective amount" is intended to include an amount of a compound of the present invention alone or an amount of the combination of compounds claimed or an amount of a compound of the present disclosure in combination with other active ingredients effective to act as an inhibitor of TLR9, or effective to treat or prevent disorders associated with a fibrotic disease or disorder, dysregulation of bile acids, such as pathological fibrosis.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting its development; and/or (c) relieving the disease-state, i.e., causing regression of the disease state.

The compounds of the present disclosure and invention are intended to include all isotopes of atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopically-labeled compounds of the disclosure can generally be prepared by conventional techniques known to those skilled in the art or by processes analogous to those described herein, using an appropriate isotopically-labeled reagent in place of the non-labeled reagent otherwise employed. For example, methyl (-CH₃) also includes deuterated methyl groups such as -CD₃.

### UTILITY

The compounds of the disclosure are useful for inhibiting the TLR9 receptor.

It is to be understood, even if not explicitly stated herein, that methods for the treatment of a disease, disorder, or condition refer to a substance or composition for use in such a method.

One instance of the disclosure (which is not claimed as such) provides a method for the treatment of a disease, disorder, or condition associated with dysregulation of bile acids in a patient in need of such treatment, and the method comprises administering a therapeutically effective amount of a compound of the present disclosure, or a stereoisomer, a tautomer, or a pharmaceutically acceptable salt or solvate thereof, to the patient.

One instance of the disclosure (which is not claimed as such) provides a method for the treatment of a disease, disorder, or condition associated with activity of the TLR9 receptor in a patient in need of such treatment comprising administering a therapeutically effective amount of a compound of the present disclosure, or a stereoisomer, a tautomer, or a pharmaceutically acceptable salt or solvate thereof, to the patient.

In one instance, a method for the treatment of the disease, disorder, or condition comprises administering to a patient in need of such treatment a therapeutically effective amount of at least one of the compounds of the present disclosure, alone, or, optionally, in combination with another compound of the present disclosure and/or at least one other type of therapeutic agent.

One instance of the disclosure (which is not claimed as such) provides a method for eliciting an TLR9 receptor agonizing effect in a patient comprising administering a therapeutically effective amount of a compound of the present disclosure, or a stereoisomer, a tautomer, or a pharmaceutically acceptable salt or solvate thereof, to the patient.

In some instances, the disease, disorder, or condition is associated with TLR9 dysfunction include pathological fibrosis, cancer, inflammatory disorders, metabolic, or cholestatic disorders.

Provided herein is a compound of the invention, or a pharmaceutical composition of the invention, for use in treating pathological fibrosis. In embodiments, the pathological fibrosis is liver fibrosis, renal fibrosis, biliary fibrosis, or pancreatic fibrosis. Also provided herein is a compound of the invention, or a pharmaceutical composition of the invention, for use in treating nonalcoholic steatohepatitis (NASH), non-alcoholic fatty liver disease (NAFLD), chronic kidney disease, diabetic kidney disease, primary sclerosing cholangitis (PSC), or primary biliary cirrhosis. Provided herein is a compound of the invention, or a pharmaceutical composition of the invention, for use in treating idiopathic pulmonary fibrosis (IPF).

The disease, disorder, or condition to be treated in accordance with the present disclosure may be associated with fibrosis, including liver, biliary, renal, cardiac, dermal, ocular, and pancreatic fibrosis.

In other instances, the disease, disorder, or condition to be treated in accordance with the present disclosure may be associated with cell-proliferative disorders, such as cancer. In some instances, the cancer includes solid tumor growth or neoplasia. In otherinstances, the cancer includes tumor metastasis. In someinstances, the cancer is of the liver, gall bladder, small intestine, large intestine, kidney, prostate, bladder, blood, bone, brain, breast, central nervous system, cervix, colon, endometrium, esophagus, genitalia, genitourinary tract, head, larynx, lung, muscle tissue, neck, oral or nasal mucosa, ovary, pancreas, skin, spleen, stomach, testicle, or thyroid. In other instances, the cancer is a carcinoma, sarcoma, lymphoma, leukemia, melanoma, mesothelioma, multiple myeloma, or seminoma.

Examples of diseases, disorders, or conditions associated with the activity of FXR that can be prevented, modulated, or treated according to the present disclosure include, but are not limited to, transplant injection, fibrotic disorders (e. g., liver fibrosis, kidney fibrosis), inflammatory disorders (e.g., acute hepatitis, chronic hepatitis, non-alcoholic steatohepatitis (NASH), irritable bowel syndrome (IBS), inflammatory bowel disease (IBD)), as well as cell-proliferative disorders (e.g., cancer, myeloma, fibroma, hepatocellular carcinoma, colorectal cancer, prostate cancer, leukemia, Kaposi's sarcoma, solid tumors).

The fibrotic disorders, inflammatory disorders, as well as cell-proliferative disorders that are suitable to be prevented or treated by the compounds of the present disclosure include, but are not limited to, non-alcoholic fatty liver disease (NAFLD), alcoholic or non-alcoholic steatohepatitis (NASH), acute hepatitis, chronic hepatitis, liver cirrhosis, primary biliary cirrhosis, primary sclerosing cholangitis, drug-induced hepatitis, biliary cirrhosis, portal hypertension, regenerative failure, liver hypofunction, hepatic blood flow disorder, nephropathy, irritable bowel syndrome (IBS), inflammatory bowel disease (IBD), abnormal pancreatic secretion, benign prostatic hyperplasia, neuropathic bladder disease, diabetic nephropathy, focal segmental glomerulosclerosis, IgA nephropathy, nephropathy induced by drugs or transplantation, autoimmune nephropathy, lupus nephritis, liver fibrosis, kidney fibrosis, chronic kidney disease (CKD), diabetic kidney disease (DKD), skin fibrosis, keloids, systemic sclerosis, scleroderma, virally-induced fibrosis, idiopathic pulmonary fibrosis (IPF), interstitial lung disease, non-specific interstitial pneumonia (NSIP), usual interstitial pneumonia (UIP), radiation-induced fibrosis, familial pulmonary fibrosis, airway fibrosis, chronic obstructive pulmonary disease (COPD), spinal cord tumor, hernia of intervertebral disk, spinal canal stenosis, heart failure, cardiac fibrosis, vascular fibrosis, perivascular fibrosis, foot-and-mouth disease, cancer, myeloma, fibroma, hepatocellular carcinoma, colorectal cancer, prostate cancer, leukemia, chronic lymphocytic leukemia, Kaposi's sarcoma, solid tumors, cerebral infarction, cerebral hemorrhage, neuropathic pain, peripheral neuropathy, age-related macular degeneration (AMD), glaucoma, ocular fibrosis, corneal scarring, diabetic retinopathy, proliferative vitreoretinopathy (PVR), cicatricial pemphigoid glaucoma filtration surgery scarring, Crohn's disease or systemic lupus erythematosus; keloid formation resulting from abnormal wound healing; fibrosis occurring after organ transplantation, myelofibrosis, and fibroids. The present disclosure describes a method for the treatment of a fibrotic disorder, an inflammatory disorder, or a cell-proliferative disorder, comprising administering to a patient in need of such treatment a therapeutically effective amount of at least one of the compounds of the present disclosure, alone, or, optionally, in combination with another compound of the present disclosure and/or at least one other type of therapeutic agent.

In another instance, the present disclosure provides a compound of the present disclosure for use in therapy.

For example, a compound of the present disclosure may be used in therapy for the treatment of a fibrotic disorder, an inflammatory disorder, or a cell-proliferative disorder thereof.

A method for the treatment of a fibrotic disorder, an inflammatory disorder, or a cell-proliferative disorder in accordance with the present disclosure may comprise administering to a patient in need thereof a therapeutically effective amount of a first and second therapeutic agent, wherein the first therapeutic agent is a compound of the present disclosure.

In another instance, the present disclosure provides a combined preparation of a compound of the present disclosure and additional therapeutic agent(s) for simultaneous, separate or sequential use in therapy.

The combined preparation of a compound of the present disclosure and additional therapeutic agent(s) may be for simultaneous, separate or sequential use in the treatment of a fibrotic disorder, an inflammatory disorder, or a cell-proliferative disorder.

The compounds of the present disclosure may be employed in combination with additional therapeutic agent(s), such as one or more anti-fibrotic and/or antiinflammatory therapeutic agents.

Additional therapeutic agent(s) used in combined pharmaceutical compositions or combined methods or combined uses, may be selected from one or more, preferably one to three, of the following therapeutic agents: TGFβ receptor inhibitors (for example, galunisertib), inhibitors of TGFβ synthesis (for example, pirfenidone), inhibitors of vascular endothelial growth factor (VEGF), platelet-derived growth factor (PDGF) and fibroblast growth factor (FGF) receptor kinases (for example, nintedanib), humanized anti-α_{V}β6 integrin monoclonal antibody (for example, 3G9), human recombinant pentraxin-2, recombinant human Serum Amyloid P, recombinant human antibody against TGFβ-1, -2, and -3, endothelin receptor antagonists (for example, macitentan), interferon gamma, c-Jun amino-terminal kinase (JNK) inhibitor (for example, 4-[[9-[(3S)-tetrahydro-3-furanyl]-8-[(2,4,6-trifluorophenyl)amino]-9*H*-purin-2-yl]amino]-trans-cyclohexanol, 3-pentylbenzeneacetic acid (PBI-4050), tetra-substituted porphyrin derivative containing manganese (III), monoclonal antibody targeting eotaxin-2, interleukin-13 (IL-13) antibody (for example, lebrikizumab, tralokinumab), bispecific antibody targeting interleukin 4 (IL-4) and interleukin 13 (IL-13), NK1 tachykinin receptor agonist (for example, Sar⁹, Met(O₂)¹¹-Substance P), Cintredekin Besudotox, human recombinant DNA-derived, IgG1 kappa monoclonal antibody to connective growth factor, and fully human IgG1 kappa antibody, selective for CC-chemokine ligand 2 (for example, carlumab, CCX140), antioxidants (for example, N-acetylcysteine), phosphodiesterase 5 (PDE5) inhibitors (for example, sildenafil), agents for treatment of obstructive airway diseases such as muscarinic antagonists (for example, tiotropium, ipatropium bromide), adrenergic β2 agonists (for example, salbutamol, salmeterol), corticosteroids (for example, triamcinolone, dexamethasone, fluticasone), immunosuppressive agents (for example, tacrolimus, rapamycin, pimecrolimus), and therapeutic agents useful for the treatment of fibrotic conditions, such as liver, biliary, and kidney fibrosis, Non-Alcoholic Fatty Liver Disease (NALFD), Non-Alcoholic Steato-Hepatitis (NASH), cardiac fibrosis, Idiopathic Pulmonary Fibrosis (IPF), and systemic sclerosis. The therapeutic agents useful for the treatment of such fibrotic conditions include, but are not limited to, FXR agonists (for example OCA, GS-9674, and LJN452), LOXL2 inhibitors (for example simtuzumab), LPA1 antagonists (for example, BMS-986020 and SAR 100842), PPAR modulators (for example, elafibrinor, pioglitazone, and saroglitazar, IVA337), SSAO/VAP-1 inhibitors (for example, PXS-4728A and SZE5302), ASK-1 inhibitors (for example GS-4997 or selonsertib), ACC inhibitors (for example, CP-640186 and NDI-010976 or GS-0976), FGF21 mimetics (for example, LY2405319 and BMS-986036), caspase inhibitors (for example, emricasan), NOX4 inhibitors (for example, GKT137831), MGAT2 inhibitor (for example, BMS-963272), αV integrin inhibitors (for example, abituzumab)and bile acid/fatty acid conjugates (for example aramchol).The FXR agonists of various embodiments of the present disclosure may also be used in combination with one or more therapeutic agents such as CCR2/5 inhibitors (for example, cenicriviroc), Galectin-3 inhibitors (for example, TD-139, GR-MD-02), leukotriene receptor antagonists (for example, tipelukast, montelukast), SGLT2 inhibitors (for example, dapagliflozin, remogliflozin), GLP-1 receptor agonists (for example, liraglutide and semaglutide), FAK inhibitors (for example, GSK-2256098), CB1 inverse agonists (for example, JD-5037), CB2 agonists (for example, APD-371 and JBT-101), autotaxin inhibitors (for example, GLPG1690), prolyl t-RNA synthetase inhibitors (for example, halofugenone), FPR2 agonists (for example, ZK-994), and THR agonists (for example, MGL:3196). In another embodiment, additional therapeutic agent(s) used in combined pharmaceutical compositions or combined methods or combined uses, are selected from one or more, preferably one to three, of immunoncology agents, such as Alemtuzumab, Atezolizumab, Ipilimumab, Nivolumab, Ofatumumab, Pembrolizumab, and Rituximab.

When the terms "TLR9-associated condition" or "TLR9-associated disease or disorder" are used herein, each is intended to encompass all of the conditions identified above as if repeated at length, as well as any other condition that is affected by inhibition of TLR9.

The above other therapeutic agents, when employed in combination with the compounds of the present disclosure, may be used, for example, in those amounts indicated in the *Physicians' Desk Reference* (PDR) or as otherwise determined by one of ordinary skill in the art. In the methods of the present disclosure, such other therapeutic agent(s) may be administered prior to, simultaneously with, or following the administration of the compounds described herein. The present disclosure also describes pharmaceutical compositions capable of treating TLR9-associated conditions.

The compositions described herein may contain other therapeutic agents as described above and may be formulated, for example, by employing conventional solid or liquid vehicles or diluents, as well as pharmaceutical additives of a type appropriate to the mode of desired administration (*e.g*., excipients, binders, preservatives, stabilizers, flavors, etc.) according to techniques such as those well known in the art of pharmaceutical formulation.

Accordingly, the present disclosure further includes compositions comprising one or more compounds of Formula (I) and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include without limitation the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 17th Edition (1985).

Compounds in accordance with Formula (I) can be administered by any means suitable for the condition to be treated, which can depend on the need for site-specific treatment or quantity of Formula (I) compound to be delivered.

Compounds in accordance with Formula (II) can be administered by any means suitable for the condition to be treated, which can depend on the need for site-specific treatment or quantity of Formula (II) compound to be delivered.

Also embraced within this disclosure is a class of pharmaceutical compositions comprising a compound of Formula (I) and/or a compound of Formula (II), and one or more non-toxic, pharmaceutically-acceptable carriers and/or diluents and/or adjuvants (collectively referred to herein as "carrier" materials) and, if desired, other active ingredients. The compounds of Formula (I) and Formula (II) may be administered by any suitable route, preferably in the form of a pharmaceutical composition adapted to such a route, and in a dose effective for the treatment intended. The compounds and compositions of the present disclosure may, for example, be administered orally, mucosally, or parentally including intravascularly, intravenously, intraperitoneally, subcutaneously, intramuscularly, and intrasternally in dosage unit formulations containing conventional pharmaceutically acceptable carriers, adjuvants, and vehicles. For example, the pharmaceutical carrier may contain a mixture of mannitol or lactose and microcrystalline cellulose. The mixture may contain additional components such as a lubricating agent, e.g. magnesium stearate and a disintegrating agent such as crospovidone. The carrier mixture may be filled into a gelatin capsule or compressed as a tablet. The pharmaceutical composition may be administered as an oral dosage form or an infusion, for example.

For oral administration, the pharmaceutical composition may be in the form of, for example, a tablet, capsule, liquid capsule, suspension, or liquid. The pharmaceutical composition is preferably made in the form of a dosage unit containing a particular amount of the active ingredient. For example, the pharmaceutical composition may be provided as a tablet or capsule comprising an amount of active ingredient in the range of from about 0.1 to 1000 mg, preferably from about 0.25 to 250 mg, and more preferably from about 0.5 to 100 mg. A suitable daily dose for a human or other mammal may vary widely depending on the condition of the patient and other factors, but, can be determined using routine methods.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the disclosure can contain at least one agent selected from sweetening agents, flavoring agents, coloring agents, demulcents, antioxidants, and preserving agents.

A tablet can, for example, be prepared by admixing at least one compound of Formula (I) and/or Formula (II) with at least one non-toxic pharmaceutically acceptable excipient suitable for the manufacture of tablets. Exemplary excipients include, but are not limited to, for example, inert diluents, such as, for example, calcium carbonate, sodium carbonate, lactose, calcium phosphate, and sodium phosphate; granulating and disintegrating agents, such as, for example, microcrystalline cellulose, sodium crosscarmellose, corn starch, and alginic acid; binding agents, such as, for example, starch, gelatin, polyvinyl-pyrrolidone, and acacia; and lubricating agents, such as, for example, magnesium stearate, stearic acid, and talc. Additionally, a tablet can either be uncoated, or coated by known techniques to either mask the bad taste of an unpleasant tasting drug, or delay disintegration and absorption of the active ingredient in the gastrointestinal tract thereby sustaining the effects of the active ingredient for a longer period. Exemplary water soluble taste masking materials, include, but are not limited to, hydroxypropyl-methylcellulose and hydroxypropyl-cellulose. Exemplary time delay materials, include, but are not limited to, ethyl cellulose and cellulose acetate butyrate.

Hard gelatin capsules can, for example, be prepared by mixing at least one compound of Formula (I) and/or Formula (II) with at least one inert solid diluent, such as, for example, calcium carbonate; calcium phosphate; and kaolin.

Soft gelatin capsules can, for example, be prepared by mixing at least one compound of Formula (I) and/or Formula (II) with at least one water soluble carrier, such as, for example, polyethylene glycol; and at least one oil medium, such as, for example, peanut oil, liquid paraffin, and olive oil.

An aqueous suspension can be prepared, for example, by admixing at least one compound of Formula (I) and/or Formula (II) with at least one excipient suitable for the manufacture of an aqueous suspension. Exemplary excipients suitable for the manufacture of an aqueous suspension, include, but are not limited to, for example, suspending agents, such as, for example, sodium carboxymethylcellulose, methylcellulose, hydroxypropylmethyl-cellulose, sodium alginate, alginic acid, polyvinyl-pyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example heptadecaethylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) and/or Formula (II) in either a vegetable oil, such as, for example, arachis oil; olive oil; sesame oil; and coconut oil; or in mineral oil, such as, for example, liquid paraffin. An oily suspension can also contain at least one thickening agent, such as, for example, beeswax; hard paraffin; and cetyl alcohol. In order to provide a palatable oily suspension, at least one of the sweetening agents already described hereinabove, and/or at least one flavoring agent can be added to the oily suspension. An oily suspension can further contain at least one preservative, including, but not limited to, for example, an anti-oxidant, such as, for example, butylated hydroxyanisol, and alpha-tocopherol.

Dispersible powders and granules can, for example, be prepared by admixing at least one compound of Formula (I) and/or Formula (II) with at least one dispersing and/or wetting agent; at least one suspending agent; and/or at least one preservative. Suitable dispersing agents, wetting agents, and suspending agents are as already described above. Exemplary preservatives include, but are not limited to, for example, anti-oxidants, e.g., ascorbic acid. In addition, dispersible powders and granules can also contain at least one excipient, including, but not limited to, for example, sweetening agents; flavoring agents; and coloring agents.

An emulsion of at least one compound of Formula (I) and/or Formula (II) thereof can, for example, be prepared as an oil-in-water emulsion. The oily phase of the emulsions comprising compounds of Formula (I) and/or Formula (II) may be constituted from known ingredients in a known manner. The oil phase can be provided by, but is not limited to, for example, a vegetable oil, such as, for example, olive oil and arachis oil; a mineral oil, such as, for example, liquid paraffin; and mixtures thereof. While the phase may comprise merely an emulsifier, it may comprise a mixture of at least one emulsifier with a fat or an oil or with both a fat and an oil. Suitable emulsifying agents include, but are not limited to, for example, naturally-occurring phosphatides, e.g., soy bean lecithin; esters or partial esters derived from fatty acids and hexitol anhydrides, such as, for example, sorbitan monooleate; and condensation products of partial esters with ethylene oxide, such as, for example, polyoxyethylene sorbitan monooleate. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. An emulsion can also contain a sweetening agent, a flavoring agent, a preservative, and/or an antioxidant. Emulsifiers and emulsion stabilizers suitable for use in the formulation of the present disclosure include Tween 60, Span 80, cetostearyl alcohol, myristyl alcohol, glyceryl monostearate, sodium lauryl sulfate, glyceryl distearate alone or with a wax, or other materials well known in the art.

The compounds of Formula (I) and/or Formula (II) can, for example, also be delivered intravenously, subcutaneously, and/or intramuscularly via any pharmaceutically acceptable and suitable injectable form. Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e. Captisol), cosolvent solubilization (i.e. propylene glycol) or micellar solubilization (i.e. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

A sterile injectable oil-in-water microemulsion can, for example, be prepared by 1) dissolving at least one compound of Formula (I) and/or Formula (II) in an oily phase, such as, for example, a mixture of soybean oil and lecithin; 2) combining the Formula (I) and/or Formula (II) containing oil phase with a water and glycerol mixture; and 3) processing the combination to form a microemulsion.

A sterile aqueous or oleaginous suspension can be prepared in accordance with methods already known in the art. For example, a sterile aqueous solution or suspension can be prepared with a non-toxic parenterally-acceptable diluent or solvent, such as, for example, 1,3-butane diol; and a sterile oleaginous suspension can be prepared with a sterile non-toxic acceptable solvent or suspending medium, such as, for example, sterile fixed oils, e.g., synthetic mono- or diglycerides; and fatty acids, such as, for example, oleic acid.

Pharmaceutically acceptable carriers, adjuvants, and vehicles that may be used in the pharmaceutical compositions of this disclosure include, but are not limited to, ion exchangers, alumina, aluminum stearate, lecithin, self-emulsifying drug delivery systems (SEDDS) such as d-alpha-tocopherol polyethyleneglycol 1000 succinate, surfactants used in pharmaceutical dosage forms such as Tweens, polyethoxylated castor oil such as CREMOPHOR surfactant (BASF), or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as alpha-, beta-, and gamma-cyclodextrin, or chemically modified derivatives such as hydroxyalkylcyclodextrins, including 2- and 3-hydroxypropyl-cyclodextrins, or other solubilized derivatives may also be advantageously used to enhance delivery of compounds of the formulae described herein.

The pharmaceutically active compounds of this disclosure can be processed in accordance with conventional methods of pharmacy to produce medicinal agents for administration to patients, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

The amounts of compounds that are administered and the dosage regimen for treating a disease condition with the compounds and/or compositions of this disclosure depends on a variety of factors, including the age, weight, sex, the medical condition of the subject, the type of disease, the severity of the disease, the route and frequency of administration, and the particular compound employed. Thus, the dosage regimen may vary widely, but can be determined routinely using standard methods. A daily dose of about 0.001 to 100 mg/kg body weight, preferably between about 0.0025 and about 50 mg/kg body weight and most preferably between about 0.005 to 10 mg/kg body weight, may be appropriate. The daily dose can be administered in one to four doses per day. Other dosing schedules include one dose per week and one dose per two day cycle.

For therapeutic purposes, the active compounds of this disclosure are ordinarily combined with one or more adjuvants appropriate to the indicated route of administration. If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Pharmaceutical compositions of this disclosure comprise at least one compound of Formula (I) and optionally an additional agent selected from any pharmaceutically acceptable carrier, adjuvant, and vehicle. Alternate compositions of this disclosure comprise a compound of the Formula (I) described herein, or a prodrug thereof, and a pharmaceutically acceptable carrier, adjuvant, or vehicle.

Pharmaceutical compositions of this disclosure comprise at least one compound of Formula (II) and optionally an additional agent selected from any pharmaceutically acceptable carrier, adjuvant, and vehicle. Alternate compositions of this disclosure comprise a compound of the Formula (II) described herein, or a prodrug thereof, and a pharmaceutically acceptable carrier, adjuvant, or vehicle.

The present disclosure also encompasses an article of manufacture. As used herein, article of manufacture is intended to include, but not be limited to, kits and packages. The article of manufacture of the present disclosure, comprises: (a) a first container; (b) a pharmaceutical composition located within the first container, wherein the composition, comprises: a first therapeutic agent, comprising: a compound of the present disclosure or a pharmaceutically acceptable salt form thereof; and, (c) a package insert stating that the pharmaceutical composition can be used for the treatment of a cardiovascular disorder, diuresis, and/or natriuresis. In another embodiment, the package insert states that the pharmaceutical composition can be used in combination (as defined previously) with a second therapeutic agent to treat cardiovascular disorder, diuresis, and/or natriuresis. The article of manufacture can further comprise: (d) a second container, wherein components (a) and (b) are located within the second container and component (c) is located within or outside of the second container. Located within the first and second containers means that the respective container holds the item within its boundaries.

The first container is a receptacle used to hold a pharmaceutical composition. This container can be for manufacturing, storing, shipping, and/or individual/bulk selling. First container is intended to cover a bottle, jar, vial, flask, syringe, tube (*e.g.,* for a cream preparation), or any other container used to manufacture, hold, store, or distribute a pharmaceutical product.

The second container is one used to hold the first container and, optionally, the package insert. Examples of the second container include, but are not limited to, boxes (*e.g.,* cardboard or plastic), crates, cartons, bags (*e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached.

The package insert is a label, tag, marker, or other written sheet that recites information relating to the pharmaceutical composition located within the first container. The information recited will usually be determined by the regulatory agency governing the area in which the article of manufacture is to be sold (*e.g.,* the United States Food and Drug Administration). Preferably, the package insert specifically recites the indications for which the pharmaceutical composition has been approved. The package insert may be made of any material on which a person can read information contained therein or thereon. Preferably, the package insert is a printable material (*e.g*., paper, plastic, cardboard, foil, adhesive-backed paper or plastic) on which the desired information has been formed (*e.g.,* printed or applied).

### METHODS OF PREPARATION

The compounds of the present disclosure can be prepared in a number of ways well known to one skilled in the art of organic synthesis. The compounds of the present disclosure can be synthesized using the methods described below, together with synthetic methods known in the art of synthetic organic chemistry, or variations thereon as appreciated by those skilled in the art. Preferred methods include, but are not limited to, those described below.

The reactions and techniques described in this section are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art and alternate methods must then be used. This will sometimes require a judgment to modify the order of the synthetic steps or to select one particular process scheme over another in order to obtain a desired compound of the disclosure. It will also be recognized that another major consideration in the planning of any synthetic route in this field is the judicious choice of the protecting group used for protection of the reactive functional groups present in the compounds described in this disclosure. An authoritative account describing the many alternatives to the trained practitioner is Greene et al. (Protective Groups in Organic Synthesis, Third Edition, Wiley and Sons (1999)).

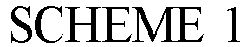

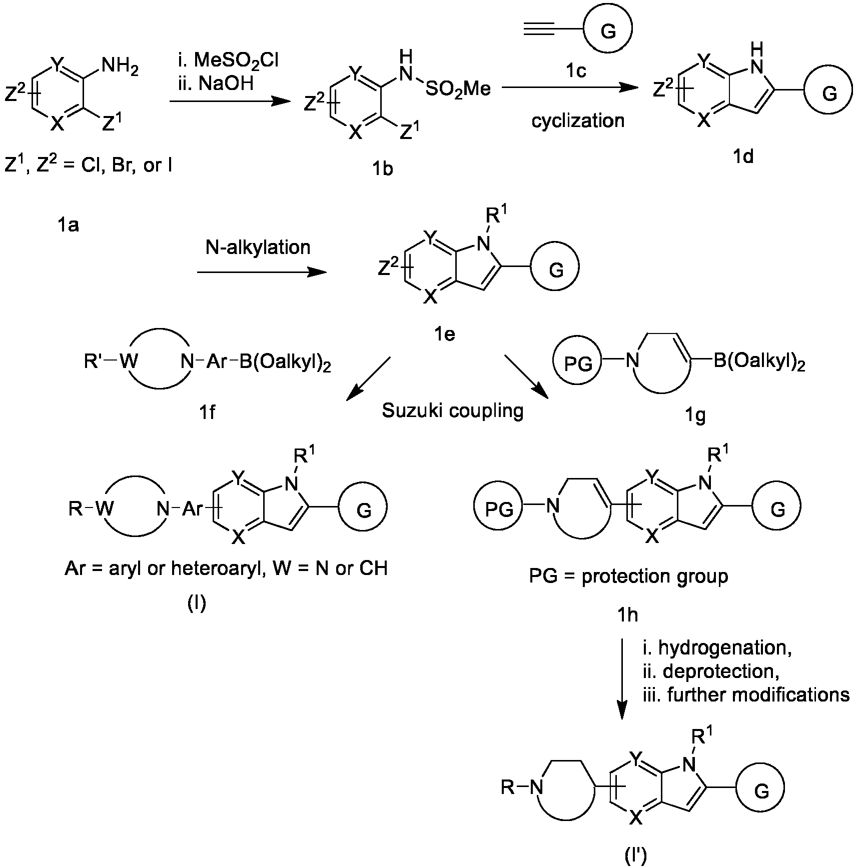

Scheme 1 describes the synthesis of compounds of Formula (I) and (I'). Reaction of 1a with methanesulfonyl chloride, followed by hydrolysis with a base such as sodium hydroxide, can provide 1b. Cyclization of 1b and alkyne 1c in the presence of a palladium catalyst such as bis(triphenylphosphine)palladium(II) chloride together with copper(I) iodide can supply 1d, which can be alkylated to give rise to 1e. Suzuki coupling of 1e with boronate ester 1f can afford the compounds of Formula (I) or the precursor to (I). Likewise, Suzuki coupling of 1e with boronate ester 1g can yield 1h, which can be converted to the compounds of Formula (I') by hydrogenation, deprotection, and further modifications.

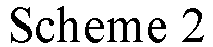

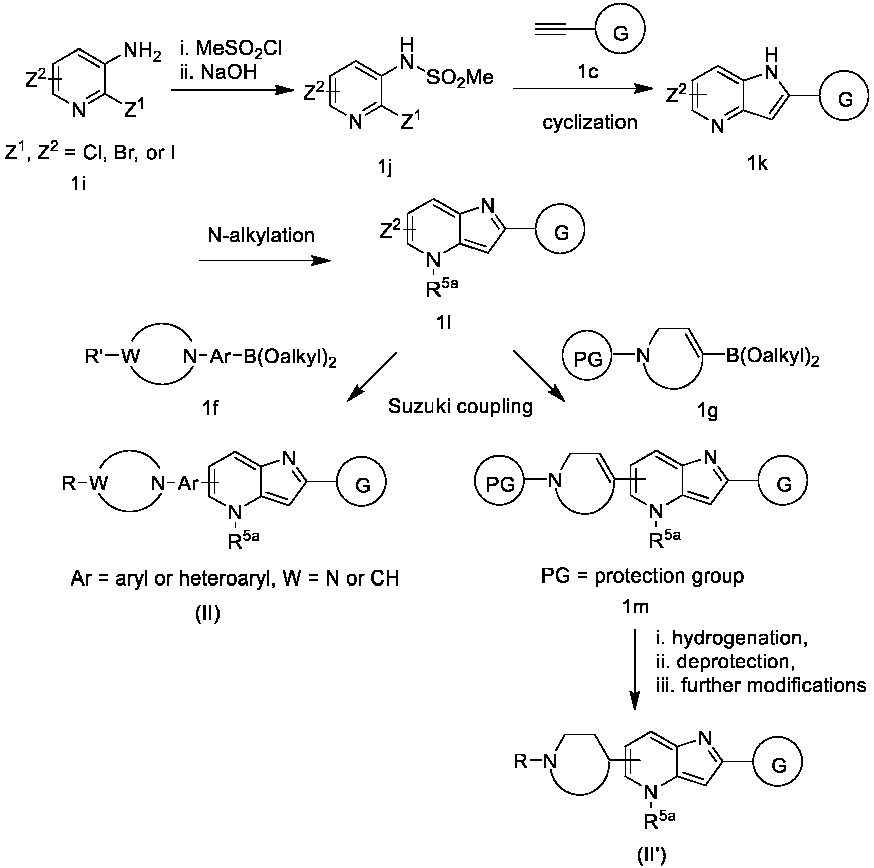

Scheme 2 describes the synthesis of compounds of Formula (II) and (II'). Reaction of 1i with methanesulfonyl chloride, followed by hydrolysis with a base such as sodium hydroxide, can provide 1bj. Cyclization of 1j and alkyne 1c in the presence of a palladium catalyst such as bis(triphenylphosphine)palladium(II) together with copper(I) iodide can supply 1k, which can be alkylated to give rise to 1l. Suzuki coupling of 1l with boronate ester 1f can afford the compounds of Formula (II) or the precursor to (II). Likewise, Suzuki coupling of 11 with boronate ester 1g can yield 1m, which can be converted to the compounds of Formula (II') by hydrogenation, deprotection, and further modifications.

### EXAMPLES

Compounds of the current disclosure and invention and intermediates used in the preparation of compounds of the current disclosure and invention can be prepared using procedures shown in the following examples and related procedures. The methods and conditions used in these examples, and the actual compounds prepared in these examples, are not meant to be limiting, but are meant to demonstrate how the compounds of the current disclosure and invention can be prepared. Starting materials and reagents used in these examples, when not prepared by a procedure described herein, are generally either commercially available, or are reported in the chemical literature, or may be prepared by using procedures described in the chemical literature. The disclosure is further defined in the following Examples. It should be understood that the Examples are given by way of illustration only. From the above discussion and the Examples, one skilled in the art can ascertain the essential characteristics of the disclosure, and can make various changes and modifications to adapt the disclosure to various uses and conditions. As a result, the present invention is not limited by the illustrative examples set forth herein below, but rather defined by the claims appended hereto.

In the examples given, the phrase "dried and concentrated" generally refers to drying of a solution in an organic solvent over either sodium sulfate or magnesium sulfate, followed by filtration and removal of the solvent from the filtrate (generally under reduced pressure and at a temperature suitable to the stability of the material being dried and concentrated).

Column chromatography was performed with pre-packed silica gel cartridges using an Isco medium pressure chromatography apparatus (Teledyne Corporation), eluting with the solvent or solvent mixture indicated. Preparative high performance liquid chromatography (HPLC) was performed using a reverse phase column (Waters Sunfire C₁₈, Waters Xbridge C₁₈, PHENOMENEX^{®} Axia C₁₈, YMC S5 ODS or the like) of a size appropriate to the quantity of material being separated, generally eluting with a gradient of increasing concentration of methanol or acetonitrile in water, also containing 0.05% or 0.1% trifluoroacetic acid or 10 mM ammonium acetate, at a rate of elution suitable to the column size and separation to be achieved. Chemical names were determined using ChemDraw Ultra, version 9.0.5 (CambridgeSoft). The following abbreviations are used:
- ACN: acetonitrile
- aq.: aqueous
- BOP: benzotriazol-1-yloxytris-(dimethylamino)-phosphonium hexafluorophosphate
- brine: saturated aqueous sodium chloride
- DMF: *N,N*-dimethylformamide
- DMSO: dimethyl sulfoxide
- DPPF: 1,1'-*bis*(diphenylphosphino)ferrocene
- Et₃N: triethyl amine
- EtOAc: ethyl acetate
- g: gram(s)
- h: hour(s)
- HPLC: High Performance Liquid Chromatography
- LCMS: Liquid Chromatography-Mass Spectroscopy
- MeI: methyl iodide
- MeOH: methanol
- Pd(PPh₃)₂Cl₂: bis(triphenylphosphine)palladium(II) dichloride
- pet ether: petroleum ether
- t-BuOK: potassium tertiary-butoxide
- TBAF: tetrabutylammonium fluoride
- TFA: trifluoroacetic acid
- THF: tetrahydrofuran

### PREPARATION

All reagents purchased from commercial sources were used without further purification unless otherwise noted. All reactions involving air or moisture sensitive reagents were performed under an inert atmosphere. Proton magnetic resonance spectra were recorded either on a Bruker Avance 400 or a JEOL Eclipse 500 spectrometer. LCMS analyses were performed on Waters Acquity UPLC system coupled with Waters TUV and SQ mass detector (Column: BEH C18 2.1 x 50 mm; Mobile Phase A: water with 0.05% TFA; Mobile Phase B: acetonitrile with 0.05% TFA; Gradient: 2-98% B over 1.6 minutes; Flow: 0.8 mL/min); HPLC analyses were performed on Shimadzu LC10-AT HPLC system coupled with SPD-10AV UV detector (Column YMC S5 Combiscreen ODS 4.6 x 50 mm; Mobile Phase A: 5:95 acetonitrile:waterwith 0.1% TFA; Mobile Phase B: 95:5 acetonitrile:waterwith 0.1% TFA; Gradient: 0-100% B over 40 minutes, then a 1-minute hold at 100% B; Flow: 1 mL/min); Preparative HPLC purifications were conducted on Shimadzu LC-8 preparative HPLC system coupled with SPD 20 UV detector. Detailed conditions are described in experimental procedures.

### EXAMPLE 1

### 2-(3,4-Dimethoxyphenyl)-6-(4-(4-isopropylpiperazin-1-yl)phenyl)-1-methyl-1H-pyrrolo[3,2-b]pyridine

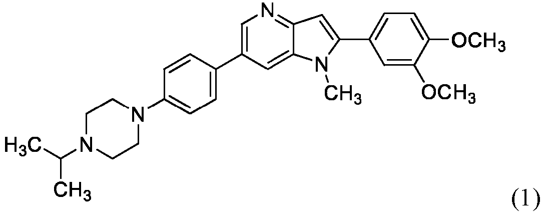

### Step 1. N-(2,5-Dibromopyridin-3-yl)-N-(methylsulfonyl)methanesulfonamide

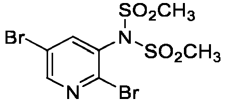

To a solution of 2,5-dibromopyridin-3-amine (3.0 g, 11.91 mmol) and triethylamine (8.30 mL, 59.5 mmol) in dichloromethane (40 mL) at 0 °C was added methanesulfonyl chloride (4.61 mL, 59.5 mmol) in dichloromethane (40 mL) over 10 min. The mixture was stirred at room temperature for 24 h. The mixture was diluted with dichloromethane (80 mL), washed with water (2 x 30 mL) and brine (30 mL), and dried over anhydrous MgSO₄. The product, N-(2,5-dibromopyridin-3-yl)-N-(methylsulfonyl)methanesulfonamide (3.69 g, 9.04 mmol, 76 % yield), was isolated as a white solid by ISCO chromatography (220 g silica gel, 10-50% ethyl acetate/hexane). LCMS (M+H)⁺ = 406.9. ¹H NMR (500 MHz, DMSO-d₆) δ 8.74 (d, *J*=2.2 Hz, 1H), 8.64 (d, *J*=2.5 Hz, 1H), 3.68 (s, 6H).

### Step 2. N-(2,5-Dibromopyridin-3-yl)methanesulfonamide

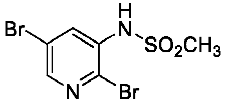

To a solution of N-(2,5-dibromopyridin-3-yl)-N-(methylsulfonyl) methanesulfonamide (3.68 g, 9.02 mmol) in tetrahydrofuran (16 mL) at room temperature was added 10% sodium hydroxide (16 ml, 44.0 mmol) over 3 min. The mixture was stirred at room temperature for 15 h, and then concentrated under vacuum to a volume of approximately 10 mL. The residue was diluted with water (5 mL) and neutralized with concentrated hydrochloric acid to pH 6-7. The precipitating product, N-(2,5-dibromopyridin-3-yl)methanesulfonamide (2.79 g, 8.45 mmol, 94 % yield), was collected as a white solid by suction filtration and dried at 50 °C under vacuum. LCMS (M+H)⁺ = 328.9. ¹H NMR (500 MHz, DMSO-d₆) δ 9.85 (br s, 1H), 8.42 (d, *J*=2.2 Hz, 1H), 8.04 (d, *J*=2.2 Hz, 1H), 3.19 (s, 3H).

### Step 3. 6-Bromo-2-(3,4-dimethoxyphenyl)-1H-pyrrolo[3,2-b]pyridine

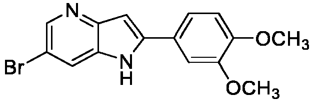

A mixture of N-(2,5-dibromopyridin-3-yl)methanesulfonamide (1.00 g, 3.03 mmol), 4-ethynyl-1,2-dimethoxybenzene (0.614 g, 3.79 mmol), bis(triphenylphosphine) palladium(II) chloride (0.128 g, 0.182 mmol) and copper(I) iodide (0.035 g, 0.182 mmol) in DMF (12 mL) was degassed and heated in a sealed vial at 100 °C for 15 h. Upon cooling to room temperature, the mixture was diluted with ethyl acetate (50 mL) and filtered through Celite. The filtrate was further diluted with ethyl acetate (150 mL), washed with water (3 x 40 mL) and brine (40 mL), and dried over anhydrous MgSO₄. After the solvent was removed under vacuum, the residue was subjected to ISCO chromatography (80 g silica gel, solid loading, 0-5% methanol/dichloromethane). The product was purified by preparative HPLC (Column: Phenomenex Luna AXIA 5u C18 30.0 x 100. Solvent A: 90% H₂O-10% methanol-0.1%TFA; Solvent B: 10% methanol-90% H₂O 0.1% TFA. Flow rate: 40 mL/min. Gradient Time: 12 minutes. Start %B: 15; Final %B: 100). The correct fractions were combined, concentrated under vacuum, basified with saturated NaHCO₃ solution to pH 10, and extracted with dichloromethane (3 x 50 mL). The combined extract was dried over anhydrous MgSO₄. Removal of the solvent under vacuum provided the 6-bromo-2-(3,4-dimethoxyphenyl)-1H-pyrrolo[3,2-b] pyridine (178 mg, 0.534 mmol, 17.63 % yield) as a white solid. LCMS (M+H)⁺ = 333.4. ¹H NMR (500 MHz, DMSO-d₆) δ 8.33 (d, *J*=1.9 Hz, 1H), 7.90 (d, *J*=1.4 Hz, 1H), 7.56-7.47 (m, 2H), 7.09 (d, *J*=8.3 Hz, 1H), 7.04 (s, 1H), 3.89 (s, 3H), 3.83 (s, 3H).

### Step 4. 6-Bromo-2-(3,4-dimethoxyphenyl)-1-methyl-1H-pyrrolo[3,2-b]pyridine

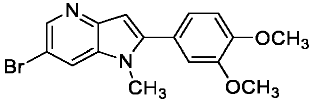

To a solution of 6-bromo-2-(3,4-dimethoxyphenyl)-1H-pyrrolo[3,2-b]pyridine (170 mg, 0.510 mmol) and iodomethane (181 mg, 1.276 mmol) in DMF (5 mL) at 0 °C was added sodium hydride (60% dispersion) (51.0 mg, 1.276 mmol) in one portion. The mixture was stirred at room temperature for 1 h. The reaction was quenched with acetic acid (0.5 mL). The reaction mixture was diluted with ethyl acetate (150 mL), washed with 1 N K₂HPO₄ solution (2 x 35 mL), water (2 x 35 mL), and brine (35 mL). The organic solution was then dried over anhydrous MgSO₄. The product, 6-bromo-2-(3,4-dimethoxyphenyl)-1-methyl-1H-pyrrolo[3,2-b]pyridine (75 mg, 0.216 mmol, 42.3 % yield), was isolated as a white solid by ISCO chromatography (40 g silica gel, 10-50% ethyl acetate). LCMS (M+H)⁺ = 347.9.

### Step 5. 2-(3,4-Dimethoxyphenyl)-6-(4-(4-isopropylpiperazin-1-yl)phenyl)-1-methyl-1H-pyrrolo[3,2-b]pyridine

A mixture of 6-bromo-2-(3,4-dimethoxyphenyl)-1-methyl-1H-pyrrolo[3,2-b] pyridine (35 mg, 0.101 mmol), 1-isopropyl-4-(4-(4,4,5,5-tetramethyl-1,3,2-dioxaborolan-2-yl)phenyl)piperazine (46.6 mg, 0.141 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3) (8.53 mg, 10.08 µmol), and potassium phosphate tribasic (0.176 mL, 0.353 mmol) in 1,4-dioxane (1.2 mL) was degassed and heated in a closed vial at 85 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 14; Final %B: 100). The correct fractions were concentrated under vacuum, basified with 1 N NaOH solution, and extracted with dichloromethane (4 x 40 mL). The combined extract was dried over anhydrous Na₂SO₄. Removal of the solvent under vacuum provided the product, 2-(3,4-dimethoxyphenyl)-6-(4-(4-isopropylpiperazin-1-yl)phenyl)-1-methyl-1H-pyrrolo[3,2-b]pyridine(34.5 mg, 0.073 mmol, 72.0 % yield), as a pale yellow solid. LCMS (M+H)⁺ = 471.2. ¹H NMR (500 MHz, chloroform-d) δ 8.73 (d, *J*=1.4 Hz, 1H), 7.76 (s, 1H), 7.62 (d, *J*=8.8 Hz, 2H), 7.12 (dd, *J*=8.3, 1.7 Hz, 1H), 7.10-7.06 (m, 3H), 7.03 (d, *J*=8.3 Hz, 1H), 6.75 (s, 1H), 3.99 (s, 3H), 3.98 (s, 3H), 3.81 (s, 3H), 3.35-3.28 (m, 4H), 2.80-2.73 (m, 5H), 1.14 (d, *J*=6.6 Hz, 6H).

### EXAMPLE 2

### 6-(4-(4-Isopropylpiperazin-1-yl)phenyl)-1-methyl-2-(4-(methylsulfonyl)phenyl)-1H-pyrrolo[3,2-b]pyridine

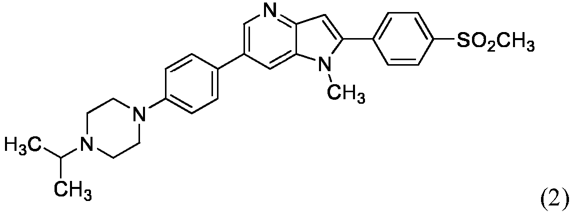

### Step 1. 6-Bromo-2-(4-(methylsulfonyl)phenyl)-1H-pyrrolo[3,2-b]pyridine

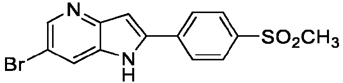

A mixture of N-(2,5-dibromopyridin-3-yl)methanesulfonamide (0.48 g, 1.455 mmol), 1-ethynyl-4-(methylsulfonyl)benzene (0.328 g, 1.818 mmol), bis(triphenylphosphine)palladium(II) chloride (0.061 g, 0.087 mmol) and copper(I) iodide (0.017 g, 0.087 mmol) in DMF (5 mL) was degassed and heated in a sealed vial at 100 °C for 15 h. Upon cooling to room temperature, the mixture was diluted with ethyl acetate (50 mL) and filtered through Celite. The filtrate was further diluted with ethyl acetate (150 mL), washed with water (3 x 40 mL) and brine (40 mL), and dried over anhydrous MgSO₄. After the solvent was removed under vacuum, the residue was subjected to ISCO chromatography (80 g silica gel, solid loading, 0-5% methanol/dichloromethane). The product (0.275 g) was purified by preparative HPLC (Column: Phenomenex Luna AXIA 5u C18 30.0 x 100. Solvent A: 90% H₂O-10% methanol-0.1%TFA; Solvent B: 10% methanol-90% H₂O 0.1% TFA. Flow rate: 40 mL/min. Gradient Time: 12 minutes. Start %B: 15; Final %B: 100). The correct fractions were combined concentrated under vacuum, basified with saturated NaHCO₃ solution to pH 10, and extracted with dichloromethane (3 x 50 mL). The combined extract was dried over anhydrous MgSO₄. Removal of the solvent under vacuum provided 6-bromo-2-(4-(methylsulfonyl)phenyl)-1H-pyrrolo[3,2-b]pyridine (119 mg, 0.339 mmol, 23.29 % yield) as a white solid. LCMS (M+H)⁺ = 351.1. ¹H NMR (500 MHz, DMSO-d₆) δ 12.21 (s, 1H), 8.43 (d, *J*=2.2 Hz, 1H), 8.24-8.18 (m, 2H), 8.08-8.03 (m, 2H), 8.02 (dd, *J*=1.9, 0.8 Hz, 1H), 7.34 (s, 1H), 3.28 (s, 3H).

### Step 2. 6-Bromo-1-methyl-2-(4-(methylsulfonyl)phenyl)-1H-pyrrolo[3,2-b]pyridine and 6-bromo-4-methyl-2-(4-(methylsulfonyl)phenyl)-4H-pyrrolo[3,2-b]pyridine

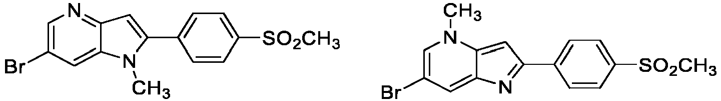

To a solution of 6-bromo-2-(4-(methylsulfonyl)phenyl)-1H-pyrrolo[3,2-b] pyridine (454 mg, 1.293 mmol) and iodomethane (459 mg, 3.23 mmol) in DMF (10 mL) at 0 °C was added sodium hydride (60% oil dispersion) (129 mg, 3.23 mmol) in one portion. The mixture was stirred at room temperature for 1 h. The reaction was quenched with acetic acid (0.370 mL, 6.46 mmol). The mixture was concentrated under vacuum to a volume of approximately 5 mL. The residue was diluted with methanol (10 mL) and injected with multiple injections to preparative HPLC (Column: Phenomenex Luna AXIA 5u C18 30.0 x 100. Solvent A: 90% H₂O-10% methanol-0.1%TFA; Solvent B: 10% methanol-90% H₂O 0.1% TFA. Flow rate: 40 mL/min. Gradient Time: 12 minutes. Start %B: 12; Final %B: 100). The fractions containing the same product were combined, concentrated under vacuum, basified with saturated NaHCO₃ solution, and extracted with dichloromethane (4 x 30 mL). The combined extracts were dried over anhydrous Na₂SO₄. Removal of the solvent under vacuum provided 6-bromo-1-methyl-2-(4-(methylsulfonyl)phenyl)-1H-pyrrolo[3,2-b]pyridine (154 mg, 0.422 mmol, 32.6 % yield) and 6-bromo-4-methyl-2-(4-(methylsulfonyl)phenyl)-4H-pyrrolo[3,2-b]pyridine(64 mg, 0.175 mmol, 13.56 % yield). Both products were light yellow solids.

6-bromo-1-methyl-2-(4-(methylsulfonyl)phenyl)-1H-pyrrolo[3,2-b]pyridine: LCMS (M+H)⁺ = 365.0. ¹H NMR (500 MHz, ACETONITRILE-d₃) δ 8.50 (d, *J*=1.9 Hz, 1H), 8.11-8.06 (m, 3H), 7.90-7.84 (m, 2H), 6.85 (d, *J*=0.6 Hz, 1H), 3.79 (s, 3H), 3.16 (s, 3H).

6-bromo-4-methyl-2-(4-(methylsulfonyl)phenyl)-4H-pyrrolo[3,2-b]pyridine: LCMS (M+H)⁺ = 365.1. ¹H NMR (500 MHz, ACETONITRILE-d₃) δ 8.42-8.37 (m, 2H), 8.25 (s, 1H), 8.05 (d, *J*=1.3 Hz, 1H), 8.02-7.98 (m, 2H), 7.09 (d, *J*=0.6 Hz, 1H), 4.19 (s, 3H), 3.12 (s, 3H).

### Step 3. 6-(4-(4-Isopropylpiperazin-1-yl)phenyl)-1-methyl-2-(4-(methylsulfonyl)phenyl)-1H-pyrrolo[3,2-b]pyridine

A mixture of 6-bromo-1-methyl-2-(4-(methylsulfonyl)phenyl)-1H-pyrrolo[3,2-b] pyridine (21 mg, 0.057 mmol), 1-isopropyl-4-(4-(4,4,5,5-tetramethyl-1,3,2-dioxaborolan-2-yl)phenyl)piperazine (26.6 mg, 0.080 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3) (4.87 mg, 5.75 µmol), and potassium phosphate tribasic (0.101 mL, 0.201 mmol) in 1,4-dioxane (0.8 mL) was degassed and heated in a closed vial at 85 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 14; Final %B: 100). The correct fractions were concentrated under vacuum, basified with 1 N NaOH solution, and extracted with dichloromethane (4 x 40 mL). The combined extract was dried over anhydrous Na₂SO₄. Removal of the solvent under vacuum provided 6-(4-(4-isopropylpiperazin-1-yl)phenyl)-1-methyl-2-(4-(methylsulfonyl)phenyl)-1H-pyrrolo[3,2-b]pyridine (14 mg, 0.028 mmol, 49.3 % yield) as a light pale yellow solid. LCMS (M+H)⁺ = 489.5. ¹H NMR (500 MHz, DMSO-d₆) δ 8.71 (d, J=1.9 Hz, 1H), 8.18 (d, *J*=1.1 Hz, 1H), 8.09 (d, *J*=8.3 Hz, 2H), 7.97 (d, *J*=8.5 Hz, 2H), 7.70 (d, *J*=8.8 Hz, 2H), 7.07 (d, *J*=8.8 Hz, 2H), 6.90 (s, 1H), 3.90 (s, 3H), 3.30 (m, 3H), 3.24-3.18 (m, 4H), 2.70 (dt, *J*=13.1, 6.4 Hz, 1H), 2.64-2.59 (m, 4H), 1.03 (d, *J*=6.6 Hz, 6H).

### EXAMPLE 3

### 6-(4-(4-isopropylpiperazin-1-yl)phenyl)-4-methyl-2-(4-(methylsulfonyl)phenyl)-4H-pyrrolo[3,2-b]pyridine

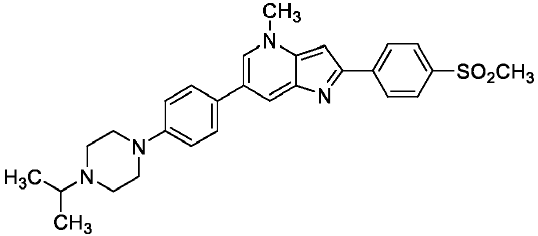

A mixture of 6-bromo-4-methyl-2-(4-(methylsulfonyl)phenyl)-4H-pyrrolo[3,2-b] pyridine (step 2 in Example 2) (22 mg, 0.060 mmol), 1-isopropyl-4-(4-(4,4,5,5-tetramethyl-1,3,2-dioxaborolan-2-yl)phenyl)piperazine (26.9 mg, 0.081 mmol), (2-dicyclohexylphosphino-2',4',6'-triisopropyl-1,1'-biphenyl)[2-(2'-amino-1,1'-biphenyl)] palladium(II) methanesulfonate (XPhos-Pd-G3) (5.10 mg, 6.02 µmol), and potassium phosphate tribasic (0.105 mL, 0.211 mmol) in 1,4-dioxane (0.8 mL) was degassed and heated in a closed vial at 85 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 10; Final %B: 100). The correct fractions were concentrated under vacuum, basified with 1 N NaOH, and extracted with dichloromethane (4 x 40 mL). The combined extract was dried over anhydrous Na₂SO₄. Removal of the solvent under vacuum provided the product, 6-(4-(4-isopropylpiperazin-1-yl)phenyl)-4-methyl-2-(4-(methylsulfonyl)phenyl)-4H-pyrrolo[3,2-b]pyridine (12 mg, 0.024 mmol, 40.0 % yield), as a light pale yellow solid. LCMS (M+H)⁺ = 489.4. ¹H NMR (500 MHz, DMSO-d₆) δ 8.45 (d, *J*=0.9 Hz, 1H), 8.40 (d, *J*=8.5 Hz, 2H), 8.36 (s, 1H), 7.98 (d, *J*=8.5 Hz, 2H), 7.65 (d, *J*=8.8 Hz, 2H), 7.16 (s, 1H), 7.07 (d, *J*=8.8 Hz, 2H), 4.28 (s, 3H), 3.26 (s, 3H), 3.23-3.17 (m, 4H), 2.70 (quin, *J*=6.5 Hz, 1H), 2.63-2.58 (m, 4H), 1.03 (d, *J*=6.6 Hz, 6H).

### EXAMPLE 4

### 1-(4-(4-(2-(3,4-Dimethoxyphenyl)-1-methyl-1H-pyrrolo[3,2-b]pyridin-6-yl)phenyl) piperazin-1-yl)-2-methylpropan-2-ol

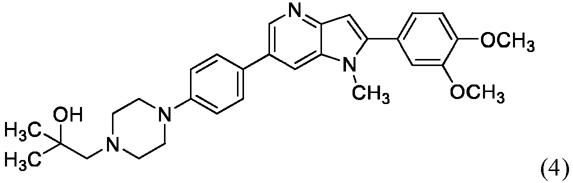

### Step 1. 2-Methyl-1-(4-(4-(4,4,5,5-tetramethyl-1,3,2-dioxaborolan-2-yl)phenyl)piperazin-1-yl)propan-2-ol

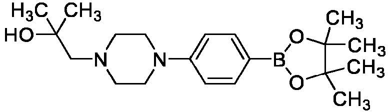

To a mixture of 1-(4-(4,4,5,5-tetramethyl-1,3,2-dioxaborolan-2-yl)phenyl) piperazine (150 mg, 0.520 mmol) and potassium carbonate (108 mg, 0.781 mmol) in MeOH (2 mL) at 0 °C was added 2,2-dimethyloxirane (56.3 mg, 0.781 mmol) in DMF (0.2 mL) in one portion. The mixture was stirred at room temperature for 28 h, diluted with ethyl acetate (10 mL), and filtered through Celite. The filtrate was diluted with ethyl acetate (60 mL), washed with water (2 x 20 mL) and brine (20 mL), and dried over anhydrous MgSO₄. The product, 2-methyl-1-(4-(4-(4,4,5,5-tetramethyl-1,3,2-dioxaborolan-2-yl)phenyl)piperazin-1-yl)propan-2-ol (113 mg, 0.314 mmol, 60.3 % yield), was isolated as a white solid by ISCO chromatography (24 g silica gel, solid loading, 1-10% ethyl acetate/hexane). LCMS (M+H)⁺ = 361.3. ¹H NMR (500 MHz, chloroform-d) δ 7.73 (d, *J*=8.8 Hz, 2H), 6.91 (d, *J*=8.5 Hz, 2H), 3.35-3.25 (m, 4H), 2.88-2.76 (m, 4H), 2.41 (s, 2H), 1.35 (s, 12H), 1.22 (s, 6H).

### Step 2. 1-(4-(4-(2-(3,4-Dimethoxyphenyl)-1-methyl-1H-pyrrolo[3,2-b]pyridin-6-yl) phenyl)piperazin-1-yl)-2-methylpropan-2-ol

A mixture of 6-bromo-2-(3,4-dimethoxyphenyl)-1-methyl-1H-pyrrolo[3,2-b] pyridine (20 mg, 0.058 mmol), 2-methyl-1-(4-(4-(4,4,5,5-tetramethyl-1,3,2-dioxaborolan-2-yl)phenyl)piperazin-1-yl)propan-2-ol (28.0 mg, 0.078 mmol), (2-dicyclohexylphosphino-2',4',6'-triisopropyl-1,1'-biphenyl)[2-(2'-amino-1,1'-biphenyl)] palladium(II) methanesulfonate (XPhos-Pd-G3) (4.88 mg, 5.76 µmol), and potassium phosphate tribasic (0.101 mL, 0.202 mmol) in 1,4-dioxane (0.8 mL) was degassed and heated in a closed vial at 85 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 15; Final %B: 100). The correct fractions were concentrated under vacuum, basified with 1 N NaOH, and extracted with dichloromethane (4 x 40 mL). The combined extract was dried over anhydrous Na₂SO₄. Removal of the solvent under vacuum provided 1-(4-(4-(2-(3,4-dimethoxyphenyl)-1-methyl-1H-pyrrolo[3,2-b]pyridin-6-yl)phenyl)piperazin-1-yl)-2-methylpropan-2-ol (18 mg, 0.036 mmol, 61.8 % yield) as a pale solid. LCMS (M+H)⁺ = 501.5. ¹H NMR (500 MHz, CHLOROFORM-d) δ 8.73 (d, *J*=1.9 Hz, 1H), 7.76 (d, *J*=1.1 Hz, 1H), 7.62 (d, *J*=8.8 Hz, 2H), 7.13 (dd, *J*=8.3, 1.9Hz, 1H), 7.10-7.05 (m, 3H), 7.03 (d, *J*=8.3 Hz, 1H), 6.75 (s, 1H), 3.99 (s, 3H), 3.98 (s, 3H), 3.82 (s, 3H), 3.33-3.28 (m, 4H), 2.90-2.85 (m, 4H), 2.45 (s, 2H), 1.24 (s, 6H).

### EXAMPLE 5

### (4-(2-(3,4-Dimethoxyphenyl)-1-methyl-1H-pyrrolo[3,2-b]pyridin-6-yl)phenyl)(4-isopropylpiperazin-1-yl)methanone

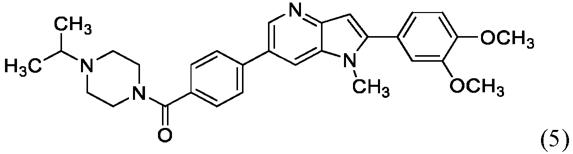

### Step 1. (4-Isopropylpiperazin-1-yl)(4-(4,4,5,5-tetramethyl-1,3,2-dioxaborolan-2-yl) phenyl)methanone

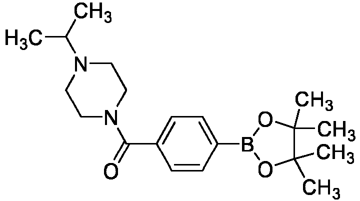

A mixture of 4-(4,4,5,5-tetramethyl-1,3,2-dioxaborolan-2-yl)benzoic acid (100 mg, 0.403 mmol),1-isopropylpiperazine (64.6 mg, 0.504 mmol), benzotriazol-1-yloxytris(dimethylamino)phosphonium hexafluorophosphate (BOP) (267 mg, 0.605 mmol), and N,N-diisopropylethylamine (0.282 mL, 1.612 mmol) in DMF (1 mL) was stirred at room temperature for 2 h. The mixture was diluted with ethyl acetate (50 mL), washed with water (3 x 15 mL) and brine (15 mL) successively, and dried over anhydrous MgSO₄. The product, (4-isopropylpiperazin-1-yl)(4-(4,4,5,5-tetramethyl-1,3,2-dioxaborolan-2-yl)phenyl)methanone (92 mg, 0.257 mmol, 63.7 % yield), was isolated as a white solid by ISCO chromatography (24 g silica gel, solid loading, 0-8% methanol/dichloromethane). LCMS (M+H)⁺ = 359.1.

### Step 2. (4-(2-(3,4-Dimethoxyphenyl)-1-methyl-1H-pyrrolo[3,2-b]pyridin-6-yl)phenyl)(4-isopropylpiperazin-1-yl)methanone

A mixture of 6-bromo-2-(3,4-dimethoxyphenyl)-1-methyl-1H-pyrrolo[3,2-b] pyridine (15 mg, 0.043 mmol), (4-isopropylpiperazin-1-yl)(4-(4,4,5,5-tetramethyl-1,3,2-dioxaborolan-2-yl)phenyl)methanone (19.35 mg, 0.054 mmol), (2-dicyclohexylphosphino-2',4',6'-triisopropyl-1,1'-biphenyl)[2-(2'-amino-1,1'-biphenyl)] palladium(II) methanesulfonate (XPhos-Pd-G3) (3.66 mg, 4.32 µmol), and potassium phosphate tribasic (0.076 mL, 0.151 mmol) in 1,4-dioxane (0.8 mL) was degassed and heated in a closed vial at 85 °C for 15 h. Upon cooling to room temperature, the reaction mixture was diluted with methanol, filtered through an acrodisc, and injected to preparative HPLC (Column: Waters Symmetry Shield 5u 19 X 100mm. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 14; Final %B: 100). The correct fractions were combined, concentrated under vacuum, basified with 1 N NaOH solution, and extracted with dichloromethane (4 x 35 mL). The combined extract was dried over anhydrous Na₂SO₄. Removal of the solvent under vacuum provided (4-(2-(3,4-dimethoxyphenyl)-1-methyl-1H-pyrrolo[3,2-b]pyridin-6-yl)phenyl)(4-isopropylpiperazin-1-yl)methanone (2.4 mg, 4.67 µmol, 10.81 % yield) as a colorless film. LCMS (M+H)⁺ = 499.3. ¹H NMR (500 MHz, chloroform-d) δ 8.76 (d, J=1.7 Hz, 1H), 7.81 (d, *J*=0.8 Hz, 1H), 7.74 (d, *J*=8.3 Hz, 2H), 7.57 (d, *J*=8.3 Hz, 2H), 7.14 (dd, *J*=8.1, 1.8 Hz, 1H), 7.09 (d, *J*=1.7 Hz, 1H), 7.04 (d, *J*=8.3 Hz, 1H), 6.78 (s, 1H), 4.00 (s, 3H), 3.98 (s, 3H), 3.91-3.85 (m, 2H), 3.84 (s, 3H), 3.56 (br s, 2H), 2.78 (dt, *J*=12.9, 6.4 Hz, 1H), 2.70-2.48 (m, 4H), 1.10 (d, *J*=6.6 Hz, 6H).

### EXAMPLE 6

### 6-(4-(4-Isobutylpiperazin-1-yl)phenyl)-1-methyl-2-(4-(methylsulfonyl)phenyl)-1H-pyrrolo[3,2-b]pyridine

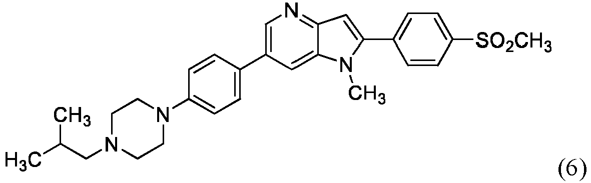

### Step 1. 1-Isobutyl-4-(4-(4,4,5,5-tetramethyl-1,3,2-dioxaborolan-2-yl)phenyl)piperazine

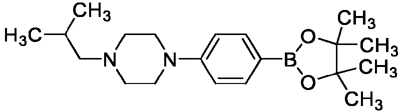

To a solution of 1-(4-(4,4,5,5-tetramethyl-1,3,2-dioxaborolan-2-yl)phenyl) piperazine (0.715 g, 2.481 mmol), isobutyraldehyde (0.789 mL, 8.68 mmol), magnesium sulfate (5.97 g, 49.6 mmol), and acetic acid (1.420 mL, 24.81 mmol) in DMF (15 mL) at room temperature was added sodium triacetoxyborohydride (2.366 g, 11.16 mmol) in one portion. The mixture was stirred at room temperature for 60 h. The heterogeneous mixture was diluted with ethyl acetate (20 mL) and filtered through Celite. The filtrate was further diluted with ethyl acetate (80 mL), washed with saturated NaHCO₃ solution (25 mL), water (2 x 25 mL) and brine (25 mL), and dried over anhydrous MgSO₄. The product, 1-isobutyl-4-(4-(4,4,5,5-tetramethyl-1,3,2-dioxaborolan-2-yl)phenyl)piperazine (0.444 g, 1.290 mmol, 52.0 % yield), was isolated as a white solid by ISCO chromatography (40 g silica gel, solid loading, 0-5% methanol/dichloromethane). LCMS (M+H)⁺ = 345.3. ¹H NMR (500 MHz, chloroform-d) δ 7.72 (d, *J*=8.3 Hz, 2H), 6.91 (d, *J*=8.3 Hz, 2H), 3.37-3.20 (m, 4H), 2.65-2.49 (m, 4H), 2.15 (d, *J*=7.4 Hz, 2H), 1.84 (dt, *J*=13.5, 6.7 Hz, 1H), 1.35 (s, 12H), 0.95 (d, *J*=6.6 Hz, 6H).

### Step 2. 6-(4-(4-isobutylpiperazin-1-yl)phenyl)-1-methyl-2-(4-(methylsulfonyl)phenyl)-1H-pyrrolo[3,2-b]pyridine

A mixture of 6-bromo-1-methyl-2-(4-(methylsulfonyl)phenyl)-1H-pyrrolo[3,2-b] pyridine (40 mg, 0.110 mmol), 1-isobutyl-4-(4-(4,4,5,5-tetramethyl-1,3,2-dioxaborolan-2-yl)phenyl)piperazine (52.8 mg, 0.153 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3 (9.27 mg, 10.95 µmol), and potassium phosphate tribasic (0.192 mL, 0.383 mmol) in 1,4-dioxane (1.5 mL) was degassed and heated in a closed vial at 85 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 11; Final %B: 100). The correct fractions were concentrated under vacuum, basified with 1 N NaOH solution, and extracted with dichloromethane (4 x 40 mL). The combined extract was dried over anhydrous Na₂SO₄. Removal of the solvent under vacuum provided 6-(4-(4-isobutylpiperazin-1-yl)phenyl)-1-methyl-2-(4-(methylsulfonyl)phenyl)-1H-pyrrolo[3,2-b]pyridine (30 mg, 0.058 mmol, 53.4 % yield) as a pale yellow solid. LCMS (M+H)⁺ = 503.4. ¹H NMR (400 MHz, chloroform-d) δ 8.78 (d, *J*=1.8 Hz, 1H), 8.13-8.07 (m, 2H), 7.82-7.76 (m, 3H), 7.62 (d, *J*=8.6 Hz, 2H), 7.08 (d, *J*=8.8 Hz, 2H), 6.89 (s, 1H), 3.85 (s, 3H), 3.33-3.28 (m, 4H), 3.16 (s, 3H), 2.65-2.58 (m, 4H), 2.19 (d, *J*=7.4 Hz, 2H), 1.86 (dt, *J*=13.5, 6.7 Hz, 1H), 0.97 (d, J=6.7 Hz, 6H).

### EXAMPLE 7

### 2-(3,4-Dimethoxyphenyl)-6-(4-(4-isopropylpiperazin-1-yl)phenyl)-1-methyl-1H-pyrrolo[2,3-b]pyridine (reference compound)

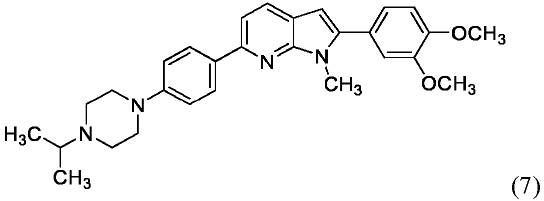

### Step 1. N-(6-Chloro-3-iodopyridin-2-yl)-N-(methylsulfonyl)methanesulfonamide

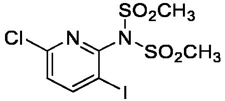

To a solution of 6-chloro-3-iodopyridin-2-amine (2.0 g, 7.86 mmol) in pyridine (20 mL) at 0 °C was added methanesulfonyl chloride (3.04 mL, 39.3 mmol) over 10 min. The mixture was stirred at room temperature for 36 h. The mixture was diluted with dichloromethane (50 mL) and filtered through Celite. The filtrate was concentrated under vacuum to dryness. To the residue was added ethyl acetate (180 mL) and the mixture was filtered through Celite. The filtrate was washed with water (3 x 40 mL) and brine (40 mL) successively, and dried over anhydrous MgSO₄. The product, N-(6-chloro-3-iodopyridin-2-yl)-N-(methylsulfonyl)methanesulfonamide (1.89 g, 4.60 mmol, 58.6 % yield), was isolated as a beige solid by ISCO chromatography (220 g silica gel, 10-50% ethyl acetate/hexane). LCMS (M+H)⁺ = 410.9.

### Step 2. N-(6-Chloro-3-iodopyridin-2-yl)methanesulfonamide

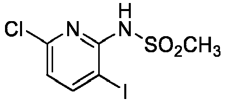

To a suspension of N-(6-chloro-3-iodopyridin-2-yl)-N-(methylsulfonyl) methanesulfonamide (1.89 g, 4.60 mmol) in THF (11 mL) at room temperature was added 10% sodium hydroxide (11 ml, 30.3 mmol) over 3 min. The mixture was stirred at room temperature for 14 h, and then concentrated under vacuum to a volume of approximately 10 mL. The residue was diluted with water (10 mL) and neutralized with concentrated hydrochloric acid to pH 6-7. The precipitating product, N-(6-chloro-3-iodopyridin-2-yl)methanesulfonamide (1.46 g, 4.39 mmol, 95 % yield), was collected as a beige solid by suction filtration and dried at 50 °C under vacuum. LCMS (M+H)⁺ = 332.8. ¹H NMR (500 MHz, DMSO-d₆) δ 10.06 (br s, 1H), 8.28 (d, *J*=8.3 Hz, 1H), 7.08 (d, *J*=8.3 Hz, 1H), 3.34 (s, 3H).

### Step 3. 6-Chloro-2-(3,4-dimethoxyphenyl)-1H-pyrrolo[2,3-b]pyridine

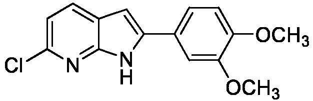

A mixture of N-(6-chloro-3-iodopyridin-2-yl)methanesulfonamide (600 mg, 1.804 mmol), 4-ethynyl-1,2-dimethoxybenzene (439 mg, 2.71 mmol), bis(triphenylphosphine) palladium(II) chloride (76 mg, 0.108 mmol), and copper(I) iodide (20.62 mg, 0.108 mmol) in DMF (8 mL) was degassed and heated in a sealed vial at 100 °C for 15 h. Upon cooling to room temperature, the mixture was diluted with ethyl acetate (30 mL) and filtered through Celite. The filtrate was concentrated under vacuum to near dryness. The residue was diluted with ethyl acetate (150 mL), washed with water (2 x 30 mL) and brine (30 mL) successively, and dried over anhydrous MgSO₄. The product, 6-chloro-2-(3,4-dimethoxyphenyl)-1H-pyrrolo[2,3-b]pyridine (154 mg, 0.533 mmol, 29.6 % yield), was isolated as a tan solid by ISCO chromatography (80 g silica gel, 0-5% methanol/dichloromethane). LCMS (M+H)⁺ = 289.1. ¹H NMR (500 MHz, DMSO-d₆) δ 12.30 (s, 1H), 7.95 (d, *J*=8.3 Hz, 1H), 7.52 (d, *J*=1.9 Hz, 1H), 7.48 (dd, *J*=8.4, 2.1 Hz, 1H), 7.10 (d, *J*=8.3 Hz, 1H), 7.06 (d, *J*=8.3 Hz, 1H), 6.90 (d, *J*=1.9 Hz, 1H), 3.87 (s, 3H), 3.81 (s, 3H).

### Step 4. 6-Chloro-2-(3,4-dimethoxyphenyl)-1-methyl-1H-pyrrolo[2,3-b]pyridine

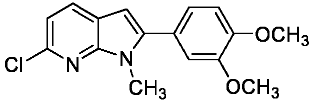

To a solution of 6-chloro-2-(3,4-dimethoxyphenyl)-1H-pyrrolo[2,3-b]pyridine (152 mg, 0.526 mmol) and iodomethane (187 mg, 1.316 mmol) in DMF (5 mL) at 0 °C was added sodium hydride (60% oil dispersion) (52.6 mg, 1.316 mmol) in one portion. The mixture was stirred at room temperature for 1 h. The reaction was quenched with acetic acid (0.5 mL). The mixture was diluted with ethyl acetate (150 mL). The resulting solution was washed with 1 N K₂HPO₄ solution (2 x 35 mL), water (2 x 35 mL) and brine (35 mL) successively, and dried over anhydrous MgSO₄. The product, 6-chloro-2-(3,4-dimethoxyphenyl)-1-methyl-1H-pyrrolo[2,3-b]pyridine (175 mg, 0.578 mmol, 110 % yield), was isolated as a tan solid by ISCO chromatography (40 g silica gel, 10-50% ethyl acetate). LCMS (M+H)⁺ = 303.3.

### Step 5. 2-(3,4-Dimethoxyphenyl)-6-(4-(4-isopropylpiperazin-1-yl)phenyl)-1-methyl-1H-pyrrolo[2,3-b]pyridine

A mixture of 6-chloro-2-(3,4-dimethoxyphenyl)-1-methyl-1H-pyrrolo[2,3-b] pyridine (25 mg, 0.074 mmol), 1-isopropyl-4-(4-(4,4,5,5-tetramethyl-1,3,2-dioxaborolan-2-yl)phenyl)piperazine (30.7 mg, 0.093 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3) (6.29 mg, 7.43 µmol), and potassium phosphate tribasic (0.130 mL, 0.260 mmol) in 1,4-dioxane (1 mL) was degassed and heated in a closed vial at 80 °C for 15 h. Upon cooling to room temperature, the reaction mixture was diluted with methanol, filtered through an acrodisc, and injected to preparative HPLC (Column: Phenomenex Luna AXIA 5u C18 21.2 x 100. Solvent A: 90% H₂O-10% methanol-0.1%TFA; Solvent B: 10% methanol-90% H₂O 0.1% TFA. Flow rate: 20 mL/min. Gradient Time: 15 minutes. Start %B: 22; Final %B: 100). The correct fractions were concentrated under vacuum, basified with 1 N NaOH solution, and extracted with dichloromethane (4 x 40 mL). The combined extract was dried over anhydrous Na₂SO₄. Removal of the solvent under vacuum provided 2-(3,4-dimethoxyphenyl)-6-(4-(4-isopropylpiperazin-1-yl) phenyl)-1-methyl-1H-pyrrolo[2,3-b]pyridine (19 mg, 0.040 mmol, 53.8 % yield) as a pale solid. LCMS (M+H)⁺ = 471.4. ¹H NMR (500 MHz, DMSO-d₆) δ 8.06 (d, *J*=9.1 Hz, 2H), 7.95 (d, *J*=8.2 Hz, 1H), 7.62 (d, *J*=8.2 Hz, 1H), 7.24-7.19 (m, 2H), 7.12 (d, *J*=8.2 Hz, 1H), 7.04 (d, *J*=9.1 Hz, 2H), 6.56 (s, 1H), 3.89 (s, 3H), 3.86 (s, 3H), 3.84 (s, 3H), 3.25-3.19 (m, 4H), 2.74-2.66 (m, 1H), 2.63-2.58 (m, 4H), 1.03 (d, *J*=6.6 Hz, 6H).

Examples 8-19 in Table 1 were prepared according to the synthetic routes described for the preparation of example 1-7.

**TABLE 1**

| Ex. No. | Structure |
|---|---|
| 8 | 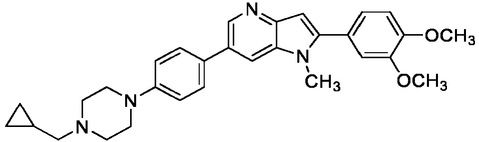 |
| 9 | 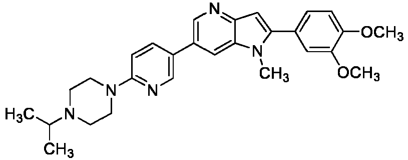 |
| 10 | 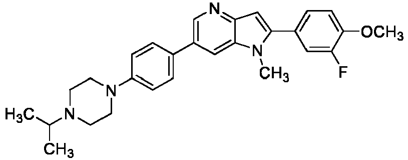 |
| 11 | 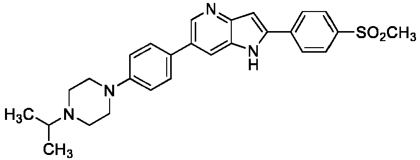 |
| 12 | 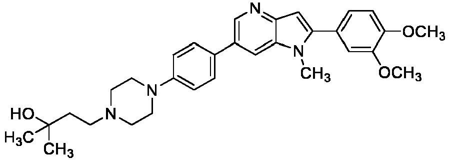 |
| 13 | 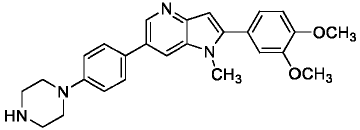 |
| 14 | 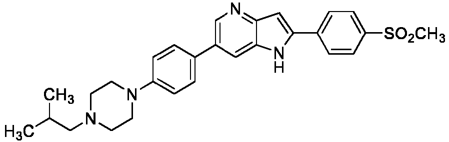 |
| 15 | 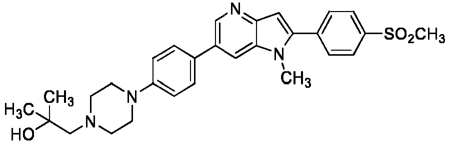 |
| 16 | 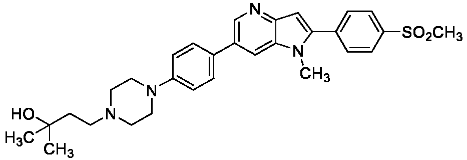 |
| 17 | 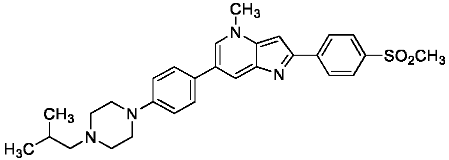 |
| 18 | 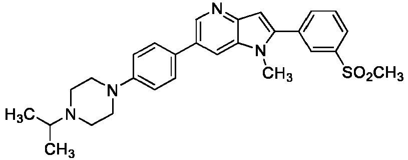 |

| Ex. No. | Analytical data |
|---|---|
| 8 | LCMS (M+H)⁺ = 483.2. ¹H NMR (500 MHz, chloroform-d) δ 8.73 (d, *J*=1.9 Hz, 1H), 7.76 (d, *J*=0.8 Hz, 1H), 7.62 (d, *J*=8.5 Hz, 2H), 7.13 (dd, *J*=8.3, 1.9 Hz, 1H), 7.1 0-7.07 (m, 3H), 7.03 (d, *J*=8.5 Hz, 1H), 6.75 (s, 1H), 3.99 (s, 3H), 3.98 (s, 3H), 3.82 (s, 3H), 3.40-3.31 (m, 4H), 2.81-2.73 (m, 4H), 2.37 (d, *J*=6.6 Hz, 2H), 1.01-0.91 (m, 1H), 0.65-0.55 (m, 2H), 0.19 (q, *J=*5.0 Hz, 2H) |
| 9 | LCMS (M+H)⁺ = 472.5. ¹H NMR (500 MHz, chloroform-d) δ 8.68 (d, *J*=1.9 Hz, 1H), 8.54 (d, *J*=2.5 Hz, 1H), 7.82 (dd, *J*=8.5, 2.5 Hz, 1H), 7.72 (d, *J*=1.4 Hz, 1H), 7.12 (dd, *J*=8.0, 1.9 Hz, 1H), 7.08 (d, *J*=1.7 Hz, 1H), 7.03 (d, *J*=8.3 Hz, 1H), 6.80 (d, *J*=8.8 Hz, 1H), 6.75 (s, 1H), 3.99 (s, 3H), 3.98 (s, 3H), 3.82 (s, 3H), 3.69-3.63 (m, 4H), 2.78 (dt, *J*=13.0, 6.6 Hz, 1H), 2.73-2.68 (m, 4H), 1.14 (d, *J*=6.3 Hz, 6H) |
| 10 | LCMS (M+H)⁺ = 459.4. ¹H NMR (500 MHz, chloroform-d) δ 8.74 (d, *J*=1.7 Hz, 1H), 7.76 (d, *J*=1.1 Hz, 1H), 7.61 (d, *J*=8.8 Hz, 2H), 7.34-7.30 (m, 1H), 7.30-7.28 (m, 2H), 7.14-7.06 (m, 3H), 6.75 (s, 1H), 4.00 (s, 3H), 3.81 (s, 3H), 3.35-3.29 (m, 4H), 2.78-2.74 (m, 4H), 1.15 (d, *J*=6.6 Hz, 6H) |
| 11 | LCMS (M+H)⁺ = 475.4. ¹H NMR (500 MHz, DMSO-d₆) δ 12.02 (s, 1H), 8.65 (d, *J*=2.2 Hz, 1H), 8.21 (d, *J*=8.5 Hz, 2H), 8.04 (d, *J*=8.5 Hz, 2H), 7.88 (d, *J*=1.1 Hz, 1H), 7.62 (d, *J*=8.8 Hz, 2H), 7.32 (s, 1H), 7.07 (d, *J*=8.8 Hz, 2H), 3.28 (s, 3H), 3.23-3.17 (m, 4H), 2.74-2.66 (m, 1H), 2.64-2.59 (m, 4H), 1.03 (d, *J*=6.6 Hz, 6H) |
| 12 | LCMS (M+H)⁺ = 515.5. ¹H NMR (500 MHz, chloroform-d) δ 8.73 (d, *J*=1.7 Hz, 1H), 7.75 (d, *J*=0.8 Hz, 1H), 7.62 (d, *J*=8.5 Hz, 2H), 7.13 (dd, *J*=8.3, 1.9 Hz, 1H), 7.10-7.00 (m, 4H), 6.75 (s, 1H), 3.99 (s, 3H), 3.98 (s, 3H), 3.81 (s, 3H), 3.34-3.26 (m, 4H), 2.76 (br d, *J*=5.2 Hz, 6H), 1.75-1.69 (m, 2H), 1.29 (s, 6H) |
| 13 | LCMS (M+H)⁺ = 529.4. ¹H NMR (500 MHz, methanol-d₄) δ 8.57 (d, *J*=1.7 Hz, 1H), 8.05 (s, 1H), 7.67 (d, *J*=8.8 Hz, 2H), 7.21-7.17 (m, 2H), 7.16-7.12 (m, 3H), 6.64 (s, 1H), 3.94 (s, 3H), 3.94 (s, 3H), 3.87 (s, 3H), 3.27-3.24 (m, 4H), 3.07-3.04 (m, 4H) |
| 14 | LCMS (M+H)⁺ = 489.4. ¹H NMR (500 MHz, DMSO-d₆) δ 12.02 (s, 1H), 8.65 (d, *J*=1.9 Hz, 1H), 8.21 (d, *J*=8.5 Hz, 2H), 8.04 (d, *J*=8.5 Hz, 2H), 7.88 (d, *J*=1.4 Hz, 1H), 7.62 (d, *J*=8.8 Hz, 2H), 7.32 (s, 1H), 7.07 (d, *J*=8.8 Hz, 2H), 3.28 (s, 3H), 3.24-3.19 (m, 4H), 2.54-2.50 (m, 4H), 2.11 (d, *J*=7.2 Hz, 2H), 1.83 (dt, *J*=13.5, 6.8 Hz, 1H), 0.90 (d, *J*=6.3 Hz, 6H) |
| 15 | LCMS (M+H)⁺ = 519.4. ¹H NMR (500 MHz, chloroform-d) δ 8.78 (d, *J*=1.9 Hz, 1H), 8.11 (d, *J*=8.2 Hz, 2H), 7.85-7.73 (m, 3H), 7.62 (d, *J*=8.8 Hz, 2H), 7.07 (d, *J*=8.5 Hz, 2H), 6.90 (s, 1H), 3.86 (s, 3H), 3.36-3.26 (m, 4H), 3.16 (s, 3H), 2.92-2.83 (m, 4H), 2.45 (s, 2H), 1.24 (s, 6H) |
| 16 | LCMS (M+H)⁺ = 533.4. ¹H NMR (500 MHz, chloroform-d) δ 8.78 (d, *J*=1.9 Hz, 1H), 8.14-8.07 (m, 2H), 7.82-7.76 (m, 3H), 7.65-7.59 (m, 2H), 7.06 (d, *J*=8.8 Hz, 2H), 6.90 (d, *J*=0.6 Hz, 1H), 3.85 (s, 3H), 3.33-3.27 (m, 4H), 3.16 (s, 3H), 2.75 (br t, *J*=5.7 Hz, 6H), 1.75-1.68 (m, 2H), 1.29 (s, 6H) |
| 17 | LCMS (M+H)⁺ = 503.4. ¹H NMR (500 MHz, CHLOROFORM-d) δ 8.46-8.38 (m, 3H), 8.03 (d, *J*=8.5 Hz, 2H), 7.79 (s, 1H), 7.51 (d, *J*=8.5 Hz, 2H), 7.06 (d, *J*=8.5 Hz, 2H), 6.91 (s, 1H), 4.27 (s, 3H), 3.32-3.27 (m, 4H), 3.13 (s, 3H), 2.63-2.57 (m, 4H), 2.18 (d, *J*=7.6 Hz, 2H), 1.86 (dt, *J*=13.4, 6.9 Hz, 1H), 0.96 (d, *J*=6.6 Hz, 6H) |
| 18 | LCMS (M+H)⁺ = 489.3. ¹H NMR (400 MHz, METHANOL-d₄) δ 8.62 (d, *J*=1.6 Hz, 1H), 8.21 (s, 1H), 8.13-8.06 (m, 2H), 8.03-7.97 (m, 1H), 7.87-7.79 (m, 1H), 7.67 (dd, *J*=8.6, 2.0 Hz, 2H), 7.17-7.11 (m, 2H), 6.83 (s, 1H), 3.88 (d, *J*=2.0 Hz, 3H), 3.39 (br s, 4H), 3.24 (s, 3H), 3.08-2.98 (m, 5H), 1.26 (d, *J*=6.5 Hz, 6H) |

### EXAMPLE 19

### 2-Methyl-4-(4-(4-(1-methyl-2-(4-(methylsulfonyl)phenyl)-1H-pyrrolo[3,2-b]pyridin-6-yl) benzyl)piperazin-1-yl)butan-2-ol

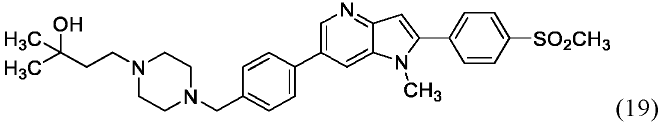

### Step 1. Benzyl 4-(3-hydroxy-3-methylbutyl)piperazine-1-carboxylate

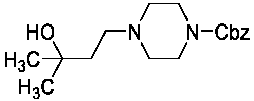

To a mixture of benzyl piperazine-1-carboxylate (150 mg, 0.681 mmol) and potassium carbonate (188 mg, 1.362 mmol) in DMF (2 mL) at 0 °C was added 4-bromo-2-methylbutan-2-ol (143 mg, 0.858 mmol) in DMF (0.2 mL) in one portion. The mixture was stirred at room temperature for 60 h, diluted with ethyl acetate (10 mL), and filtered through Celite. The filtrate was diluted with ethyl acetate (60 mL), washed with water (2 x 20 mL) and brine (20 mL), and dried over anhydrous MgSO₄. The title intermediate (110 mg, 0.359 mmol, 52.7 % yield) was isolated as a white solid by ISCO chromatography (24 g silica gel, solid loading, 1-8% ethyl acetate/hexane). LCMS (M+H)⁺ = 307.4. ¹H NMR (400 MHz, chloroform-d) δ 7.39-7.29 (m, 5H), 5.13 (s, 2H), 3.55-3.48 (m, 4H), 2.68-2.60 (m, 2H), 2.49 (br s, 4H), 1.66-1.61 (m, 2H), 1.23 (s, 6H).

### Step 2. 2-Methyl-4-(piperazin-1-yl)butan-2-ol

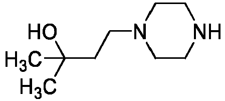

A mixture of benzyl 4-(3-hydroxy-3-methylbutyl)piperazine-1-carboxylate (105 mg, 0.343 mmol) and 10% Pd/C (22.98 mg, 0.022 mmol) in MeOH (9 mL) and THF (3 mL) was stirred under H₂, provided with a H₂ balloon, at room temperature for 3.5 h. The catalyst was removed by suction filtration through Celite. Removal of the solvent under vacuum provided the title intermediate (56 mg, 0.325 mmol, 95 % yield) as a white solid, which was used in the next step without further purification. LCMS (M+H)⁺ = 173.2.

### Step 3. 6-Chloro-2-(4-(methylsulfonyl)phenyl)-1H-pyrrolo[3,2-b]pyridine

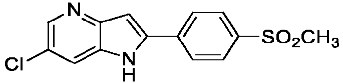

To a solution of 2-bromo-5-chloro-pyridin-3-amine (5.0 g, 24.1 mmol) in DMF (50 mL) at room temperature was added 1-ethynyl-4-methylsulfonyl-benzene (6.0 g, 33.74 mmol), CuI (459 mg, 2.41 mmol), t-BuOK (5.4 g, 48.2 mmol) and Pd(PPh₃)₂Cl₂ (500 mg, 0.43 mmol) in portions. The resulting mixture was degassed three times with nitrogen and stirred at 100 °C for 3 hours. Upon cooling to room temperature, the mixture was diluted with EtOAc, washed with brine, dried over Na₂SO₄. After the solvent was removed under vacuum, the residue was purified by silica flash chromatography with 1:1 Petroleum ether/EtOAc as the eluent to afford the title intermediate (1.937 g, 5.8 mmol, 24% yield) as a yellow solid. LCMS (M+H)⁺ = 307.1. ¹H NMR (400 MHz, DMSO-d₆) δ 12.22 (s, 1H), 8.37 (s, 1H), 8.20 (d, *J =* 8.4 Hz, 2H), 8.05 (d, *J*=8.8 Hz, 2H), 7.89 (s, 1H), 7.35 (s, 1H), 3.28 (s, 3H).

### Step 4. 6-Chloro-1-methyl-2-(4-(methylsulfonyl)phenyl)-1H-pyrrolo[3,2-b]pyridine

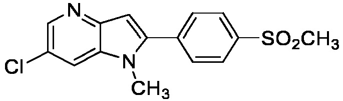

To a stirred solution of 6-chloro-2-(4-methylsulfonylphenyl)-1H-pyrrolo[3,2-b] pyridine (5.0 g, purity~80%, 13.07 mmol) and MeI (2.03 g, 14.38 mmol) in DMF (60 mL) at room temperature was added Cs₂CO₃ (8.52 g, 26.14 mmol) in portions. The resulting mixture was stirred at room temperature for 1 h, then diluted with EtOAc (100 mL), washed with brine, dried over anhydrous Na₂SO₄, and concentrated under vacuum. The residue was purified by reverse phase chromatography (Column: XSelect CSH Prep C18 OBD Column, 19 x 250 mm, 5 µm; Mobile Phase A: water (10 mmol/L NH₄HCO₃), Mobile Phase B: ACN; Flow rate: 50 mL/min; Gradient: 25% B to 40% B in 15 min; UV detection at 254/210 nm) to afford 6-chloro-1-methyl-2-(4-methylsulfonylphenyl) pyrrolo[3,2-b]pyridine(1.73 g, 5.22 mmol, 41.4% yield) as an off-white solid. LCMS (M+H)⁺ = 321.1. ¹H NMR (300 MHz, DMSO-d₆) δ 8.40 (s, 1H), 8.23 (s, 1H), 8.09 (d, *J* = 6.9 Hz, 2H), 7.95 (d, *J* = 8.4 Hz, 2H), 6.92 (s, 1H), 3.83 (s, 3H), 3.29 (s, 3H).

### Step 5. 4-(1-Methyl-2-(4-(methylsulfonyl)phenyl)-1H-pyrrolo[3,2-b]pyridin-6-yl) benzaldehyde

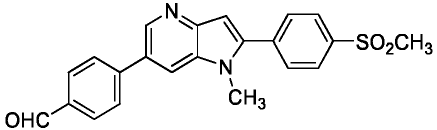

A mixture of 6-chloro-1-methyl-2-(4-(methylsulfonyl)phenyl)-1H-pyrrolo[3,2-b] pyridine (300 mg, 0.935 mmol), 4-(4,4,5,5-tetramethyl-1,3,2-dioxaborolan-2-yl)benzaldehyde (271 mg, 1.169 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3) (79 mg, 0.094 mmol), and potassium phosphate tribasic (1.637 mL, 3.27 mmol) in 1,4-dioxane (10 mL) was degassed and heated in a closed vial at 110 °C for 8 h. Upon cooling to room temperature, the mixture was diluted with ethyl acetate (30 mL) and filtered through Celite. The filtrate was concentrated under vacuum to near dryness. The residue was dissolved in ethyl acetate (150 mL), washed with brine (25 mL), and dried over anhydrous MgSO₄. The title intermediate (261 mg, 0.668 mmol, 71.5 % yield) was isolated as a yellow solid by ISCO chromatography (40 g silica gel, solid loading, 40-100% ethyl acetate). LCMS (M+H)⁺ = 391.0. ¹H NMR (400 MHz, DMSO-d₆) δ 10.09 (s, 1H), 8.87 (d, *J*=2.0 Hz, 1H), 8.45 (d, *J*=1.4 Hz, 1H), 8.15-8.08 (m, 4H), 8.08-8.03 (m, 2H), 8.00 (d, *J*=8.4 Hz, 2H), 6.97 (s, 1H), 3.94 (s, 3H), 3.33 (s, 3H).

### Step 6. 2-Methyl-4-(4-(4-(1-methyl-2-(4-(methylsulfonyl)phenyl)-1H-pyrrolo[3,2-b] pyridin-6-yl)benzyl)piperazin-1-yl)butan-2-ol

To a solution of 4-(1-methyl-2-(4-(methylsulfonyl)phenyl)-1H-pyrrolo[3,2-b] pyridin-6-yl)benzaldehyde (40 mg, 0.102 mmol), 2-methyl-4-(piperazin-1-yl)butan-2-ol (52.9 mg, 0.307 mmol), magnesium sulfate (247 mg, 2.049 mmol), and acetic acid (0.059 mL, 1.024 mmol) in DMF (1.2 mL) at room temperature was added sodium triacetoxyborohydride (87 mg, 0.410 mmol) in one portion. The mixture was stirred at room temperature for 18 h. The heterogeneous mixture was diluted with ethyl acetate (5 mL) and filtered through Celite. The filtrate was concentrated under vacuum to dryness. The residue was dissolved in MeOH and injected to prep. HPLC. The correct fractions were combined, concentrated under vacuum, basified with 1 N NaOH solution, and extracted with dichloromethane (4 x 35 mL). The combined extract was dried over anhydrous Na₂SO₄. Removal of the solvent under vacuum provided the title product (22.5 mg, 0.040 mmol, 39.4 % yield) as a white solid. LCMS (M+H)⁺ = 547.2. ¹H NMR (400 MHz, CHLOROFORM-d) δ 8.80 (d, *J*=2.0 Hz, 1H), 8.15-8.09 (m, 2H), 7.87-7.84 (m, 1H), 7.82-7.76 (m, 2H), 7.67 (d, *J*=8.0 Hz, 2H), 7.48 (br d, *J*=6.7 Hz, 2H), 6.92 (d, *J*=0.8 Hz, 1H), 3.87 (s, 3H), 3.69 (br s, 2H), 3.16 (s, 3H), 3.01-2.61 (m, 6H), 1.97-1.50 (m, 6H), 1.27 (s, 6H).

### EXAMPLE 20

### 1-Methyl-2-(4-(methylsulfonyl)phenyl)-6-(4-(piperazin-1-yl)phenyl)-1H-pyrrolo[3,2-b] pyridine

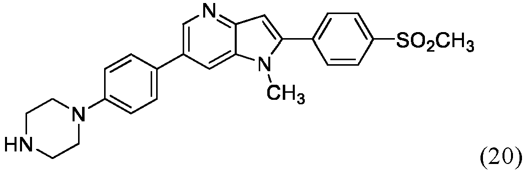

### Step 1. Tert-Butyl 4-(4-(1-methyl-2-(4-(methylsulfonyl)phenyl)-1H-pyrrolo[3,2-b] pyridin-6-yl)phenyl)piperazine-1-carboxylate

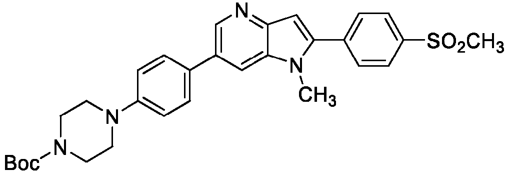

A mixture of 6-chloro-1-methyl-2-(4-(methylsulfonyl)phenyl)-1H-pyrrolo[3,2-b] pyridine (Example 19, Step 4) (80 mg, 0.249 mmol), tert-butyl 4-(4-(4,4,5,5-tetramethyl-1,3,2-dioxaborolan-2-yl)phenyl)piperazine-1-carboxylate(121 mg, 0.312 mmol), (2-dicyclohexylphosphino-2',4',6'-triisopropyl-1,1'-biphenyl)[2-(2'-amino-1,1'-biphenyl)]palladium(II) methanesulfonate (XPhos-Pd-G3) (21.11 mg, 0.025 mmol), and potassium phosphate tribasic (0.436 mL, 0.873 mmol) in 1,4-Dioxane (4 mL) was degassed and heated in a closed vial at 110 °C for 7 h. Upon cooling to room temperature, the mixture was diluted with ethyl acetate (60 mL), washed with brine (15 mL), and dried over anhydrous MgSO₄. The title product (95 mg, 0.170 mmol, 68.3 % yield) was isolated as a pale yellow solid by ISCO chromatography (40 g silica gel, 0-4% methanol/dichloromethane). LCMS (M+H)⁺ = 547.2. ¹H NMR (400 MHz, chloroform-d) δ 8.76(d, *J*=2.0 Hz, 1H), 8.09 (d, *J*=8.4 Hz, 2H), 7.80-7.74 (m, 3H), 7.61 (d, *J*=8.8 Hz, 2H), 7.06 (d, *J*=8.8 Hz, 2H), 6.88 (s, 1H), 3.83 (s, 3H), 3.66-3.59 (m, 4H), 3.26-3.18 (m, 4H), 3.14 (s, 3H), 1.50 (s, 9H).

### Step 2. 1-Methyl-2-(4-(methylsulfonyl)phenyl)-6-(4-(piperazin-1-yl)phenyl)-1H-pyrrolo[3,2-b]pyridine

To a solution of tert-butyl 4-(4-(1-methyl-2-(4-(methylsulfonyl)phenyl)-1H-pyrrolo[3,2-b]pyridin-6-yl)phenyl)piperazine-1-carboxylate (90 mg, 0.165 mmol) in dichloromethane (2 mL) at 0 °C was added TFA (2 mL) over 1 min. The mixture was stirred at 0 °C for 1 h, and then concentrated under vacuum to dryness. To the residue was added saturated NaHCO₃ solution. The mixture was extracted with dichloromethane (4 x 40 mL). The combined extract was dried over anhydrous Na₂SO₄. Removal of the solvent under vacuum provided the title product (61 mg, 0.134 mmol, 81 % yield) as a yellow solid. LCMS (M+H)⁺ = 447.1. ¹H NMR (400 MHz, DMSO-d₆) δ 8.71 (d, *J*=2.0 Hz, 1H), 8.19 (d, *J*=1.2 Hz, 1H), 8.09 (d, *J*=8.4 Hz, 2H), 7.97 (d, *J*=8.4 Hz, 2H), 7.73 (d, *J*=8.8 Hz, 2H), 7.09 (d, *J*=9.0 Hz, 2H), 6.90 (s, 1H), 3.90 (s, 3H), 3.32 (s, 3H), 3.27-3.22 (m, 4H), 3.07-2.99 (m, 4H).

The following Examples were prepared according to the general methods described herein using appropriate starting materials, reagents and conditions.

**TABLE 2**

| Ex. No. | Structure |
|---|---|
| 21 | 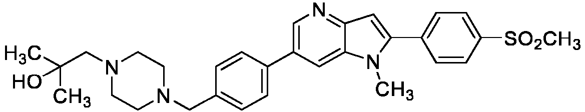 |
| 22 | 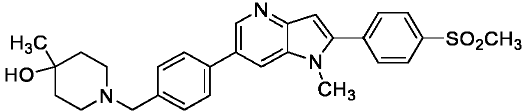 |
| 23 | 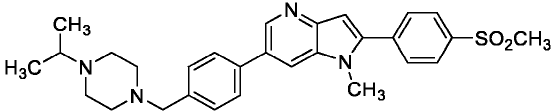 |
| 24 (reference compound) | 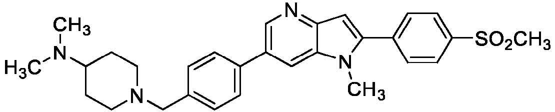 |
| 25 | 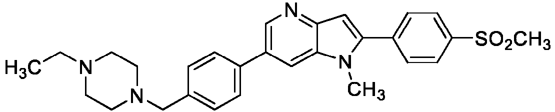 |
| 26 | 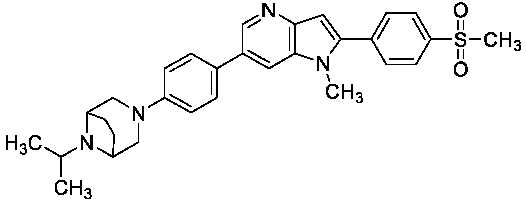 |
| 27 | 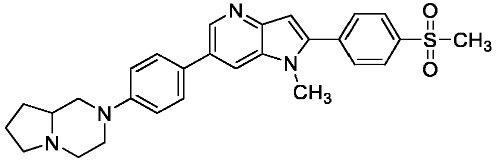 |
| 28 | 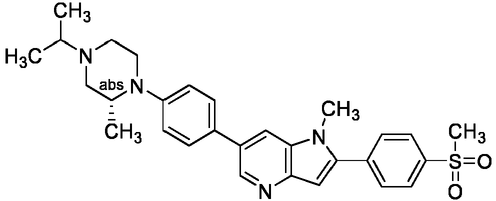 |
| 29 | 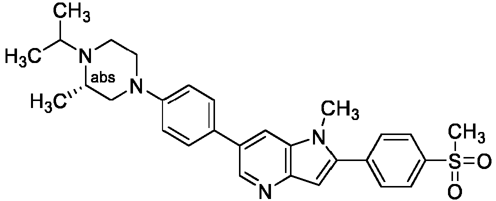 |
| 30 | 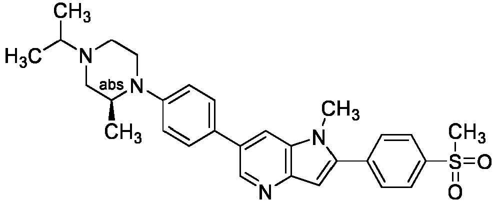 |
| 31 | 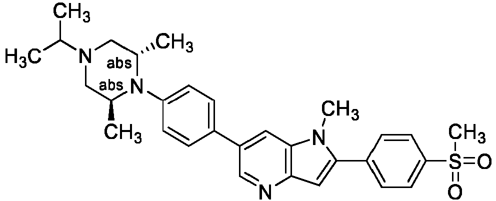 |
| 32 | 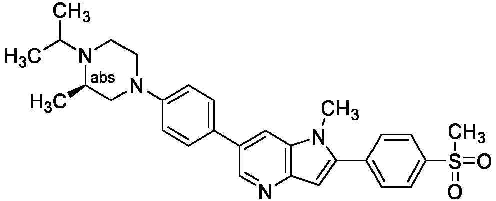 |
| 33 | 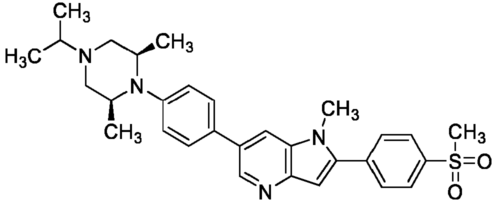 |
| 34 | 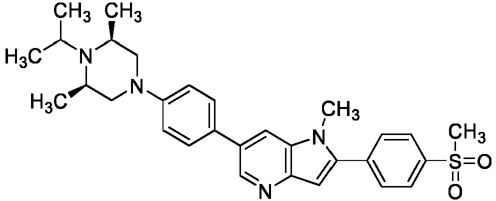 |
| 35 | 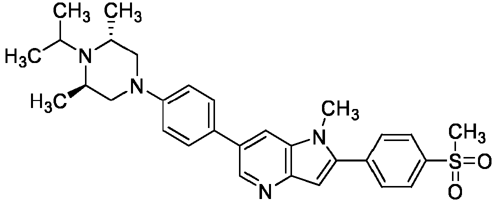 |
| 36 | 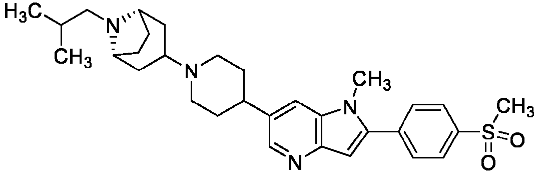 |

| Ex. No. | Analytical data |
|---|---|
| 21 | LCMS (M+H)⁺ = 533.2. ¹H NMR (400 MHz, chloroform-d) δ 8.84-8.77 (m, 1H), 8.16-8.08 (m, 2H), 7.86 (br s, 1H), 7.83-7.78 (m, 2H), 7.70-7.65 (m, 2H), 7.52 (br s, 2H), 6.92 (br d, J=3.1 Hz, 1H), 3.87 (d, J=4.1 Hz, 3H), 3.67 (br s, 2H), 3.17 (d, J=3.9 Hz, 3H), 2.94-2.34 (m, 10H), 1.22 (br s, 6H). |
| 22 | LCMS (M+H)⁺ = 490.3. ¹H NMR (400 MHz, chloroform-d) δ 8.84-8.77 (m, 1H), 8.12 (br dd, J=7.4, 2.5 Hz, 2H), 7.86 (br d, J=2.3 Hz, 1H), 7.81 (dd, J=4.3, 2.2 Hz, 2H), 7.68 (br s, 2H), 7.54 (br s, 2H), 6.92 (br d, J=2.5 Hz, 1H), 3.91-3.83 (br s, 3H), 3.76-3.57 (br s, 2H), 3.21-3.11 (br s, 3H), 2.31 -2.46 (m, 4H), 1.91-1.54 (m, 4H), 1.31 (br s, 3H). |
| 23 | LCMS (M+H)⁺ = 503.3. ¹H NMR (400 MHz, chloroform-d) δ 8.80 (d, J=2.0 Hz, 1H), 8.15-8.08 (m, 2H), 7.87-7.83 (m, 1H), 7.82-7.77 (m, 2H), 7.66 (d, J=8.2 Hz, 2H), 7.48 (d, J=8.2 Hz, 2H), 6.92 (d, J=0.8 Hz, 1H), 3.87 (s, 3H), 3.63 (s, 2H), 3.16 (s, 3H), 2.81-2.56 (m, 5H), 1.72-1.55 (m, 4H), 1.14 (br s, 6H). |
| 24 | LCMS (M+H)⁺ = 503.2. ¹H NMR (400 MHz, chloroform-d) δ 8.81 (d, J=2.0 Hz, 1H), 8.14-8.08 (m, 2H), 7.88-7.83 (m, 1H), 7.82-7.77 (m, 2H), 7.66 (d, J=8.0 Hz, 2H), 7.48 (d, J=8.2 Hz, 2H), 6.92 (s, 1H), 3.87 (s, 3H), 3.59 (s, 2H), 3.16 (s, 3H), 3.03 - 2.98 (m, 1H), 2.32 (s, 6H). 2.08- 2.02 (m, 2H), 1.86-1.81 (m, 2H), 1.64-1.58 (m, 4H). |
| 25 | LCMS (M+H)⁺ = 489.1. ¹H NMR (500 MHz, chloroform-d) δ 8.80 (d, J=1.8 Hz, 1H), 8.14-8.09 (m, 2H), 7.85 (d, J=1.1 Hz, 1H), 7.82-7.76 (m, 2H), 7.66 (d, J=8.0 Hz, 2H), 7.48 (d, J=8.0 Hz, 2H), 6.92 (s, 1H), 3.87 (s, 3H), 3.64 (s, 2H), 3.16 (s, 3H), 2.79-2.47 (m, 6H), 1.74 - 1.56 (m, 4H), 1.17 (br s, 3H). |
| 26 | LCMS (M+H)⁺ = 515.1. ¹H NMR (300 MHz, Chloroform-d) δ 8.79 (d, J = 1.9 Hz, 1H), 8.15-8.00 (m, 2H), 7.92-7.73 (m, 3H), 7.59 (d, J = 8.5 Hz, 2H), 7.02-6.80 (m, 3H), 3.83 (s, 3H), 3.69(s, 2H), 3.48-3.30 (m, 2H), 3.22-3.10 (m, 5H), 2.80-2.60 (m, 1H), 2.12-1.92 (m, 2H), 1.85-1.78 (m, 2H), 1.17 (d, J = 6.1 Hz, 6H) |
| 27 | LCMS (M+H)⁺ = 487.1. ¹H NMR (400 MHz, Chloroform-d) δ 8.78 (d, J = 1.9 Hz, 1H), 8.16-8.06 (m, 2H), 7.83-7.74 (m, 3H), 7.68-7.57 (m, 2H), 7.15-7.04 (m, 2H), 6.90 (d, J = 0.8 Hz, 1H), 3.91-3.80 (m, 4H), 3.74 (d, J = 12.0 Hz, 1H), 3.21-3.09 (m, 5H), 3.08-2.95 (m, 1H), 2.65 (t, J = 10.7 Hz, 1H), 2.45 (t, J = 11.0 Hz, 1H), 2.30-2.10 (m, 2H), 2.00-1.79 (m, 3H), 1.59-1.49 (m, 1H) |
| 28 | LCMS (M+H)⁺ = 503.2. ¹H NMR (300 MHz, Chloroform-d) δ 8.77 (d, J = 1.9 Hz, 1H), 8.09 (d, J = 8.4 Hz, 2H), 7.79-7.76 (m, 3H), 7.60 (d, J = 8.7 Hz, 2H), 7.07 (d, J = 8.6 Hz, 2H), 6.87 (d, J = 0.9 Hz, 1H), 3.99-3.83 (m, 4H), 3.36 (d, J = 12.1 Hz, 1H), 3.25-3.11 (m, 4H), 2.89-2.65 (m, 5H), 1.16-1.09 (m, 9H) |
| 29 | LCMS (M+H)⁺ = 503.3. ¹H NMR (300 MHz, Chloroform-d) δ 8.78 (d, J = 1.9 Hz, 1H), 8.08 (d, J = 8.7 Hz, 2H), 7.84-7.74 (m, 3H), 7.59 (d, J = 9.0 Hz, 2H), 7.05 (d, J = 9.0 Hz, 2H), 6.90 (d, J = 0.9 Hz, 1H), 3.85 (s, 3H), 3.70-3.51 (m, 2H), 3.39 (s, 1H), 3.16 (s, 3H), 3.10-2.46 (m, 5H), 1.22 (s, 6H), 1.00 (s, 3H) |
| 30 | LCMS (M+H)⁺ = 503.2. ¹H NMR (300 MHz, Chloroform-d) δ 8.78 (d, J = 1.9 Hz, 1H), 8.09 (d, J = 8.4 Hz, 2H), 7.78-7.60 (m, 3H), 7.60 (d, J = 8.6 Hz, 2H), 7.06 (d, J= 8.4 Hz, 2H), 6.88 (s, 1H), 3.98-3.84 (m, 1H), 3.82 (s, 3H), 3.42-3.28 (m, 1H), 3.23-3.14 (m, 4H), 2.95-2.48 (m, 5H), 1.18-1.00 (m, 9H) |
| 31 | LCMS (M+H)⁺ = 517.2. ¹H NMR (300 MHz, Chloroform-d) δ 8.81 (s, 1H), 8.10 (d, J = 8.4 Hz, 2H), 7.88-7.72 (m, 3H), 7.61 (d, J = 8.4 Hz, 2H), 7.18 (d, J = 5.1 Hz, 2H), 6.89 (s, 1H), 3.84 (s, 3H), 3.72-3.60 (m, 2H), 3.15 (s, 3H), 2.90-2.65 (m, 3H), 2.64-2.38 (m, 2H), 1.20-0.90 (m, 12H) |
| 32 | LCMS (M+H)⁺ = 503.2. ¹H NMR (300 MHz, Chloroform-d) δ 8.76 (d, J = 1.9 Hz, 1H), 8.09 (d, J = 8.1 Hz, 2H), 7.92-7.68 (m, 3H), 7.60 (d, J = 8.4 Hz, 2H), 7.06 (d, J = 8.4 Hz, 2H), 6.88 (s, 1H), 3.84 (s, 3H), 3.67-3.48 (m, 2H), 3.45-3.42 (m, 1H), 3.14 (s, 3H), 3.06-2.68 (m, 4H), 2.60-245 (m, 1H), 1.30-1.10 (m, 6H), 0.97 (d, J = 6.5 Hz, 3H) |
| 33 | LCMS (M+H)⁺ = 517.2. ¹H NMR (300 MHz, Chloroform-d) δ 8.79 (d, J = 1.9 Hz, 1H), 8.10 (d, J = 8.4 Hz, 2H), 7.98-7.74 (m, 3H), 7.64 (d, J = 8.5 Hz, 2H), 7.29 (s, 1H), 7.26 (s, 1H), 6.89 (s, 1H), 3.85 (s, 3H), 3.40-3.21 (m, 2H), 3.15 (s, 3H), 2.98-2.68 (m, 3H), 2.38-2.18 (m, 2H), 1.14 (d, J = 6.5 Hz, 6H), 0.91 (d, J = 6.3 Hz, 6H) |
| 34 | LCMS (M+H)⁺ = 517.1. ¹H NMR (300 MHz, Methanol-d₄) δ 8.62 (s, 1H), 8.18-8.07 (m, 3H), 7.96-7.87 (m, 2H), 7.72-7.64 (m, 2H), 7.08-6.99 (m, 1H), 6.83 (s, 1H), 3.90 (s, 3H), 3.67-3.61 (m, 5H), 3.21 (s, 5H), 1.39-1.33 (m, 12H). |
| 35 | LCMS (M+H)⁺ = 517.3. ¹H NMR (400 MHz, Chloroform-d) δ 8.77 (d, J = 1.9 Hz, 1H), 8.16-8.04 (m, 2H), 7.88-7.78 (m, 3H), 7.66-7.58 (m, 2H), 7.09-7.03 (m, 2H), 6.90 (d, J = 0.9 Hz, 1H), 3.85 (s, 3H), 3.41 (s, 3H), 3.31 (d, J = 10.5 Hz, 2H), 3.16 (s, 3H), 3.03 (s, 2H), 1.33-1.18 (m, 9H), 1.10 (d, J = 6.4 Hz, 3H) |
| 36 | LCMS (M+H)⁺ = 535.3. ¹H NMR (300 MHz, Methanol-d₄) δ 8.32 (d, J = 1.8 Hz, 1H), 8.17-8.08 (m, 2H), 7.95-7.86 (m, 3H), 6.79 (s, 1H), 3.87 (s, 3H), 3.41-3.27 (m, 2H), 3.25-3.20 (m, 4H), 3.19-3.12 (m, 1H), 2.92-2.74 (m, 1H), 2.74-2.59 (m, 1H), 2.42-2.19 (m, 4H), 2.10-1.89 (m, 5H), 1.89-1.72 (m, 6H), 1.71- 1.52 (m, 2H), 0.98 (d, J = 6.6 Hz, 6H) |

### EXAMPLE 37

### 2-(4-cyclopropylsulfonylphenyl)-1-methyl-6-[1-[rac-(1S,5R)-8-isobutyl-8-azabicyclo[3.2.1]octan-3-yl]-4-piperidyl]pyrrolo[3,2-b]pyridine

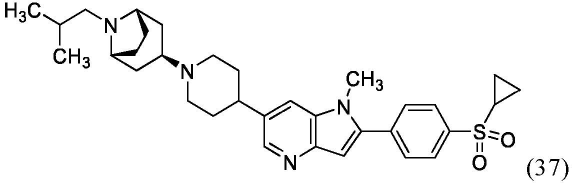

### Step 1. ((4-(cyclopropylsulfonyl)phenyl)ethynyl)trimethylsilane

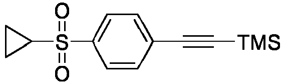

To a stirred solution of 1-bromo-4-cyclopropylsulfonyl-benzene (2.6 g, 9.96 mmol) and Et₃N (4.16 mL, 29.87 mmol) in THF (20 mL) were added CuI (189.6 mg, 1 mmol), Pd(PPh₃)₂Cl₂ (697.94 mg, 1 mmol) and ethynyl(trimethyl)silane (1.47 g, 14.93 mmol) at 0 °C. The resulting solution was degassed three times with nitrogen and stirred for 2 h at room temperature under a nitrogen atmosphere. The mixture was then concentrated and purified by column chromatography with petroleum ether / EtOAc (2:1) as eluent to afford the title compound (1.7 g, 6.1 mmol, 61.3 % yield) as a brown solid. LCMS (M+H)⁺ = 279.1.

### Step 2. 1-(cyclopropylsulfonyl)-4-ethynylbenzene

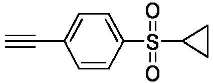

To a stirred solution of 2-(4-cyclopropylsulfonylphenyl)ethynyl-trimethylsilane (1.7 g, 6.11 mmol) in THF (20 mL) was added TBAF (1 M solution in THF) (2.44 mL, 2.44 mmol). The resulting solution was stirred for 10 min. at room temperature under a nitrogen atmosphere. The mixture was then concentrated and purified by column chromatography with petroleum ether / EtOAc (2:1) as eluent to afford the title compound (720 mg, 3.49 mmol, 57.2 % yield) as a yellow solid. ¹H NMR (400 MHz, Chloroform-*d*) δ 7.91-7.85 (m, 2H), 7.70-7.65 (m, 2H), 3.30(s, 1H), 2.51-2.44 (m, 1H), 1.40-1.30 (m, 2H), 1.10-1.03 (m, 2H).

### Step 3. 6-chloro-2-(4-(cyclopropylsulfonyl)phenyl)-1H-pyrrolo[3,2-b]pyridine

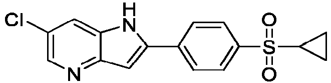

To a stirred solution of 2-bromo-5-chloro-pyridin-3-amine (350 mg, 1.69 mmol) and 1-cyclopropylsulfonyl-4-ethynyl-benzene (348 mg, 1.69 mmol) in DMF (5 mL) were added CuI (32.1 mg, 0.17 mmol), Pd(PPh₃)₂Cl₂ (118.2 mg, 0.17 mmol) and Et₃N (0.71 mL, 5.06 mmol). The resulting solution was degassed three times with nitrogen and stirred overnight at 90 °C under a nitrogen atmosphere. The reaction was then quenched with water and the mixture was extracted with EtOAc (3 x 30 mL). The organic layer was washed with NaCl solution and dried with Na₂SO₄, and concentrated. The crude product was dissolved in DMF (5 mL) and t-BuOK (566.7 mg, 5.06 mmol) was added. The resulting solution was degassed three times with nitrogen and stirred overnight at 90 °C. The reaction was then quenched with water and the mixture was extracted with EtOAc (3 x 30 mL). The organic layer was washed with NaCl solution and dried with Na₂SO₄, and concentrated. The crude product was purified by column chromatography with petroleum ether / EtOAc (1:1) as eluent to afford the title compound (281 mg, 0.76 mmol, 63.8 % yield) as a brown solid. LCMS (M+H)⁺ = 333.1.

### Step 4. 6-chloro-2-(4-cyclopropylsulfonylphenyl)-1-methyl-pyrrolo[3,2-b]pyridine

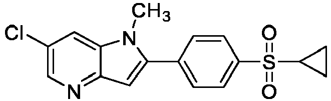

To a solution of 6-chloro-2-(4-cyclopropylsulfonylphenyl)-1H-pyrrolo[3,2-b] pyridine (350 mg, 1.05 mmol) in DMF (8 mL) were added CH₃I (0.2 mL, 1.09 mmol) and Cs₂CO₃ (700 mg, 2.15 mmol) at room temperature. After stirring for 1 h, the mixture was diluted with water, extracted with EtOAc (3 x 50 mL), washed by water (3 x 50 mL) and dried over anhydrous Na₂SO₄ and concentrated. The residue was then purified by silica gel chromatography to afford the title compound (150 mg, 41.1 % yield) as a light yellow solid. LCMS (M+H)⁺ = 347.1.

### Step 5. 2-(4-cyclopropylsulfonylphenyl)-1-methyl-6-(4-piperidyl)pyrrolo[3,2-b]pyridine

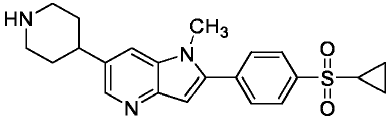

To a mixture of 6-chloro-2-(4-cyclopropylsulfonylphenyl)-1-methyl-pyrrolo[3,2-b]pyridine (140 mg, 0.4 mmol) and tert-butyl 4-(4,4,5,5-tetramethyl-1,3,2-dioxaborolan-2-yl)-3,6-dihydro-2H-pyridine-1-carboxylate (190 mg, 0.61 mmol) in 1,4-dioxane (8 mL) was added a solution of K₃PO₄ (260 mg, 1.23 mmol) in water (2 mL). To above mixture was added Xphos Pd G₃ (70 mg, 0.08 mmol). The resulting mixture was degassed three times with nitrogen and stirred at 90 °C overnight. The mixture was then cooled, diluted with water (100 mL) and extracted with ethyl acetate (3 x 50 mL). The combined organic phase was washed with water (3 x 50 mL) and dried over anhydrous Na₂SO₄. After filtration, the organic layer was concentrated under reduced pressure and the residue was purified by silica gel chromatography. The obtained intermediate was dissolved in MeOH (8 mL), and degassed three times with nitrogen. To this mixture was added Pd/C (20 mg), the reaction vessel was evacuated and back-filled with nitrogen three times, then back-filled with hydrogen (1 atm). After stirring 3 h, the catalyst was filtered and the filtrate was concentrated. The residue was then dissolved in DCM (8 mL) and HCl (4 M solution in 1,4-dioxane) (2 mL). After 1 h, the solvent was concentrated and the product was dried in vacuo to afford the title compound (70 mg crude) as a light yellow solid. LCMS (M+H)⁺ = 396.5.

### Step 6. Example 37

To a solution of ZnCl₂ (2 mL) in THF was added NaBH₃CN (320 mg, 5 mmol). The above mixture was stirred at room temperature for 30 minutes. To this mixture was added a solution of 2-(4-cyclopropylsulfonylphenyl)-1-methyl-6-(4-piperidyl)pyrrolo[3,2-b]pyridine (100 mg, 0.25 mmol) and 8-isobutyl-8-azabicyclo[3 .2.I]octan-3-one (600 mg, 3.31 mmol) in methanol (8 mL). The resulting mixture was stirred at 60 °C. After 18 h, the solvent was concentrated, and the crude mixture was purified by reverse chromatography flash and Prep-HPLC (10 mmol/L NH₄HCO₃) and B: ACN (40% B to 50% B in 5.5 min); Flow rate: 20 mL/min; Detector: UV 254/210 nm) to afford the title compound (19.2 mg, 13.2 % yield) as an off-white solid. ¹H NMR (400 MHz, Methanol-*d*₄) δ 8.31 (d, *J* = 1.8 Hz, 1H), 8.09-8.07 (m, 2H), 7.90-7.86 (m, 3H), 6.79 (s, 1H), 3.86 (s, 3H), 3.21 (d, *J* = 11.4 Hz, 2H), 2.81-2.75 (m, 2H), 2.71-2.61 (m, 1H), 2.33-2.26 (m, 4H), 2.05-1.75 (m, 13H), 1.72-1.63 (m, 2H), 1.32-1.29 (m, 2H), 1.15-1.12 (m, 2H), 0.97 (d, *J* = 6.6 Hz, 6H). LCMS (M+H)⁺ = 561.2.

### EXAMPLE 38

### 4-[6-[4-(4-isopropylpiperazin-1-yl)phenyl]-1-methyl-pyrrolo[3,2-b]pyridin-2-yl]-2-methoxy-benzonitrile

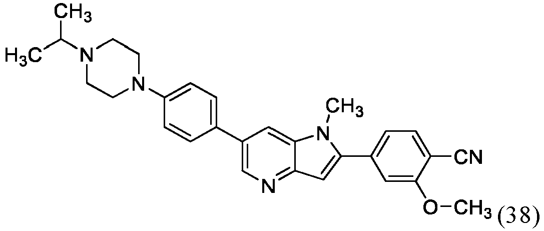

### Step 1. 2-methoxy-4-(2-trimethylsilylethynyl)benzonitrile

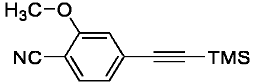

To a solution of 4-bromo-2-methoxy-benzonitrile (1 g, 4.72 mmol) in THF (10 mL) were added ethynyl(trimethyl)silane (2.3 g, 23.58 mmol), TEA (3.3 mL, 23.58 mmol), Pd(pph₃)₂Cl₂ (0.33 g, 0.47 mmol) and CuI (0.09 g, 0.47 mmol). The resulting mixture was degassed three times with nitrogen and stirred at room temperature. After 2 h, the mixture was diluted with water and extracted with ethyl acetate (3x). The combined organic layers were washed with brine, dried over Na₂SO₄ and concentrated under reduced pressure. The crude product was purified by silica column chromatography with petroleum ether / ethyl acetate (10:1) as eluent to afford the title compound (1 g, 92.5% yield) as a yellow oil. LCMS (M+H)⁺ = 230.2.

### Step 2. 4-ethynyl-2-methoxy-benzonitrile

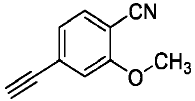

To a solution of 2-methoxy-4-(2-trimethylsilylethynyl)benzonitrile (1 g, 4.36 mmol) in methanol (10 mL) was added K₂CO₃ (1.2 g, 8.72 mmol) at 0 °C. After stirring 5 min. at room temperature, the mixture purified by silica column chromatography with petroleum ether/ ethyl acetate (10:1) as eluent to afford the title compound (600 mg, 87.6% yield) as a yellow solid. LCMS (M+H)⁺ = 158.2.

### Step 3. 4-(6-chloro-1H-pyrrolo[3,2-b]pyridin-2-yl)-2-methoxy-benzonitrile

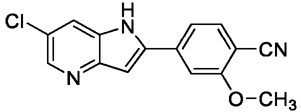

To a solution of 2-bromo-5-chloro-pyridin-3-amine (655 mg, 3.16 mmol) in DMF (5 mL) were added 4-ethynyl-2-methoxy-benzonitrile (600 mg, 3.82 mmol), t-BuOK (855.1 mg, 7.64 mmol), CuI (72.5 mg, 0.38 mmol) and Pd(pph₃)₂Cl₂ (267.6 mg, 0.38 mmol). The resulting mixture was degassed three times with nitrogen and stirred at 100 °C for 2 hours. The reaction was then concentrated under vacuum and diluted with water (30 mL) and extracted with DCM (3 x 30 mL). The combined organic extracts were washed with water (2 x 30 mL), dried over anhydrous sodium sulfate and concentrated under vacuum. The crude was purified by Prep-Flash (Column: SunFire Prep C18 OBD Column, 19×150 mm 5 um 10 nm; Mobile Phase A: Water (0.05% NH₄HCO₃), Mobile Phase B: ACN; Flow rate:20 mL/min; Gradient:60% B to 80% B in 5 min; 254/210 nm) to afford the title compound (160 mg, 14.8 % yield) as a yellow solid. LCMS (M+H)⁺ = 284.

### Step 4. 4-(6-chloro-1-methyl-pyrrolo[3,2-b]pyridin-2-yl)-2-methoxy-benzonitrile

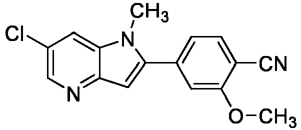

To a solution of 4-(6-chloro-1H-pyrrolo[3,2-b]pyridin-2-yl)-2-methoxybenzonitrile (160 mg, 0.56 mmol) and CH₃I (0.13 mL, 0.68 mmol) in DMF (5 mL) at 0 °C was added Cs₂CO₃ (367.5 mg, 1.13 mmol). The mixture was stirred at room temperature for 1 h. The resulting mixture was then diluted with water (30 mL), and extracted with ethyl acetate (3 x 30 mL). The combined organic extracts were dried over anhydrous sodium sulfate, and concentrated under vacuum. The crude was purified by silica column chromatography with ethyl acetate as eluent to afford the title compound (40 mg, 23.8 % yield) as a yellow solid. LCMS (M+H)⁺ = 298.1.

### Step 5. Example 38

To a solution of 4-(6-chloro-1-methyl-pyrrolo[3,2-b]pyridin-2-yl)-2-methoxybenzonitrile (30 mg, 0.1 mmol) and 1-isopropyl-4-[4-(4,4,5,5-tetramethyl-1,3,2-dioxaborolan-2-yl)phenyl]piperazine (66.6 mg, 0.2 mmol) in 1,4-dioxane (2.5 mL) and water (0.5 mL) were added K₃PO₄ (64.2 mg, 0.3 mmol), and XPhos Pd G₃ (8.5 mg, 0.01 mmol). The resulting mixture was degassed three times with nitrogen and stirred at 90 °C for 2 hours. The mixture was then cooled to room temperature, diluted with water and extracted with ethyl acetate (3x). The combined organic layers were washed with brine, dried over Na₂SO₄ and concentrated under reduced pressure. The crude product was purified by silica column chromatography with DCM / methanol (10:1) as eluent to afford the title compound (25.1 mg, 53.2%) as a yellow solid. ¹H NMR (300 MHz, Chloroform-d) δ 8.76 (s, 1H), 7.77 (s, 1H), 7.69 (d, J = 7.9 Hz, 1H), 7.59 (d, J = 8.4 Hz, 2H), 7.22-7.12 (m, 2H), 7.07 (d, J = 8.7 Hz, 2H), 6.85 (s, 1H), 4.03 (s, 3H), 3.82 (s, 3H), 3.38-3.25 (m, 4H), 2.83-2.66 (m, 5H), 1.13 (d, J = 6.5 Hz, 6H). LCMS (M+H)⁺ = 466.2.

The following Examples were prepared according to the general methods described elsewhere herein using appropriate starting materials, reagents and conditions.

**TABLE 3**

| Ex. No. | Structure |
|---|---|
| 39 | 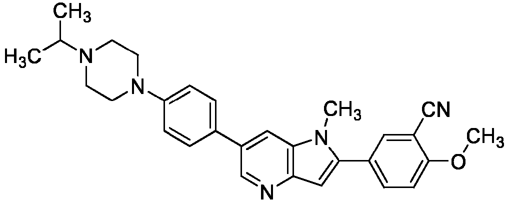 |
| 40 | 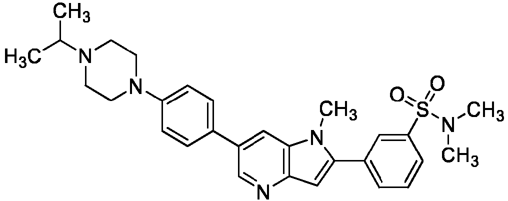 |
| 41 | 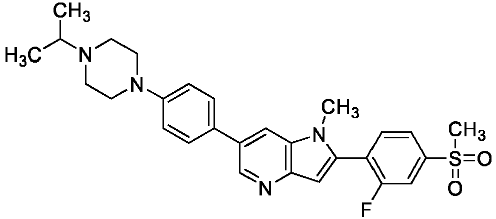 |
| 42 | 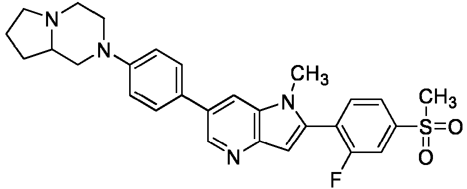 |
| 43 | 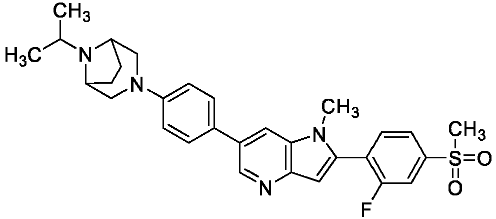 |
| 44 | 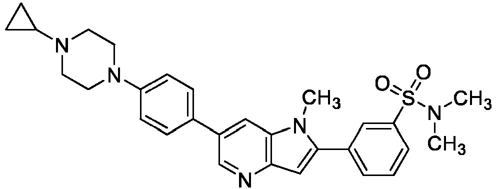 |
| 45 | 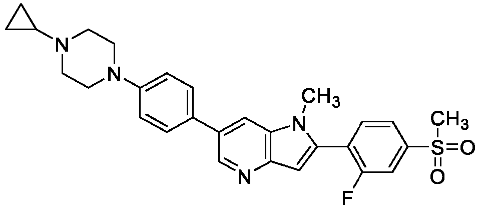 |

| Ex. No. | Analytical data |
|---|---|
| 39 | LCMS (M+H)⁺ = 466.2. ¹H NMR (300 MHz, Chloroform-d) δ 8.72 (s, 1H), 7.78-7.68 (m, 3H), 7.59 (d, J = 8.8 Hz, 2H), 7.17 -7.00 (m, 3H), 6.74 (s, 1H), 4.02 (s, 3H), 3.77 (s, 3H), 3.38-3.30 (m, 4H), 2.90-2.71 (m, 5H), 1.16 (d, J = 5.7 Hz, 6H) |
| 40 | LCMS (M+H)⁺ = 518.2. ¹H NMR (300 MHz, Chloroform-d) δ 8.76 (d, J = 1.9 Hz, 1H), 7.96 (s, 1H), 7.90-7.75 (m, 3H), 7.75-7.66 (m, 1H), 7.60 (d, J = 8.7 Hz, 2H), 7.07 (d, J = 8.7 Hz, 2H), 6.85 (s, 1H), 3.82 (s, 3H), 3.35 - 3.25 (m, 4H), 2.85-2.68 (m, 11H), 1.14 (d, J = 6.5 Hz, 6H) |
| 41 | LCMS (M+H)⁺ = 507.2. ¹H NMR (300 MHz, Chloroform-d) δ 8.77 (s, 1H), 7.93-7.70 (m, 4H), 7.60 (d, J = 8.6 Hz, 2H), 7.07 (d, J = 8.7 Hz, 2H), 6.88 (s, 1H), 3.75 (s, 3H), 3.37-3.25 (m, 4H), 3.16 (s, 3H), 2.84-2.65 (m, 5H), 1.14 (d, J = 6.6 Hz, 6H) |
| 42 | LCMS (M+H)⁺ = 505.2. ¹H NMR (400 MHz, Chloroform-d) δ 8.76 (s, 1H), 7.88 (dd, J=8.0, 1.8 Hz, 1H), 7.83 (dd, J=8.8, 1.7 Hz, 1H), 7.79 (dd, J = 2.0, 0.9 Hz, 1H), 7.74 (dd, J = 8.0, 6.7 Hz, 1H), 7.59 (d, J = 8.8 Hz, 2H), 7.07 (d, J = 8.8 Hz, 2H), 6.87 (s, 1H), 3.89-3.81 (m, 1H), 3.79-3.69 (m, 4H), 3.23-3.12 (m, 5H), 3.05-2.95 (m, 1H), 2.63 (t, J = 10.4, 11.2 Hz, 1H), 2.49-2.39 (m, 1H), 2.27-2.16 (m, 2H), 1.96-1.87 (m, 2H), 1.83-1.77 (m, 1H), 1.58-1.50 (m, 1H) |
| 43 | LCMS (M+H)⁺ = 533.2. ¹H NMR (400 MHz, Chloroform-d) δ 8.76 (s, 1H), 7.88 (dd, J=8.0, 1.8 Hz, 1H), 7.83 (dd, J = 8.8, 1.7 Hz, 1H), 7.77 (dd, J = 2.0, 0.9 Hz, 1H), 7.73 (dd, J = 8.0, 6.7 Hz, 1H), 7.58 (d, J = 8.0 Hz, 2H), 6.92 (d, J = 8.4 Hz, 2H), 6.87 (s, 1H), 3.74 (s, 3H), 3.68 (d, J = 4.7 Hz, 2H), 3.38 (dd, J = 11.2, 2.4 Hz, 2H), 3.21-3.12 (m, 5H), 2.75-2.66 (m, 1H), 2.05-1.93 (m, 2H), 1.80-1.78 (m, 2H), 1.16 (d, J = 6.4 Hz, 6H) |
| 44 | LCMS (M+H)⁺ = 516.2. ¹H NMR (300 MHz, DMSO-d₆) δ 8.68 (d, J = 2.0 Hz, 1H), 8.16 (s, 1H), 8.05-7.97 (m, 1H), 7.92 (s, 1H), 7.83 (d, J = 6.1 Hz, 2H), 7.68 (d, J = 8.5 Hz, 2H), 7.06 (d, J = 8.6 Hz, 2H), 6.86 (s, 1H), 3.85 (s, 3H), 3.17-3.10 (m, 4H), 2.80-2.60 (m, 10H), 1.75-1.65 (m, 1H), 0.60-0.18 (m, 4H) |
| 45 | LCMS (M+H)⁺ = 505.2. ¹H NMR (400 MHz, Chloroform-d) δ 8.78 (s, 1H), 7.88 (dd, J = 8.0, 1.7 Hz, 1H), 7.83 (dd, J = 8.9, 1.7 Hz, 1H), 7.79 (dd, J = 2.0, 0.9 Hz, 1H), 7.74 (dd, J = 8.0, 6.7 Hz, 1H), 7.59 (d, J = 8.8 Hz, 2H), 7.05 (d, J = 8.8 Hz, 2H), 6.89 (s, 1H), 3.74 (s, 3H), 3.32-3.20 (m, 4H), 3.15 (s, 3H), 2.95-2.83 (m, 4H), 1.72-1.68 (m, 1H), 0.60-0.45 (m, 4H) |

### BIOLOGICAL ASSAYS

The pharmacological properties of the compounds of this disclosure may be confirmed by a number of biological assays. The exemplified biological assays, which follow, have been carried out with compounds of the disclosure.

### TLR7/8/9 Inhibition Reporter Assays

HEK-Blue^{™}-cells (Invivogen) overexpressing human TLR7, TLR8 or TLR9 receptors were used for screening inhibitors of these receptors using an inducible SEAP (secreted embryonic alkaline phosphatase) reporter gene under the control of the IFN-β minimal promoter fused to five NF-κB and AP-1-binding sites. Briefly, cells are seeded into Greiner 384 well plates (15000 cells per well for TLR7, 20,000 for TLR8 and 25,000 for TLR9) and then treated with test compounds in DMSO to yield a final dose response concentration range of 0.05 nM - 50 µM. After a 30 minute compound pre-treatment at room temperature, the cells are then stimulated with a TLR7 ligand (gardiquimod at a final concentration of 7.5 µM), TLR8 ligand (R848 at a final concentration of 15.9 µM) or TLR9 ligand (ODN2006 at a final concentration of 5 nM) to activate NF-κB and AP-1 which induce the production of SEAP. After a 22 hour incubation at 37 °C, 5% CO₂, SEAP levels are determined with the addition of HEK-Blue^{™} Detection reagent (Invivogen), a cell culture medium that allows for detection of SEAP, according to manufacturer's specifications. The percent inhibition is determined as the % reduction in the HEK-Blue signal present in wells treated with agonist plus DMSO alone compared to wells treated with a known inhibitor.

| Ex. No. | TLR9 IC50 (µM) | TLR7 IC50 (µM) | TLR8 IC50 (µM) |
|---|---|---|---|
| 1 | 0.094 | 7.0 | 7.2 |
| 2 | 0.139 | 2.7 | 7.6 |
| 3 | 0.018 | 9.7 | 11.1 |
| 4 | 0.256 | - | - |
| 5 | nd | 7.2 | 10.2 |
| 6 | 0.139 | - | - |
| 7 | 4.576 | 50 | 50 |
| 8 | nd | 14.4 | 5.6 |
| 9 | nd | 4.7 | 8.7 |
| 10 | nd | 15.5 | 41.7 |
| 11 | nd | 1.9 | > 50 |
| 12 | nd | 4.8 | 1.3 |
| 13 | nd | 3.0 | 3.6 |
| 14 | nd | 29.6 | > 50 |
| 15 | nd | 50 | 11.2 |
| 16 | nd | 8.7 | 4.7 |
| 17 | nd | - | - |
| 18 | nd | - | - |
| 19 | 0.663 | 7.9 | 4.3 |
| 20 | 0.171 | 2.0 | 2.4 |
| 21 | 0.206 | 9.6 | 8.1 |
| 22 | 0.339 | 7.9 | 14.6 |
| 23 | 0.069 | > 50 | 23.2 |
| 24 | 0.014 | 4.9 | > 50 |
| 25 | 0.212 | > 50 | 43.8 |
| 26 | 0.094 | - | - |
| 27 | 0.275 | - | - |
| 28 | 0.236 | 3.2 | 5.0 |
| 29 | 0.722 | 3.3 | 2.3 |
| 30 | 0.576 | 4.8 | 5.3 |
| 31 | 0.480 | 2.5 | 4.5 |
| 32 | 0.243 | 11.4 | 15.1 |
| 33 | 0.383 | 3.8 | 4.2 |
| 34 | 0.244 | > 50 | > 50 |
| 35 | 0.091 | - | - |
| 36 | 0.022 | 10.5 | > 50 |
| 37 | 0.41 | 1.1 | > 50 |
| 38 | 0.505 | > 25 | > 25 |
| 39 | 0.101 | > 25 | > 25 |
| 40 | 0.076 | 8.1 | 2.1 |
| 41 | 0.157 | 10.0 | 1.6 |
| 42 | 0.436 | > 50 | 2.8 |
| 43 | 0.292 | > 50 | 7.8 |
| 44 | 0.700 | - | - |
| 45 | > 50 | - | - |

| | | | |
|---|---|---|---|
| nd: not determined | | | |

## Claims

1. A compound of Formula (Ia-2) or Formula (IIb): 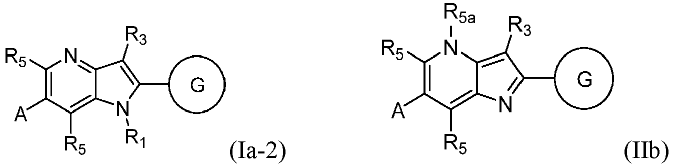 or stereoisomers, tautomer, solvates or salts thereof is provided, wherein:
G is:
(i) phenyl substituted with 1 to 3 substituents independently selected from F, Cl, Br, -CN, C₁₋₂ alkoxy, C₁₋₂ fluoroalkoxy, C₃₋₄ cycloalkyl -C(O)NR_{y}R_{y}, -S(O)₂CH₃, -S(O)₂(phenyl), -S(O)₂(cyclopropyl), -S(O)₂NRₓRₓ, and -S(O)(NH)NRₓRₓ;
(ii) 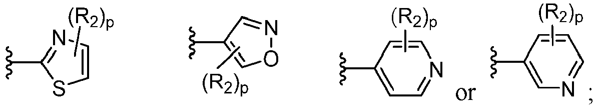
(iii) 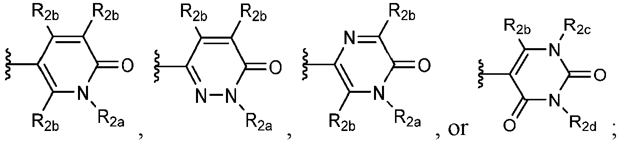
(iv) 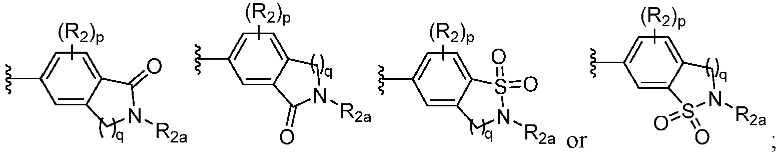
(v) a 9-membered heterocyclic ring selected from: 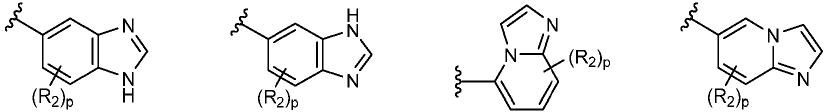 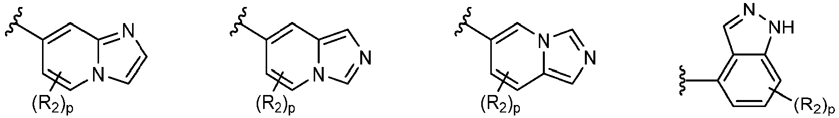 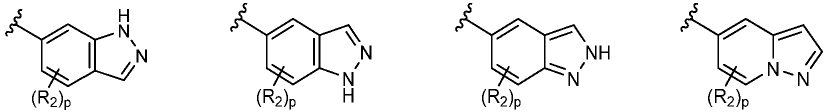 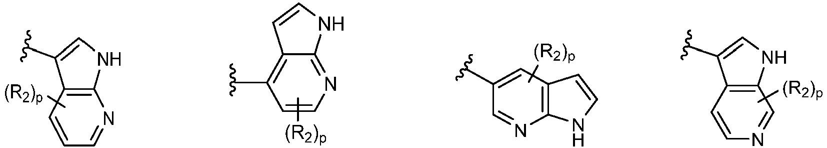 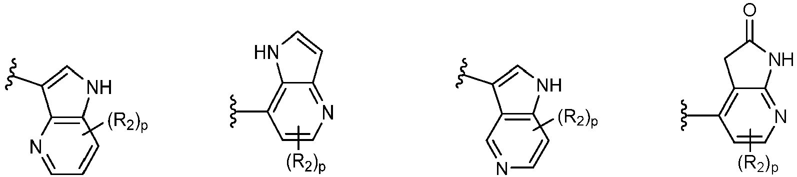 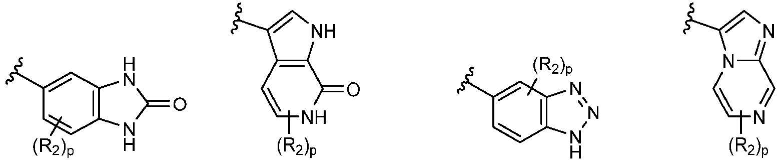 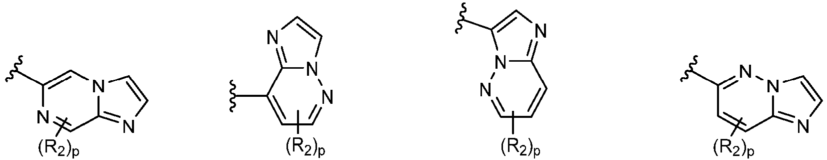 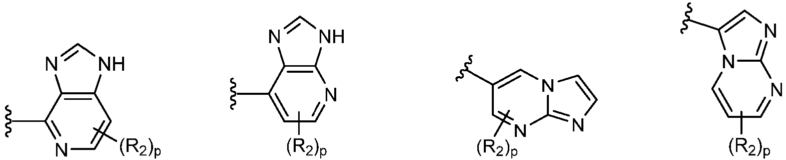 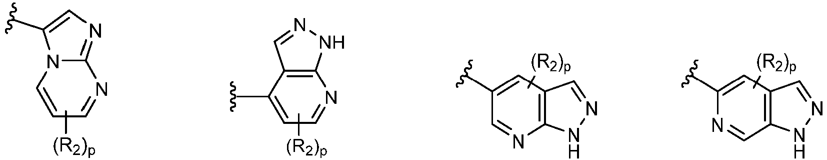 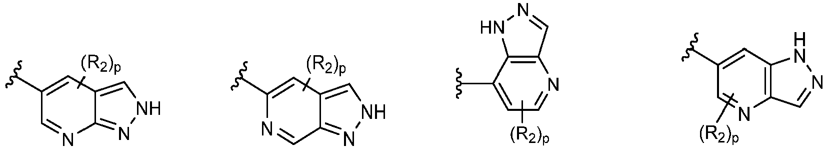 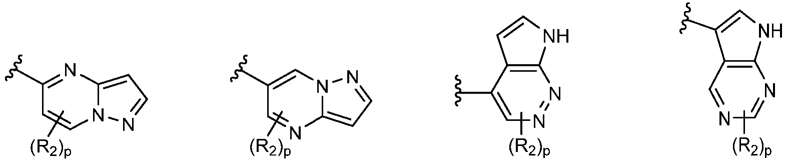 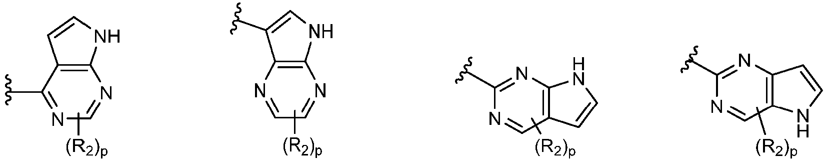 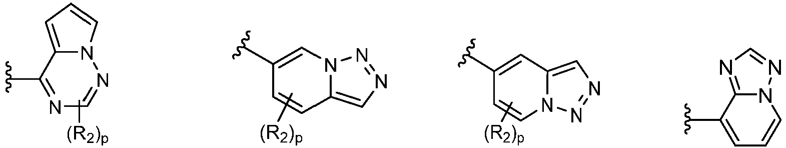 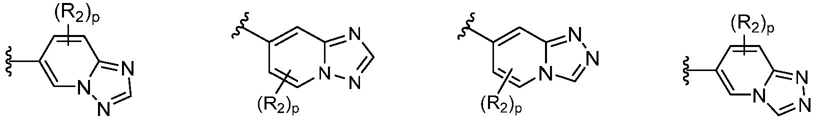 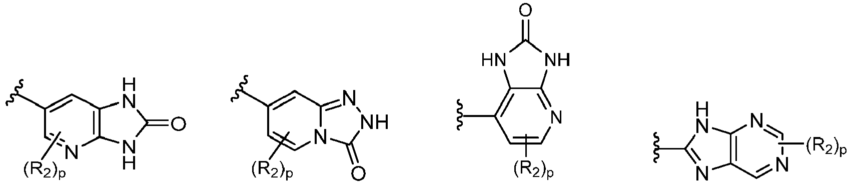 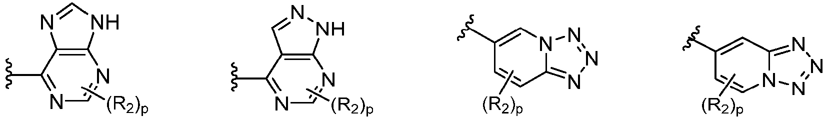 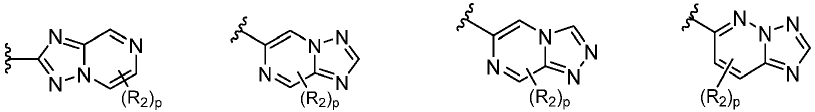 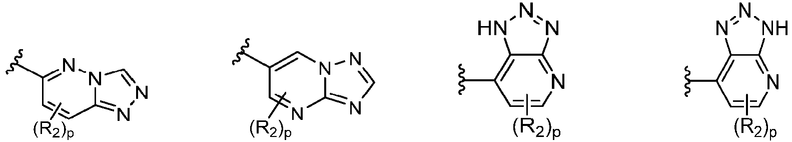 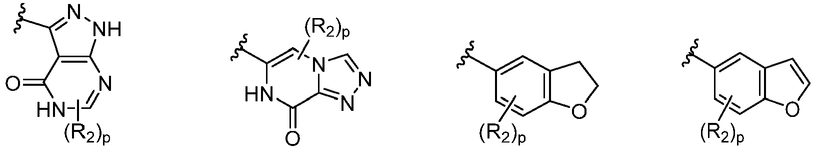 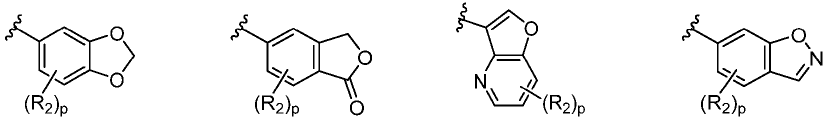 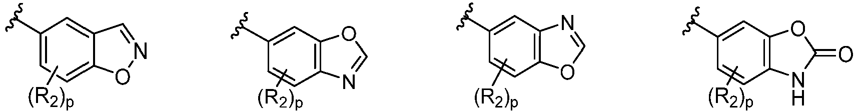 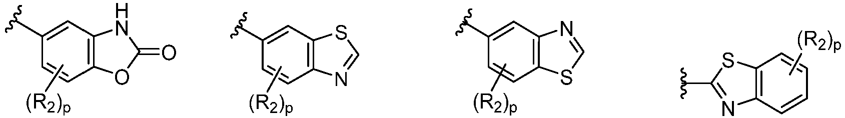 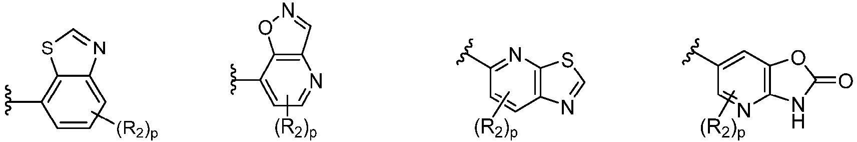 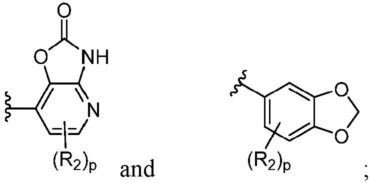 or
(vi) 10-membered heterocyclic ring selected from: 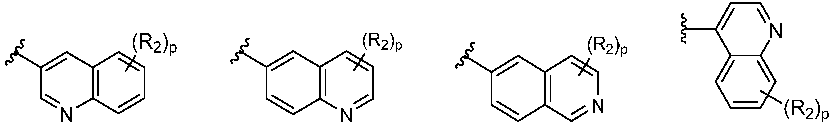 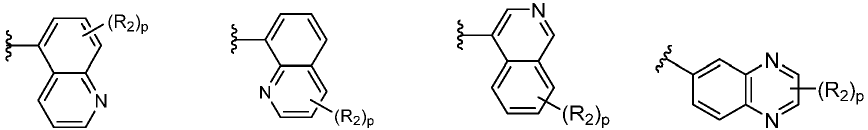 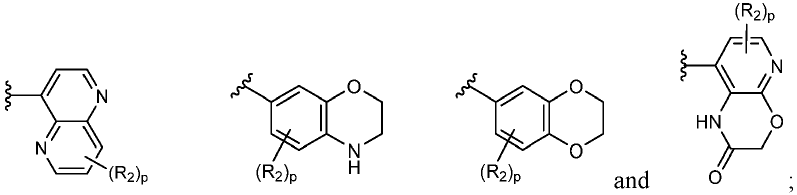
A is piperidinyl, phenyl, pyridinyl, pyrimidinyl, 6-azabicyclo[3.2.1]octanyl, or azabicyclo[3.2.1]octanyl, each substituted with -L-R₄ and zero to 1 R_{4b};
L is a bond, -CRₓRₓ- or -C(O)(CRₓRₓ)₀₋₂-;
R₁ is hydrogen, C₁₃ alkyl, C₁₋₂ fluoroalkyl, or C₃₋₄ cycloalkyl;
each R₂ is independently halo, -CN, -OH, -NO₂, C₁₋₄ alkyl, C₁₋₂ fluoroalkyl, C₁₋₂ cyanoalkyl, C₁₋₃ hydroxyalkyl, C₁₋₃ aminoalkyl, -O(CH₂)₁₋₂OH, -(CH₂)₀₋₄O(C₁₋₄ alkyl), C₁₋₃ fluoroalkoxy, -(CH₂)₁₋₄O(C₁₋₃ alkyl), -O(CH₂)₁₋₂OC(O)(C₁₋₃ alkyl), -O(CH₂)₁₋₂NRₓRₓ, -C(O)O(C₁₋₃ alkyl), -(CH₂)₀₋₂C(O)NR_{y}R_{y}, -C(O)NRₓ(C₁₋₅ hydroxyalkyl), -C(O)NRₓ(C₂₋₆ alkoxyalkyl), -C(O)NRₓ(C₃₋₆ cycloalkyl), -NR_{y}R_{y}, -NR_{y}(C₁₋₃ fluoroalkyl), -NR_{y}(C₁₋₄ hydroxyalkyl), -NRₓCH₂(phenyl), -NRₓS(O)₂(C₃₋₆ cycloalkyl), -NRₓC(O)(C₁₋₃ alkyl), -NRₓCH₂(C₃₋₆ cycloalkyl), -S(O)₂(C₁₋₃ alkyl), -S(O)₂N(C₁₋₃ alkyl)₂, -S(O)(NH)N(C₁₋₃ alkyl)₂, -(CH₂)₀₋₂(C₃₋₆ cycloalkyl), -(CH₂)₀₋₂(phenyl), morpholinyl, dioxothiomorpholinyl, dimethyl pyrazolyl, methylpiperidinyl, methylpiperazinyl, amino-oxadiazolyl, imidazolyl, triazolyl, or -C(O)(thiazolyl);
R₂ₐ is C₁₋₆ alkyl, C₁₋₃ fluoroalkyl, C₁₋₆ hydroxyalkyl, C₁₋₃ aminoalkyl, -(CH₂)₀₋₄O(C₁₋₃ alkyl), C₃₋₆ cycloalkyl, -(CH₂)₁₋₃C(O)NRₓRₓ, -CH₂(C₃₋₆ cycloalkyl), -CH₂(phenyl), tetrahydrofuranyl, tetrahydropyranyl, or phenyl;
each R_{2b} is independently hydrogen, halo, -CN, -NRₓRₓ, C₁₋₆ alkyl, C₁₋₃ fluoroalkyl, C₁₋₃ hydroxyalkyl, C₁₋₃ fluoroalkoxy, -(CH₂)₀₋₂O(C₁₋₃ alkyl), -(CH₂)₀₋₃C(O)NRₓRₓ, -(CH₂)₁₋₃(C₃₋₆ cycloalkyl), -C(O)O(C₁₋₃ alkyl), -C(O)NRₓ(C₁₋₃ alkyl), -CRₓ=CRₓRₓ, or -CRₓ=CH(C₃₋₆ cycloalkyl);
R_{2c} is R₂ₐ or R_{2b};
R_{2d} is R₂ₐ or R_{2b}; provided that one of R_{2c} and R_{2d} is R₂ₐ, and the other of R_{2c} and R_{2d} is R_{2b};
R₃ is hydrogen, F, Cl, C₁₋₃ alkyl, C₁₋₂ fluoroalkyl, or C₃₋₄ cycloalkyl;
R₄ is:
(i) -N(CH₃)₂;
(ii) pyrrolidinyl, piperidinyl, piperazinyl, pyridinyl, azaspiro[3.3]heptanyl, azabicyclo[3.2.1]octanyl, or diazabicyclo[3.2.1]octanyl, each substituted with zero to 3 R₄ₐ and zero to 2 -CH₃; or
(iii) 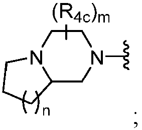
each R₄ₐ is independently -OH, C₁₋₆ alkyl, C₁₋₃ fluoroalkyl, C₁₋₆ hydroxyalkyl, C₃₋₆ cycloalkyl, -CH₂(C₃₋₆ cycloalkyl), -C(O)(C₁₋₄ alkyl), -C(O)(C₃₋₆ cycloalkyl), -C(O)(phenyl), -C(O)CH₂(C₃₋₆ cycloalkyl), -C(O)CH₂(phenyl), or -C(O)O(C₁₋₄ alkyl);
R_{4b} is F, Cl, or -CH₃;
each R_{4c} is independently C₁₋₆ alkyl, C₁₋₃ fluoroalkyl, -CH₂(C₃₋₆ cycloalkyl), -C(O)(C₁₋₄ alkyl), -C(O)(phenyl), -C(O)CH₂(phenyl), -C(O)OCH₂CH₃, or C₃₋₆ cycloalkyl;
each R₅ is independently hydrogen, F, Cl, C₁₋₂ alkyl, C₁₋₂ fluoroalkyl, or cyclopropyl;
R₅ₐ is hydrogen, C₁₋₂ alkyl, C₁₋₂ fluoroalkyl, or cyclopropyl;
each Rₓ is independently hydrogen or -CH₃;
each R_{y} is independently hydrogen or C₁₋₆ alkyl;
m is zero, 1, or 2;
n is zero, 1, or 2;
p is zero, 1, 2, 3, or 4; and
q is 1 or 2.

2. The compound according to claim 1 or stereoisomers, tautomer, solvates or salts thereof, having the structure of Formula (Ia-2):

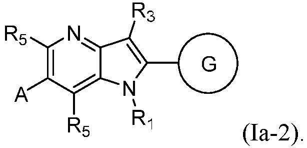

3. The compound according to claim 1 or stereoisomers, tautomer, solvates or salts thereof, wherein G is: 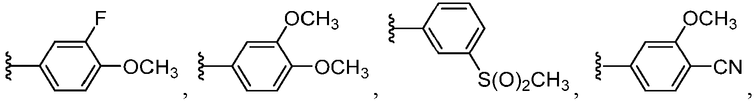 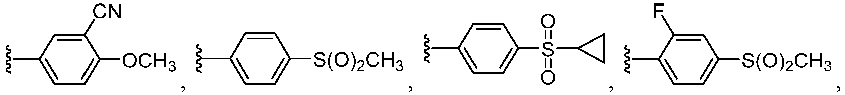 or

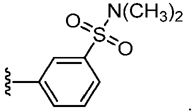

4. The compound according to claim 1 or stereoisomers, tautomer, solvates or salts 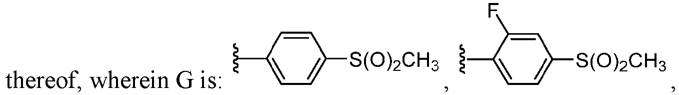 or

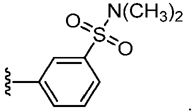

5. The compound according to claim 1 or stereoisomers, tautomer, solvates or salts thereof, wherein:
R₁ is hydrogen or -CH₃;
each R₅ is hydrogen;
G is phenyl substituted with 1 to 2 substituents independently selected from F, -CN, -OCH₃, -S(O)₂CH₃, -S(O)₂(cyclopropyl), or -S(O)₂N(CH₃)₂;
A is piperidinyl, phenyl or pyridinyl, each substituted with -L-R₄;
L is a bond, -CH₂-, or -C(O)-;
R₃ is hydrogen;
R₄ is:
(i) piperidinyl, piperazinyl, pyridinyl, azabicyclo[3.2.1]octanyl, or diazabicyclo[3.2.1]octanyl, each substituted with zero to 1 R₄ₐ and zero to 2 -CH₃; or
(ii) 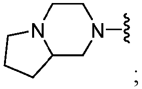
R₄ₐ is -OH, -CH₃, -CH₂CH₃, -CH(CH₃)₂, -CH₂CH(CH₃)₂, -CH₂C(CH₃)₂OH, -CH₂CH₂C(CH₃)₂OH, -CH₂(cyclopropyl), or cyclopropyl; and
each R₅ is hydrogen or -CH₃.

6. The compound according to claim 1 or stereoisomers, tautomer, solvates or salts thereof, wherein:
R₁ is hydrogen or -CH₃;
each R₅ is hydrogen;
G is phenyl substituted with 1 to 2 substituents independently selected from F, -CN, -OCH₃, -S(O)₂CH₃, -S(O)₂(cyclopropyl), or -S(O)₂N(CH₃)₂;
A is piperidinyl or phenyl, each substituted with -L-R₄;
L is a bond or -CH₂-;
R₃ is hydrogen;
R₄ is piperazinyl, or azabicyclo[3.2.1]octanyl, each substituted with zero to 1 R₄ₐ and zero to 2 -CH₃;
R₄ₐ is -CH₂CH₃, -CH(CH₃)₂, -CH₂CH(CH₃)₂, -CH₂C(CH₃)₂OH, -CH₂(cyclopropyl), or cyclopropyl; and
each R₅ is hydrogen or -CH₃.

7. The compound according to claim 1 having the structure: 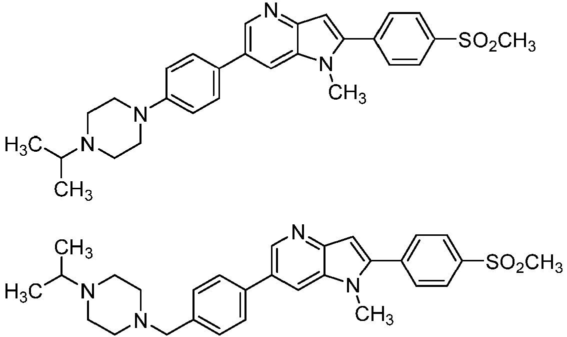 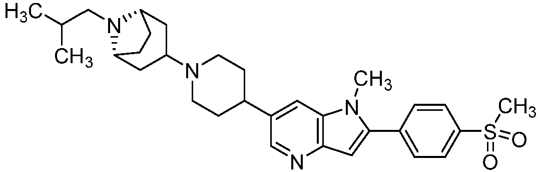 or 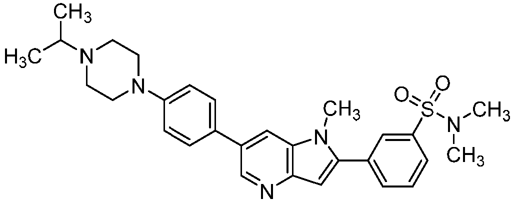 or a pharmaceutically acceptable salt thereof.

8. The compound according to claim 1 having the structure: 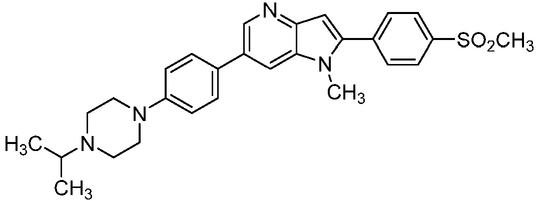 or a pharmaceutically acceptable salt thereof.

9. The compound according to claim 1 having the structure: 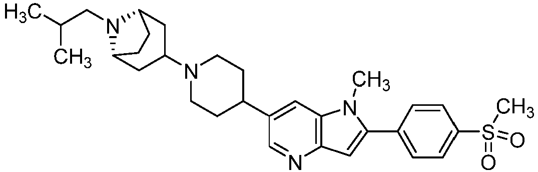 or a pharmaceutically acceptable salt thereof.

10. The compound according to claim 1 having the structure: 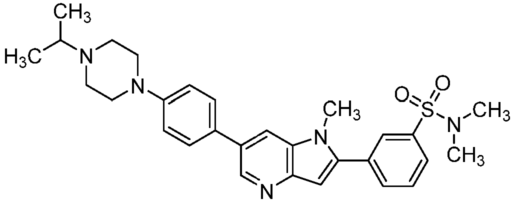 or a pharmaceutically acceptable salt thereof.

11. The compound according to claim 1 or stereoisomers, tautomer, solvates or salts thereof, wherein said compound is:
2-(3,4-dimethoxyphenyl)-6-(4-(4-isopropylpiperazin-1-yl)phenyl)-1-methyl-1H-pyrrolo[3,2-b]pyridine (1);
6-(4-(4-isopropylpiperazin-1-yl)phenyl)-1-methyl-2-(4-(methylsulfonyl)phenyl)-1H-pyrrolo[3,2-b]pyridine (2);
1-(4-(4-(2-(3,4-dimethoxyphenyl)-1-methyl-1H-pyrrolo[3,2-b]pyridin-6-yl) phenyl)piperazin-1-yl)-2-methylpropan-2-ol (4);
(4-(2-(3,4-dimethoxyphenyl)-1-methyl-1H-pyrrolo[3,2-b]pyridin-6-yl)phenyl)(4-isopropylpiperazin-1-yl)methanone (5);
6-(4-(4-isobutylpiperazin-1-yl)phenyl)-1-methyl-2-(4-(methylsulfonyl)phenyl)-1H-pyrrolo[3,2-b]pyridine (6);
6-(4-(4-(cyclopropylmethyl)piperazin-1-yl)phenyl)-2-(3,4-dimethoxyphenyl)-1-methyl-1H-pyrrolo[3,2-b]pyridine (8);
2-(3,4-dimethoxyphenyl)-6-(6-(4-isopropylpiperazin-1-yl)pyridin-3-yl)-1-methyl-1H-pyrrolo[3,2-b]pyridine (9);
2-(3-fluoro-4-methoxyphenyl)-6-(4-(4-isopropylpiperazin-1-yl)phenyl)-1-methyl-1H-pyrrolo[3,2-b]pyridine (10);
6-(4-(4-isopropylpiperazin-1-yl)phenyl)-2-(4-(methylsulfonyl)phenyl)-1H-pyrrolo[3,2-b]pyridine (11);
4-(4-(4-(2-(3,4-dimethoxyphenyl)-1-methyl-1H-pyrrolo[3,2-b]pyridin-6-yl) phenyl)piperazin-1-yl)-2-methylbutan-2-ol (12);
2-(3,4-dimethoxyphenyl)-1-methyl-6-(4-(piperazin-1-yl)phenyl)-1H-pyrrolo[3,2-b]pyridine (13);
6-(4-(4-isobutylpiperazin-1-yl)phenyl)-2-(4-(methylsulfonyl)phenyl)-1H-pyrrolo[3,2-b]pyridine (14);
2-methyl-1-(4-(4-(1-methyl-2-(4-(methylsulfonyl)phenyl)-1H-pyrrolo[3,2-b] pyridin-6-yl)phenyl)piperazin-1-yl)propan-2-ol (15);
2-methyl-4-(4-(4-(1-methyl-2-(4-(methylsulfonyl)phenyl)-1H-pyrrolo[3,2-b] pyridin-6-yl)phenyl)piperazin-1-yl)butan-2-ol (16); or
6-(4-(4-isobutylpiperazin-1-yl)phenyl)-4-methyl-2-(4-(methylsulfonyl)phenyl)-4H-pyrrolo[3,2-b]pyridine (18);
6-(4-(4-isopropylpiperazin-1-yl)phenyl)-1-methyl-2-(3-(methylsulfonyl)phenyl)-1H-pyrrolo[3,2-b]pyridine (19);
1-methyl-2-(4-(methylsulfonyl)phenyl)-6-(4-(piperazin-1-yl)phenyl)-1H-pyrrolo[3,2-b]pyridine (20);
2-methyl-1-(4-(4-(1-methyl-2-(4-(methylsulfonyl)phenyl)-1H-pyrrolo[3,2-b] pyridin-6-yl)benzyl)piperazin-1-yl)propan-2-ol (21);
4-methyl-1-(4-(1-methyl-2-(4-(methylsulfonyl)phenyl)-1H-pyrrolo[3,2-b]pyridin-6-yl)benzyl)piperidin-4-ol (22);
6-(4-((4-isopropylpiperazin-1-yl)methyl)phenyl)-1-methyl-2-(4-(methylsulfonyl) phenyl)-1H-pyrrolo[3,2-b]pyridine (23);
6-(4-((4-ethylpiperazin-1-yl)methyl)phenyl)-1-methyl-2-(4-(methylsulfonyl) phenyl)-1H-pyrrolo[3,2-b]pyridine (25);
6-(4-(8-isopropyl-3,8-diazabicyclo[3.2.1]octan-3-yl)phenyl)-1-methyl-2-(4-(methylsulfonyl)phenyl)-1H-pyrrolo[3,2-b]pyridine (26);
6-(4-(hexahydropyrrolo[1,2-a]pyrazin-2(1H)-yl)phenyl)-1-methyl-2-(4-(methylsulfonyl)phenyl)-1H-pyrrolo[3,2-b]pyridine (27);
(R)-6-(4-(4-isopropyl-2-methylpiperazin-1-yl)phenyl)-1-methyl-2-(4-(methylsulfonyl)phenyl)-1H-pyrrolo[3,2-b]pyridine (28);
(S)-6-(4-(4-isopropyl-3-methylpiperazin-1-yl)phenyl)-1-methyl-2-(4-(methylsulfonyl)phenyl)-1H-pyrrolo[3,2-b]pyridine (29);
(S)-6-(4-(4-isopropyl-2-methylpiperazin-1-yl)phenyl)-1-methyl-2-(4-(methylsulfonyl)phenyl)-1H-pyrrolo[3,2-b]pyridine (30);
6-(4-((2S,6S)-4-isopropyl-2,6-dimethylpiperazin-1-yl)phenyl)-1-methyl-2-(4-(methylsulfonyl)phenyl)-1H-pyrrolo[3,2-b]pyridine (31);
(R)-6-(4-(4-isopropyl-3-methylpiperazin-1-yl)phenyl)-1-methyl-2-(4-(methylsulfonyl)phenyl)-1H-pyrrolo[3,2-b]pyridine (32);
6-(4-((2R,6S)-4-isopropyl-2,6-dimethylpiperazin-1-yl)phenyl)-1-methyl-2-(4-(methylsulfonyl)phenyl)-1H-pyrrolo[3,2-b]pyridine (33);
6-(4-((3S,5R)-4-isopropyl-3,5-dimethylpiperazin-1-yl)phenyl)-1-methyl-2-(4-(methylsulfonyl)phenyl)-1H-pyrrolo[3,2-b]pyridine (34);
6-(4-((3R,5R)-4-isopropyl-3,5-dimethylpiperazin-1-yl)phenyl)-1-methyl-2-(4-(methylsulfonyl)phenyl)-1H-pyrrolo[3,2-b]pyridine (35);
6-(1-((1R,5S)-8-isobutyl-8-azabicyclo[3.2.1]octan-3-yl)piperidin-4-yl)-1-methyl-2-(4-(methylsulfonyl)phenyl)-1H-pyrrolo[3,2-b]pyridine (36);
2-(4-cyclopropylsulfonylphenyl)-1-methyl-6-[1-[rac-(1S,5R)-8-isobutyl-8-azabicyclo[3.2.1]octan-3-yl]-4-piperidyl]pyrrolo[3,2-b]pyridine (37);
4-[6-[4-(4-isopropylpiperazin-1-yl)phenyl]-1-methyl-pyrrolo[3,2-b]pyridin-2-yl]-2-methoxy-benzonitrile (38);
5-(6-(4-(4-isopropylpiperazin-1-yl)phenyl)-1-methyl-1H-pyrrolo[3,2-b]pyridin-2-yl)-2-methoxybenzonitrile (39);
3-(6-(4-(4-isopropylpiperazin-1-yl)phenyl)-1-methyl-1H-pyrrolo[3,2-b]pyridin-2-yl)-N,N-dimethylbenzenesulfonamide (40);
2-(2-fluoro-4-(methylsulfonyl)phenyl)-6-(4-(4-isopropylpiperazin-1-yl)phenyl)-1-methyl-1H-pyrrolo[3,2-b]pyridine (41);
2-(2-fluoro-4-(methylsulfonyl)phenyl)-6-(4-(hexahydropyrrolo[1,2-a]pyrazin-2(1H)-yl)phenyl)-1-methyl-1H-pyrrolo[3,2-b]pyridine (42);
2-(2-fluoro-4-(methylsulfonyl)phenyl)-6-(4-(8-isopropyl-3,8-diazabicyclo[3.2.1]octan-3-yl)phenyl)-1-methyl-1H-pyrrolo[3,2-b]pyridine (43);
3-(6-(4-(4-cyclopropylpiperazin-1-yl)phenyl)-1-methyl-1H-pyrrolo[3,2-b]pyridin-2-yl)-N,N-dimethylbenzenesulfonamide (44); or
6-(4-(4-cyclopropylpiperazin-1-yl)phenyl)-2-(2-fluoro-4-(methylsulfonyl)phenyl)-1-methyl-1H-pyrrolo[3,2-b]pyridine (45).

12. The compound according to claim 1 or a salt thereof, wherein said compound is:
6-(4-(4-isopropylpiperazin-1-yl)phenyl)-4-methyl-2-(4-(methylsulfonyl)phenyl)-4H-pyrrolo[3,2-b]pyridine (3); or
6-(4-(4-isobutylpiperazin-1-yl)phenyl)-4-methyl-2-(4-(methylsulfonyl)phenyl)-4H-pyrrolo[3,2-b]pyridine (17).

13. A pharmaceutical composition comprising one or more compounds according to any one of claims 1 to 12 and a pharmaceutically acceptable carrier or diluent.

14. A compound according to any one of claims 1 to 12 or stereoisomers, tautomers, solvates or pharmaceutically-acceptable salts thereof, or a pharmaceutical composition according to claim 13, for use in treating pathological fibrosis.

15. The compound or stereoisomers, tautomers, solvates or a pharmaceutically-acceptable salt thereof, or pharmaceutically-acceptable salts thereof, or the pharmaceutical composition, for use according to claim 14 wherein said pathological fibrosis is liver fibrosis, renal fibrosis, biliary fibrosis, or pancreatic fibrosis.

16. A compound according to any one of claims 1 to 12 or stereoisomers, tautomers, solvates or pharmaceutically-acceptable salts thereof, or a pharmaceutical composition according to claim 13, for use in treating nonalcoholic steatohepatitis (NASH), non-alcoholic fatty liver disease (NAFLD), chronic kidney disease, diabetic kidney disease, primary sclerosing cholangitis (PSC), or primary biliary cirrhosis (PBC).

17. A compound according to any one of claims 1 to 12 or stereoisomers, tautomers, solvates or pharmaceutically-acceptable salts thereof, or a pharmaceutical composition according to claim 13, for use in treating idiopathic pulmonary fibrosis (IPF).

## Patentansprüche

1. Verbindung der Formel (la-2) oder der Formel (IIb): 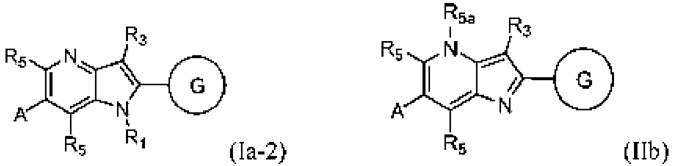 oder Stereoisomere, Tautomere, Solvate oder Salze davon, wobei:
G Folgendes ist:
(i) Phenyl substituiert mit 1 bis 3 Substituenten, unabhängig ausgewählt aus F, Cl, Br, -CN, C₁₋₂ Alkoxy, C₁₋₂ Fluoralkoxy, C₃₋₄ Cycloalkyl -C(O)NR_{y}R_{y}, -S(O)₂CH₃, - S(O)₂(Phenyl), -S(O)₂(Cyclopropyl), -S(O)₂NRₓRₓ und -S(O)(NH)NRₓRₓ;
(ii) 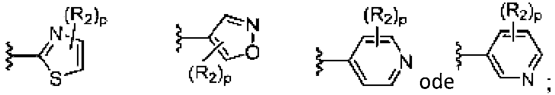
(iii) 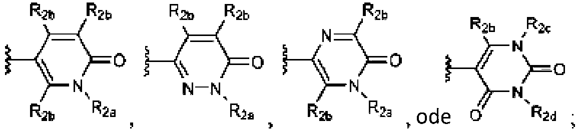
(iv) 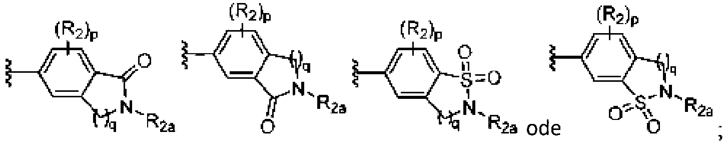
(v) ein neungliedriger heterocyclischer Ring, ausgewählt aus: 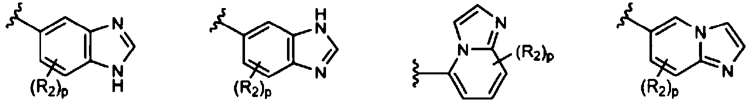 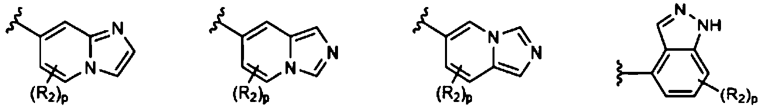 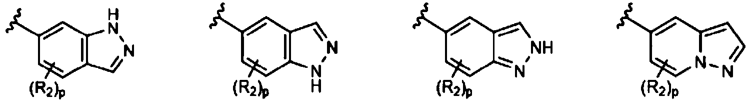 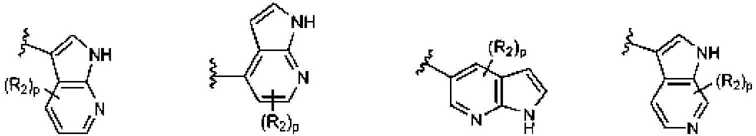 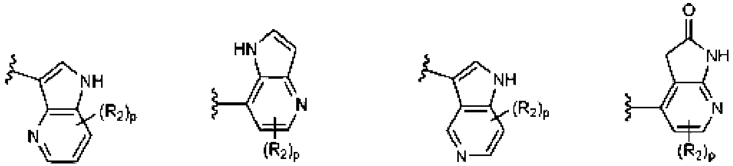 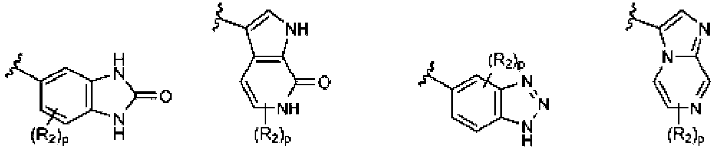 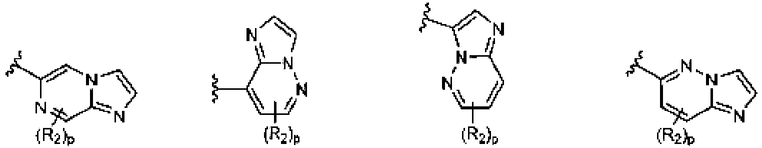 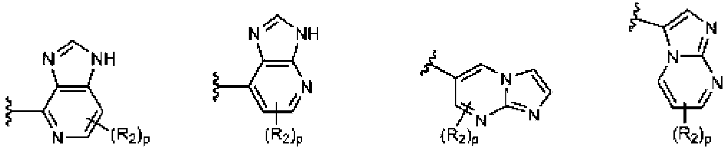 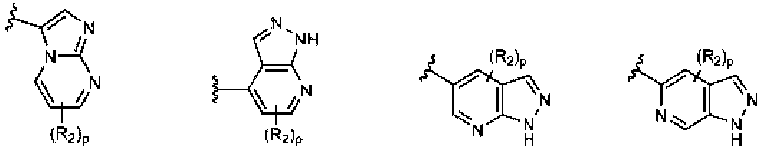 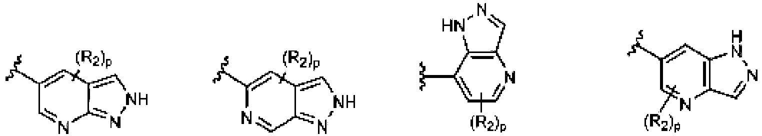 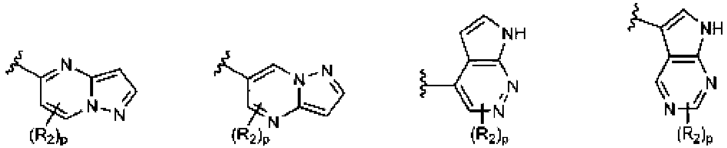 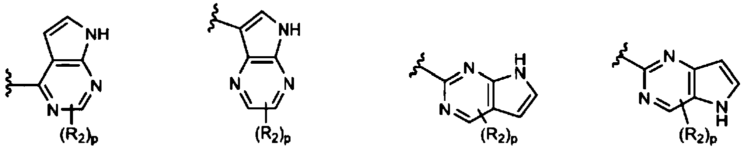 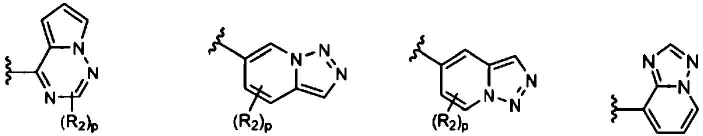 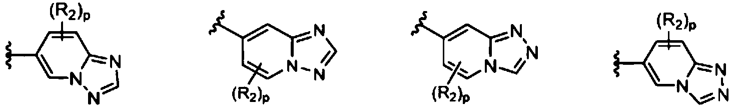 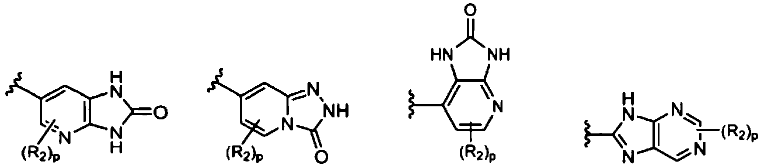 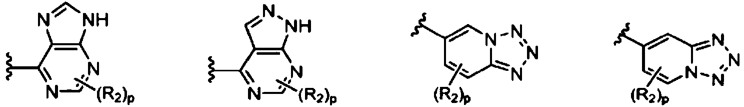 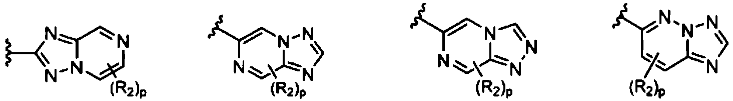 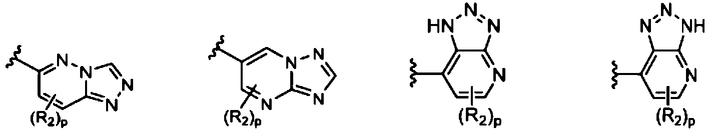 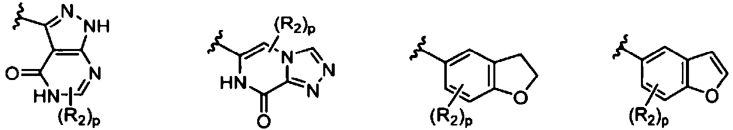 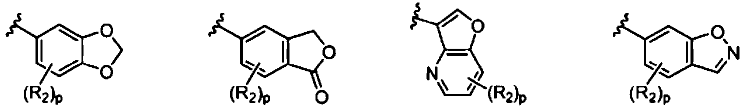 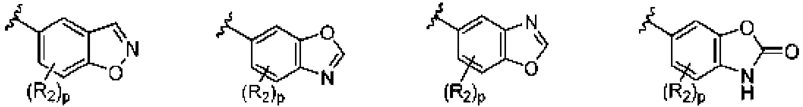 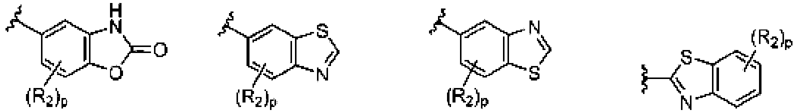 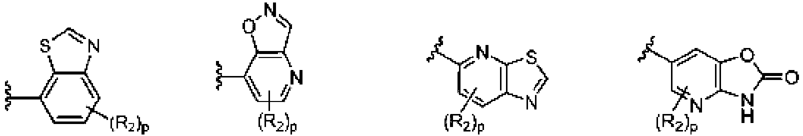 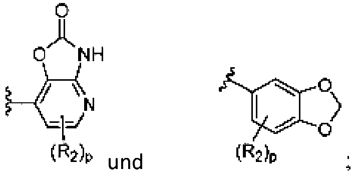 oder
(vi) 10-gliedriger heterocyclischer Ring, ausgewählt aus: 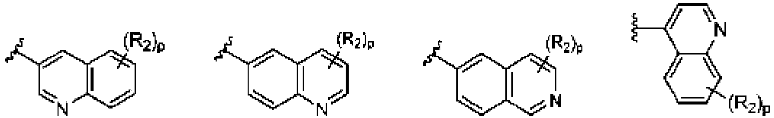 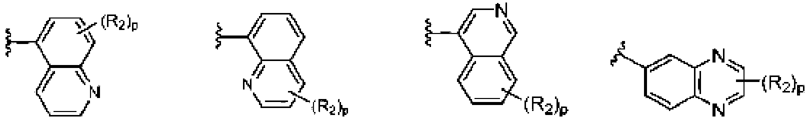 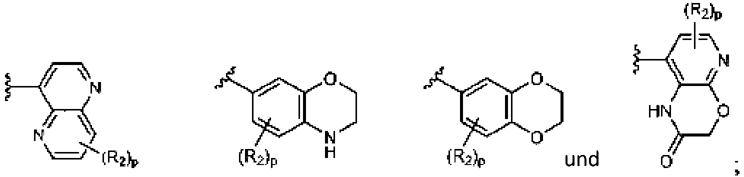
A ist Piperidinyl, Phenyl, Pyridinyl, Pyrimidinyl, 6-Azabicyclo[3.2.1]octanyl, oder Azabicyclo[3.2.1]octanyl, jeweils substituiert mit -L-R₄ und null bis 1 R_{4b};
L ist eine Bindung, -CRₓRₓ- oder -C(O)(CRₓRₓ)₀₋₂-;
R₁ ist Wasserstoff, C₁₋₃ Alkyl, C₁₋₂ Fluoralkyl oder C₃₋₄ Cycloalkyl;
jedes R₂ ist unabhängig Halogen, -CN, -OH, -NO₂, C₁₋₄ Alkyl, C₁₋₂ Fluoralkyl, C₁₋₂ Cyanoalkyl, C₁₋₃ Hydroxyalkyl, C₁₋₃ Aminoalkyl, -O(CH₂)₁₋₂OH, -(CH₂)₀₋₄O(C₁₋₄ Alkyl), C₁₋₃ Fluoralkoxy, -(CH₂)₁₋₄O(C₁₋₃ Alkyl), -O(CH₂)₁₋₂OC(O)(C₁₋₃ alkyl), -O(CH₂)₁₋₂NRₓRₓ, - C(O)O(C₁₋₃ Alkyl), -(CH₂)₀₋₂C(O)NR_{y}R_{y}, -C(O)NR_{X}(C₁₋₅ Hydroxyalkyl), -C(O)NRₓ(C₂₋₆ Alkoxyalkyl), -C(O)NRₓ(C₃₋₆ Cycloalkyl), -NR_{y}R_{y}, -NR_{y}(C₁₋₃ Fluoralkyl), -NR_{y}(C₁₋₄ Hydroxyalkyl), -NRₓCH₂(Phenyl), -NRₓS(O)₂(C₃₋₆ Cycloalkyl), -NRₓC(O)(C₁₋₃ Alkyl), - NRₓCH₂(C₃₋₆ Cycloalkyl), -S(O)₂(C₁₋₃ Alkyl), -S(O)₂N(C₁₋₃ Alkyl)₂, -S(O)(NH)N(C₁₋₃ alkyl)₂, -(CH₂)₀₋₂(C₃₋₆ Cycloalkyl), -(CH₂)₀₋₂(Phenyl), Morpholinyl, Dioxothiomorpholinyl, Dimethylpyrazolyl, Methylpiperidinyl, Methylpiperazinyl, Amino-oxadiazolyl, Imidazolyl, Triazolyl oder -C(O)(thiazolyl);
R₂ₐ ist C₁₋₆ Alkyl, C₁₋₃ Fluoralkyl, C₁₋₆ Hydroxyalkyl, C₁₋₃ Aminoalkyl, -(CH₂)₀₋₄O(C₁₋₃ Alkyl), C₃₋₆ Cycloalkyl, -(CH₂)₁₋₃C(O)NRₓRₓ, -CH₂(C₃₋₆ Cycloalkyl), -CH₂(Phenyl), Tetrahydrofuranyl, Tetrahydropyranyl oder Phenyl;
jedes R_{2b} ist unabhängig Wasserstoff, Halogen, -CN, -NRₓRₓ, C₁₋₆ Alkyl, C₁₋₃ Fluoralkyl, C₁₋₃ Hydroxyalkyl, C₁₋₃ Fluoralkoxy, -(CH₂)₀₋₂O(C₁₋₃ Alkyl), -(CH₂)₀₋₃C(O)NRₓRₓ, -(CH₂)₁₋₃(C₃₋₆ Cycloalkyl), -C(O)O(C₁₋₃ Alkyl), -C(O)NRₓ(C₁₋₃ Alkyl), -CRₓ=CRₓRₓ, oder - CRₓ=CH(C₃₋₆ Cycloalkyl);
R_{2c} ist R₂ₐ oder R_{2b};
R_{2d} ist R₂ₐ oder R_{2b}; vorausgesetzt dass eines von R_{2c} und R_{2d} gleich R₂ₐ ist und das andere von R_{2c} und R_{2d} gleich R_{2b} ist;
R₃ ist Wasserstoff, F, Cl, C₁₋₃ Alkyl, C₁₋₂ Fluoralkyl, oder C₃₋₄ Cycloalkyl;
R₄ Folgendes ist:
(i) -N(CH₃)₂;
(ii) Pyrrolidinyl, Piperidinyl, Piperazinyl, Pyridinyl, Azaspiro[3.3]heptanyl, Azabicyclo[3.2.1]octanyl, oder Diazabicyclo[3.2.1]octanyl, jeweils substituiert mit null bis 3 R₄ₐ und null bis 2 -CH₃; oder
(iii) 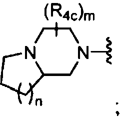
jedes R₄ₐ ist unabhängig -OH, C₁₋₆ Alkyl, C₁₋₃ Fluoralkyl, C₁₋₆ Hydroxyalkyl, C₃₋₆ Cycloalkyl, -CH₂(C₃₋₆ Cycloalkyl), -C(O)(C₁₋₄ Alkyl), -C(O)(C₃₋₆ Cycloalkyl), - C(O)(Phenyl), -C(O)CH₂(C₃₋₆ Cycloalkyl), -C(O)CH₂(Phenyl) oder -C(O)O(C₁₋₄ Alkyl);
R_{4b} ist F, Cl oder -CH₃;
jedes R_{4c} ist unabhängig C₁₋₆ Alkyl, C₁₋₃ Fluoralkyl, -CH₂(C₃₋₆ Cycloalkyl), -C(O)(C₁₋₄ Alkyl), -C(O)(Phenyl), -C(O)CH₂(Phenyl), -C(O)OCH₂CH₃ oder C₃₋₆ Cycloalkyl;
jedes R₅ ist unabhängig Wasserstoff, F, Cl, C₁₋₂ Alkyl, C₁₋₂ Fluoralkyl oder Cyclopropyl;
R₅ₐ ist Wasserstoff, C₁₋₂ Alkyl, C₁₋₂ Fluoralkyl oder Cyclopropyl;
jedes Rₓ ist unabhängig Wasserstoff oder -CH₃;
jedes R_{y} ist unabhängig Wasserstoff oder C₁₋₆ Alkyl;
m ist null, 1 oder 2;
n ist null, 1 oder 2;
p ist null, 1, 2, 3 oder 4; und
q ist 1 oder 2.

2. Verbindung nach Anspruch 1 oder Stereoisomere, Tautomere, Solvate oder Salze davon mit der Struktur der Formel (la-2):

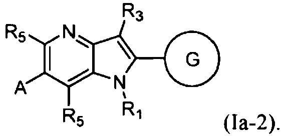

3. Verbindung nach Anspruch 1 oder Stereoisomere, Tautomere, Solvate oder Salze davon, wobei G Folgendes ist:

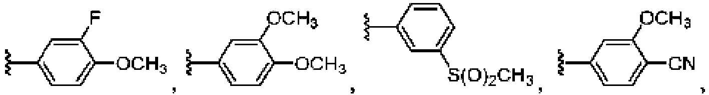

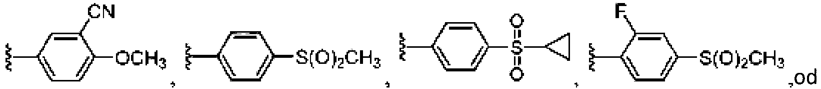

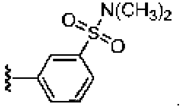

4. Verbindung nach Anspruch 1 oder Stereoisomere, Tautomere, Solvate oder Salze davon, wobei G Folgendes ist: 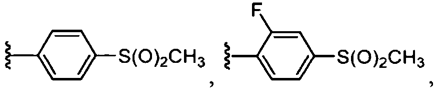 oder

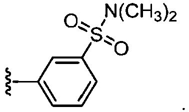

5. Verbindung nach Anspruch 1 oder Stereoisomere, Tautomere, Solvate oder Salze davon, wobei:
R₁ Wasserstoff oder -CH₃ ist;
jedes R₅ Wasserstoff ist;
G Phenyl substituiert mit 1 bis 2 Substituenten ist, unabhängig voneinander ausgewählt aus F, -CN, -OCH₃, -S(O)₂CH₃, -S(O)₂(Cyclopropyl), oder -S(O)₂N(CH₃)₂
A Piperidinyl, Phenyl oder Pyridinyl ist, jeweils substituiert mit -L-R₄;
L eine Bindung, -CH₂- oder -C(O)- ist;
R₃ Wasserstoff ist;
R₄ Folgendes ist:
(i) Piperidinyl, Piperazinyl, Pyridinyl, Azabicyclo[3.2.1]octanyl oder Diazabicyclo[3.2.1]octanyl, jeweils substituiert mit null bis 1 R₄ₐ und null bis 2-CH₃; oder
(ii) 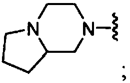
R₄ₐ -OH, -CH₃, -CH₂CH₃, -CH(CH₃)₂, -CH₂CH(CH₃)₂, -CH₂C(CH₃)₂OH, - CH₂CH₂C(CH₃)₂OH, -CH₂(Cyclopropyl) oder Cyclopropyl ist; und
jedes R₅ Wasserstoff oder -CH₃ ist.

6. Verbindung nach Anspruch 1 oder Stereoisomere, Tautomere, Solvate oder Salze davon, wobei:
R₁ Wasserstoff oder -CH₃ ist;
jedes R₅ Wasserstoff ist;
G Phenyl substituiert mit 1 bis 2 Substituenten ist, unabhängig voneinander ausgewählt aus F, -CN, -OCH₃, -S(O)₂CH₃, -S(O)₂(Cyclopropyl), oder -S(O)₂N(CH₃)₂
A Piperidinyl oder Phenyl ist, jeweils substituiert mit -L-R₄;
L eine Bindung oder -CH₂- ist;
R₃ Wasserstoff ist;
R₄ Piperazinyl oder Azabicyclo[3.2.1]octanyl ist, jedes substituiert mit null bis 1 R₄ₐ und null bis 2 -CH₃;
R₄ₐ -CH₂CH₃, -CH(CH₃)₂, -CH₂CH(CH₃)₂, -CH₂C(CH₃)₂OH, -CH₂(Cyclopropyl) oder Cyclopropyl ist; und
jedes R₅ Wasserstoff oder -CH₃ ist.

7. Verbindung nach Anspruch 1 mit folgender Struktur: 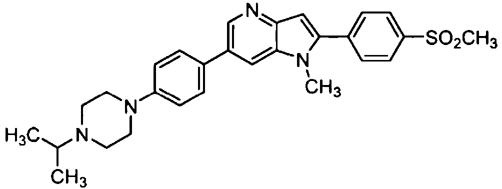 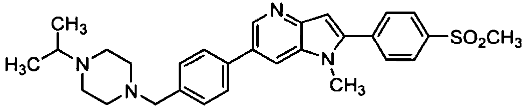 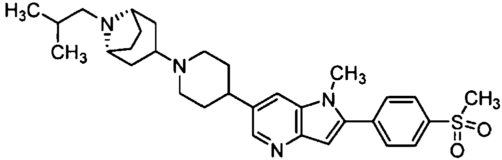 od 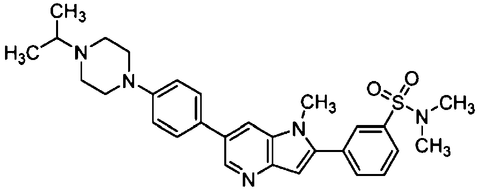 oder ein pharmazeutisch verträgliches Salz davon.

8. Verbindung nach Anspruch 1 mit folgender Struktur: 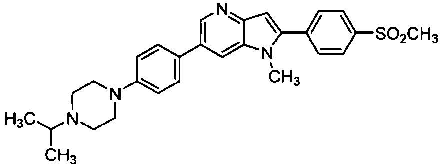 oder ein pharmazeutisch verträgliches Salz davon.

9. Verbindung nach Anspruch 1 mit folgender Struktur: 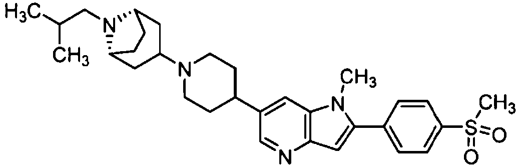 oder ein pharmazeutisch verträgliches Salz davon.

10. Verbindung nach Anspruch 1 mit folgender Struktur: 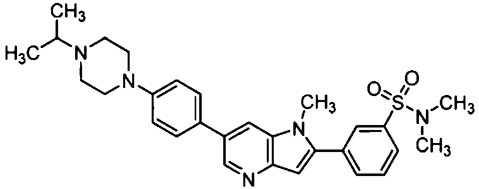 oder ein pharmazeutisch verträgliches Salz davon.

11. Verbindung nach Anspruch 1 oder Stereoisomere, Tautomere, Solvate oder Salze davon, wobei die Verbindung Folgendes ist:
2-(3,4-Dimethoxyphenyl)-6-(4-(4-isopropylpiperazin-1-yl)phenyl)-1-methyl-1H-pyrrolo[3,2-b]pyridin (1);
6-(4-(4-Isopropylpiperazin-1-yl)phenyl)-1-methyl-2-(4-(methylsulfonyl)phenyl)-1H-pyrrolo[3,2-b]pyridin (2);
1-(4-(4-(2-(3,4-Dimethoxyphenyl)-1-methyl-1H-pyrrolo[3,2-b]pyridin-6-yl)phenyl)piperazin-1-yl)-2-methylpropan-2-ol (4);
(4-(2-(3,4-Dimethoxyphenyl)-1-methyl-1H-pyrrolo[3,2-b]pyridin-6-yl)phenyl)(4-isopropylpiperazin-1-yl)methanon (5);
6-(4-(4-Isobutylpiperazin-1-yl)phenyl)-1-methyl-2-(4-(methylsulfonyl)phenyl)-1H-pyrrolo[3,2-b]pyridin (6);
6-(4-(4-(Cyclopropylmethyl)piperazin-1-yl)phenyl)-2-(3,4-dimethoxyphenyl)-1-methyl-1H-pyrrolo[3,2-b]pyridin (8);
2-(3,4-Dimethoxyphenyl)-6-(6-(4-isopropylpiperazin-1-yl)pyridin-3-yl)-1-methyl-1H-pyrrolo[3,2-b]pyridin (9);
2-(3-Fluor-4-methoxyphenyl)-6-(4-(4-isopropylpiperazin-1-yl)phenyl)-1-methyl-1H-pyrrolo[3,2-b]pyridin (10);
6-(4-(4-Isopropylpiperazin-1-yl)phenyl)-2-(4-(methylsulfonyl)phenyl)-1H-pyrrolo[3,2-b]pyridin (11);
4-(4-(4-(2-(3,4-Dimethoxyphenyl)-1-methyl-1H-pyrrolo[3,2-b]pyridin-6-yl) phenyl)piperazin-1-yl)-2-methylbutan-2-ol (12);
2-(3,4-Dimethoxyphenyl)-1-methyl-6-(4-(piperazin-1-yl)phenyl)-1H-pyrrolo[3,2-b]pyridin (13);
6-(4-(4-Usobutylpiperazin-1-yl)phenyl)-2-(4-(methylsulfonyl)phenyl)-1H-pyrrolo[3,2-b]pyridin (14);
2-Methyl-1-(4-(4-(1-methyl-2-(4-(methylsulfonyl)phenyl)-1H-pyrrolo[3,2-b]pyridin-6-yl)phenyl)piperazin-1-yl)propan-2-ol (15);
2-Methyl-4-(4-(4-(1-methyl-2-(4-(methylsulfonyl)phenyl)-1H-pyrrolo[3,2-b]pyridin-6-yl)phenyl)piperazin-1-yl)butan-2-ol (16); oder
6-(4-(4-Isobutylpiperazin-1-yl)phenyl)-4-methyl-2-(4-(methylsulfonyl)phenyl)-4H-pyrrolo[3,2-b]pyridin (18);
6-(4-(4-isopropylpiperazin-1-yl)phenyl)-1-methyl-2-(3-(methylsulfonyl)phenyl)-1H-pyrrolo[3,2-b]pyridin (19);
1-methyl-2-(4-(methylsulfonyl)phenyl)-6-(4-(piperazin-1-yl)phenyl)-1H-pyrrolo[3,2-b]pyridin (20);
2-Methyl-1-(4-(4-(1-methyl-2-(4-(methylsulfonyl)phenyl)-1H-pyrrolo[3,2-b]pyridin-6-yl)benzyl)piperazin-1-yl)propan-2-ol (21);
4-Methyl-1-(4-(1-methyl-2-(4-(methylsulfonyl)phenyl)-1H-pyrrolo[3,2-b]pyridin-6-yl)benzyl)piperidin-4-ol (22);
6-(4-((4-Isopropylpiperazin-1-yl)methyl)phenyl)-1-methyl-2-(4-(methylsulfonyl) phenyl)-1H-pyrrolo[3,2-b]pyridin (23);
6-(4-((4-Ethylpiperazin-1-yl)methyl)phenyl)-1-methyl-2-(4-(methylsulfonyl)phenyl)-1H-pyrrolo[3,2-b]pyridin (25);
6-(4-(8-Isopropyl-3,8-diazabicyclo[3.2.1]octan-3-yl)phenyl)-1-methyl-2-(4-(methylsulfonyl)phenyl)-1H-pyrrolo[3,2-b]pyridin (26);
6-(4-(Hexahydropyrrolo[1,2-a]pyrazin-2(1H)-yl)phenyl)-1-methyl-2-(4-(methylsulfonyl)phenyl)-1H-pyrrolo[3,2-b]pyridin (27);
(R)-6-(4-(4-Isopropyl-2-methylpiperazin-1-yl)phenyl)-1-methyl-2-(4-(methylsulfonyl)phenyl)-1H-pyrrolo[3,2-b]pyridin (28);
(S)-6-(4-(4-Isopropyl-3-methylpiperazin-1-yl)phenyl)-1-methyl-2-(4-(methylsulfonyl)phenyl)-1H-pyrrolo[3,2-b]pyridin (29);
(S)-6-(4-(4-Isopropyl-2-methylpiperazin-1-yl)phenyl)-1-methyl-2-(4-(methylsulfonyl)phenyl)-1H-pyrrolo[3,2-b]pyridin (30);
6-(4-((2S,6S)-4-Isopropyl-2,6-dimethylpiperazin-1-yl)phenyl)-1-methyl-2-(4-(methylsulfonyl)phenyl)-1H-pyrrolo[3,2-b]pyridin (31);
(R)-6-(4-(4-Isopropyl-3-methylpiperazin-1-yl)phenyl)-1-methyl-2-(4-(methylsulfonyl)phenyl)-1H-pyrrolo[3,2-b]pyridin (32);
6-(4-((2R,6S)-4-Isopropyl-2,6-dimethylpiperazin-1-yl)phenyl)-1-methyl-2-(4-(methylsulfonyl)phenyl)-1H-pyrrolo[3,2-b]pyridin (33);
6-(4-((3S,5R)-4-Isopropyl-3,5-dimethylpiperazin-1-yl)phenyl)-1-methyl-2-(4-(methylsulfonyl)phenyl)-1H-pyrrolo[3,2-b]pyridin (34);
6-(4-((3R,5R)-4-Isopropyl-3,5-dimethylpiperazin-1-yl)phenyl)-1-methyl-2-(4-(methylsulfonyl)phenyl)-1H-pyrrolo[3,2-b]pyridin (35);
6-(1-((1R,5S)-8-Isobutyl-8-azabicyclo[3.2.1]octan-3-yl)piperidin-4-yl)-1-methyl-2-(4-(methylsulfonyl)phenyl)-1H-pyrrolo[3,2-b]pyridin (36);
2-(4-Cyclopropylsulfonylphenyl)-1-methyl-6-[1-[rac-(1S,5R)-8-isobutyl-8-azabicyclo[3.2.1]octan-3-yl]-4-piperidyl]pyrrolo[3,2-b]pyridin (37);
4-[6-[4-(4-Isopropylpiperazin-1-yl)phenyl]-1-methyl-pyrrolo[3,2-b]pyridin-2-yl]-2-methoxy-benzonitril (38);
5-(6-(4-(4-Isopropylpiperazin-1-yl)phenyl)-1-methyl-1H-pyrrolo[3,2-b]pyridin-2-yl)-2-methoxybenzonitril (39);
3-(6-(4-(4-Isopropylpiperazin-1-yl)phenyl)-1-methyl-1H-pyrrolo[3,2-b]pyridin-2-yl)-N,N-dimethylbenzensulfonamid (40);
2-(2-Fluoro-4-(methylsulfonyl)phenyl)-6-(4-(4-isopropylpiperazin-1-yl)phenyl)-1-methyl-1H-pyrrolo[3,2-b]pyridin (41);
2-(2-Fluor-4-(methylsulfonyl)phenyl)-6-(4-(hexahydropyrrolo[1,2-a]pyrazin-2(1H)-yl)phenyl)-1-methyl-1H-pyrrolo[3,2-b]pyridin (42);
2-(2-Fluor-4-(methylsulfonyl)phenyl)-6-(4-(8-isopropyl-3,8-diazabicyclo[3.2.1]octan-3-yl)phenyl)-1-methyl-1H-pyrrolo[3,2-b]pyridin (43);
3-(6-(4-(4-Cyclopropylpiperazin-1-yl)phenyl)-1-methyl-1H-pyrrolo[3,2-b]pyridin-2-yl)-N,N-dimethylbenzensulfonamid (44); oder
6-(4-(4-Cyclopropylpiperazin-1-yl)phenyl)-2-(2-fluoro-4-(methylsulfonyl)phenyl)-1-methyl-1H-pyrrolo[3,2-b]pyridin (45).

12. Verbindung nach Anspruch 1 oder ein Salz davon, wobei die Verbindung Folgendes ist:
6-(4-(4-Isopropylpiperazin-1-yl)phenyl)-4-methyl-2-(4-(methylsulfonyl)phenyl)-4H-pyrrolo[3,2-b]pyridin (3); oder
6-(4-(4-Isobutylpiperazin-1-yl)phenyl)-4-methyl-2-(4-(methylsulfonyl)phenyl)-4H-pyrrolo[3,2-b]pyridin (17).

13. Pharmazeutische Zusammensetzung, umfassend eine oder mehrere Verbindungen nach einem der Ansprüche 1 bis 12 und einen pharmazeutisch verträglichen Träger oder Verdünner.

14. Verbindung nach einem der Ansprüche 1 bis 12 oder Stereoisomere, Tautomere, Solvate oder pharmazeutisch verträgliche Salze davon oder eine pharmazeutische Zusammensetzung nach Anspruch 13, zur Verwendung bei der Behandlung pathologischer Fibrose.

15. Verbindung oder Stereoisomere, Tautomere, Solvate oder ein pharmazeutisch verträgliches Salz davon oder pharmazeutisch verträgliche Salze davon, oder pharmazeutische Zusammensetzung, zur Verwendung nach Anspruch 14, wobei es sich bei der pathologischen Fibrose um Leberfibrose, Nierenfibrose, Gallenwegsfibrose oder Pankreasfibrose handelt.

16. Verbindung nach einem der Ansprüche 1 bis 12 oder Stereoisomere, Tautomere, Solvate oder pharmazeutisch verträgliche Salze davon oder eine pharmazeutische Zusammensetzung nach Anspruch 13, zur Verwendung bei der Behandlung von nichtalkoholischer Steatohepatitis (NASH), nichtalkoholischer Fettlebererkrankung (NAFLD), chronischer Nierenerkrankung, diabetischer Nierenerkrankung, primär sklerosierender Cholangitis (PSC) oder primär biliärer Zirrhose (PBC).

17. Verbindung nach einem der Ansprüche 1 bis 12 oder Stereoisomere, Tautomere, Solvate oder pharmazeutisch verträgliche Salze davon oder eine pharmazeutische Zusammensetzung nach Anspruch 13, zur Verwendung bei der Behandlung der idiopathischen Lungenfibrose (IPF).

## Revendications

1. Composé de Formule (la-2) ou de formule (IIb) : 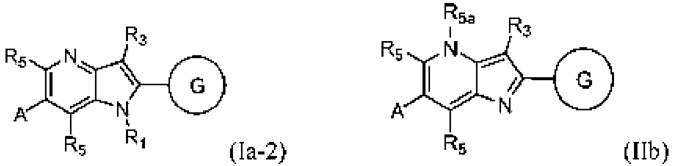 ou stéréoisomères, tautomère, solvates ou sels de ceux-ci fournis, dans lesquels :
G est :
(i) phényle substitué par 1 à 3 substituants choisis indépendamment parmi F, CI, Br, -CN, alcoxy en C₁₋₂, fluoroalcoxy en C₁₋₂, cycloalkyle en C₃₋₄ -C(O)NR_{y}R_{y}, -S(O)₂CH₃, -S(O)₂(phényle), -S(O)₂(cyclopropyle), -S(O)₂NRₓRₓ et -S(O)(NH)NRₓRₓ ;
(ii) 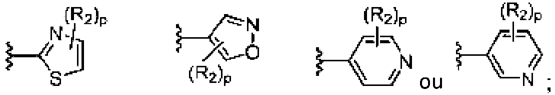
(iii) 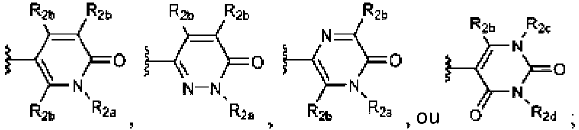
(iv) 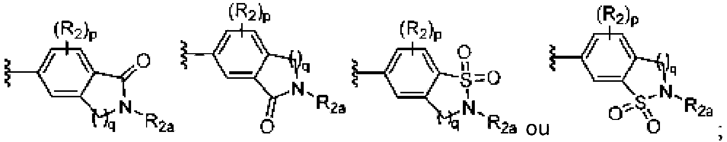
(v) un cycle hétérocyclique à 9 chaînons choisi parmi : 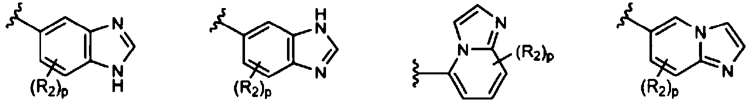 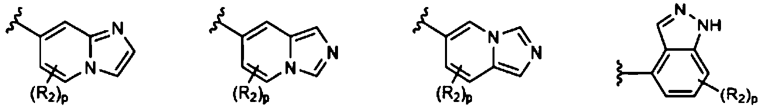 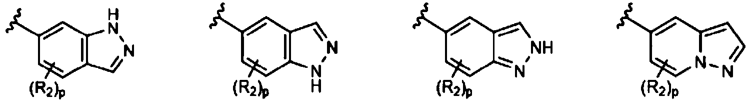 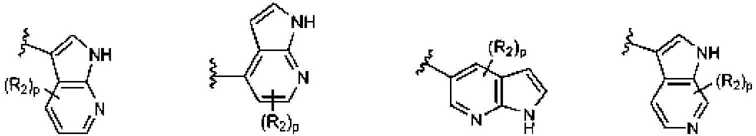 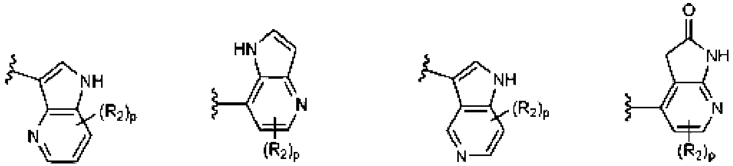 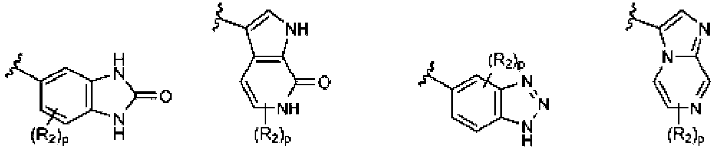 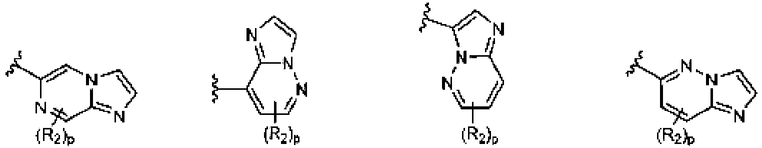 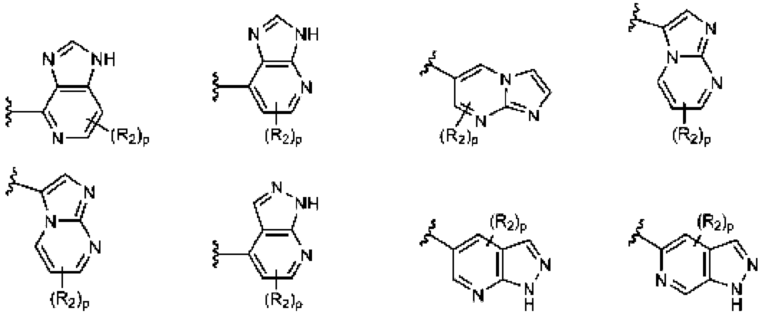 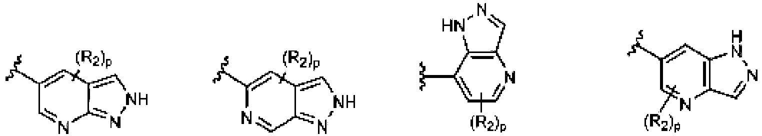 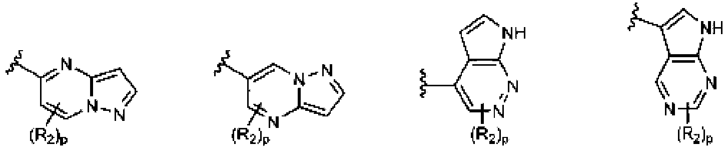 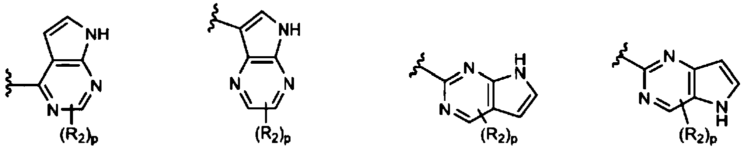 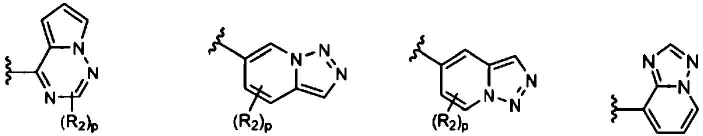 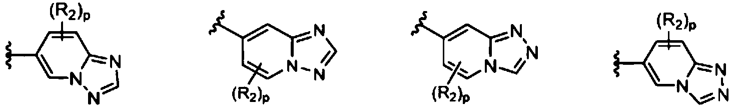 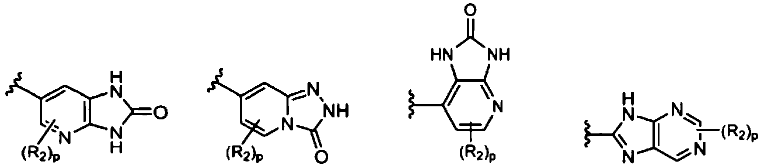 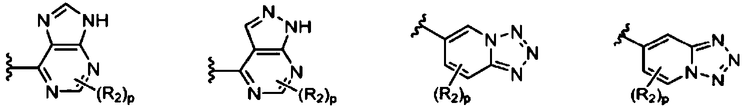 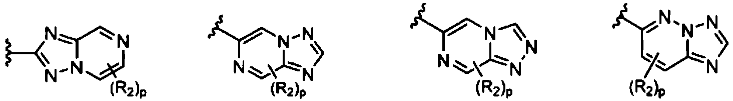 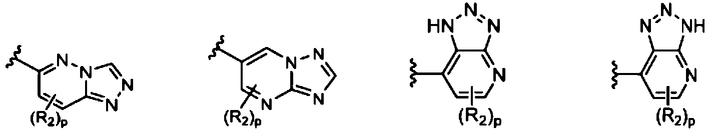 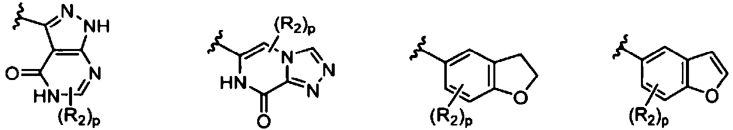 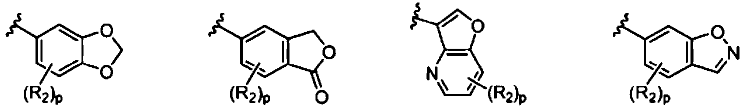 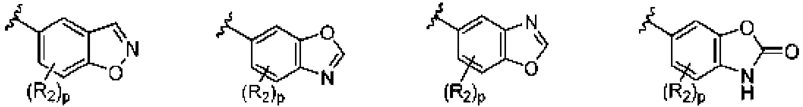 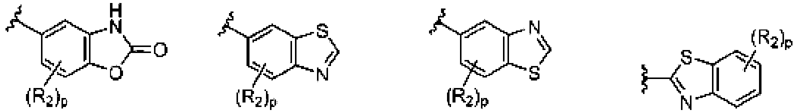 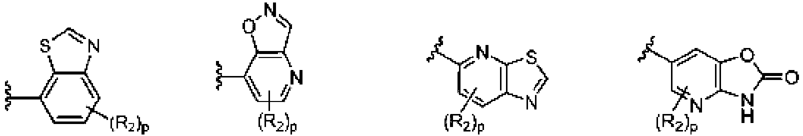 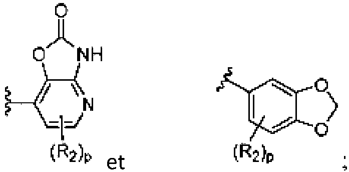 ou
(vi) un cycle hétérocyclique à 10 chaînons choisi parmi : 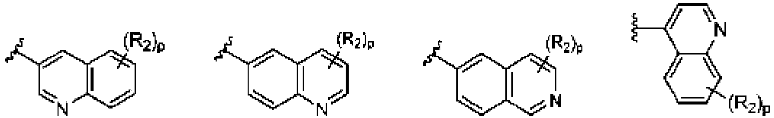 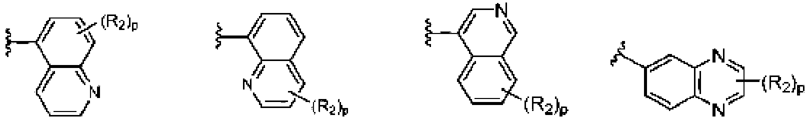 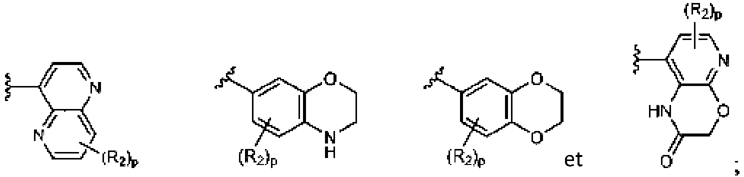
A est pipéridinyle, phényle, pyridinyle, pyrimidinyle, 6-azabicyclo[3.2.1]octanyl ou azabicyclo[3.2.1]octanyl, chacun substitué par -L-R₄ et zéro à 1 R_{4b} ;
L est une liaison, -CRₓRₓ- ou -C(O)(CRₓRₓ)₀₋₂- ;
R₁ est hydrogène, alkyle en C₁₋₃, fluoroalkyle en C₁₋₂ ou cycloalkyle en C₃₋₄;
chaque R₂ est indépendamment halo, -CN, -OH, -NO₂, alkyle en C₁₋₄, fluoroalkyle en C₁₋₂, cyanoalkyle en C₁₋₂, hydroxyalkyle en C₁₋₃, aminoalkyle en C₁₋₃, - O(CH₂)₁₋₂OH, -(CH₂)₀₋₄O(alkyle en C₁₋₄), fluoroalcoxy en C₁₋₃, -(CH₂)₁₋₄O(alkyle en C₁₋₃), - O(CH₂)₁₋₂OC(O)(alkyle en C₁₋₃), -O(CH₂)₁₋₂NRₓRₓ, -C(O)O(alkyle en C₁₋₃), -(CH₂)₀₋₂C(O)NR_{y}R_{y}, -C(O)NRₓ(hydroxyalkyle en C₁₋₅), -C(O)NRₓ(alcoxyalkyle en C₂₋₆), - C(O)NRₓ(cycloalkyle en C₃₋₆), -NR_{y}R_{y}, -NR_{y}(fluoroalkyle en C₁₋₃), -NR_{y}(hydroxyalkyle en C₁₋₄), -NRₓCH₂(phényle), -NRₓS(O)₂(cycloalkyle en C₃₋₆), -NRₓC(O)(alkyle en C₁₋₃), - NRₓCH₂(cycloalkyle en C₃₋₆), -S(O)₂(alkyle en C₁₋₃), -S(O)₂N(alkyle en C₁₋₃)₂, - S(O)(NH)N(alkyle en C₁₋₃)₂, -(CH₂)₀₋₂(cycloalkyle en C₃₋₆), -(CH₂)₀₋₂(phényle), morpholinyle, dioxothiomorpholinyle, diméthyl pyrazolyle, méthylpipéridinyle, méthylpipérazinyle, amino-oxadiazolyle, imidazolyle, triazolyle ou -C(O)(thiazolyle) ;
R₂ₐ est alkyle en C₁₋₆, fluoroalkyle en C₁₋₃, hydroxyalkyle en C₁₋₆, aminoalkyle en C₁₋₃, -(CH₂)₀₋₄O(alkyle en C₁₋₃), cycloalkyle en C₃₋₆, -(CH₂)₁₋₃C(O)NRₓRₓ, -CH₂(cycloalkyle en C₃₋₆), -CH₂(phényle), tétrahydrofuranyle, tétrahydropyranyle ou phényle ;
chaque R_{2b} est indépendamment hydrogène, halo, -CN, -NRₓRₓ, alkyle en C₁₋₆, fluoroalkyle en C₁₋₃, hydroxyalkyle en C₁₋₃, fluoroalcoxy en C₁₋₃, -(CH₂)₀₋₂O(alkyle en C₁₋₃), -(CH₂)₀₋₃C(O)NRₓRₓ, -(CH₂)₁₋₃(cycloalkyle en C₃₋₆), -C(O)O(alkyle en C₁₋₃), - C(O)NRₓ(alkyle en C₁₋₃), -CRₓ=CRₓRₓ ou -CRₓ=CH(cycloalkyle en C₃₋₆) ;
R_{2c} est R₂ₐ ou R_{2b} ;
R_{2d} est R₂ₐ ou R_{2b} ; à condition qu'un parmi R_{2c} et R_{2d} soit R₂ₐ, et que l'autre parmi R_{2c} et R_{2d} soit R_{2b} ;
R₃ est hydrogène, F, CI, alkyle en C₁₋₃, fluoroalkyle en C₁₋₂ ou cycloalkyle en C₃₋₄ ;
R₄ est :
(i) -N(CH₃)₂
(ii) pyrrolidinyle, pipéridinyle, pipérazinyle, pyridinyle, azaspiro[3.3]heptanyle, azabicyclo[3.2.1]octanyl ou diazabicyclo[3.2.1]octanyl, chacun substitué par zéro à 3 R₄ₐ et zéro à 2 -CH₃ ; ou
(iii) 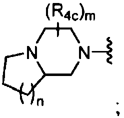
chaque R₄ₐ est indépendamment -OH, alkyle en C₁₋₆, fluoroalkyle en C₁₋₃, hydroxyalkyle en C₁₋₆, cycloalkyle en C₃₋₆, -CH₂(cycloalkyle en C₃₋₆), -C(O)(alkyle en C₁₋₄), -C(O)(cycloalkyle en C₃₋₆), -C(O)(phényle), -C(O)CH₂(cycloalkyle en C₃₋₆), - C(O)CH₂(phényle) ou -C(O)O(alkyle en C₁₋₄) ;
R_{4b} est F, Cl ou -CH₃ ;
chaque R_{4c} est indépendamment alkyle en C₁₋₆, fluoroalkyle en C₁₋₃, - CH₂(cycloalkyle en C₃₋₆), -C(O)(alkyle en C₁₋₄), -C(O)(phényle), -C(O)CH₂(phényle), - C(O)OCH₂CH₃ ou cycloalkyle en C₃₋₆ ;
chaque R₅ est indépendamment hydrogène, F, CI, alkyle en C₁₋₂, fluoroalkyle en C₁₋₂ ou cyclopropyle ;
R₅ₐ est hydrogène, alkyle en C₁₋₂, fluoroalkyle en C₁₋₂ ou cyclopropyle ;
chaque Rₓ est indépendamment hydrogène ou -CH₃ ;
chaque R_{y} est indépendamment hydrogène ou alkyle en C₁₋₆ ;
m est zéro, 1 ou 2 ;
n est zéro, 1 ou 2 ;
p est zéro, 1, 2, 3 ou 4; et
q est 1 ou 2.

2. Composé selon la revendication 1 ou stéréoisomères, tautomère, solvates ou sels de ceux-ci, présentant la structure de Formule (la-2) :

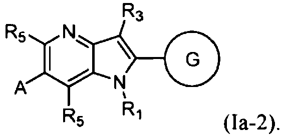

3. Composé selon la revendication 1 ou stéréoisomères, tautomère, solvates ou sels de ceux-ci, dans lesquels G est :

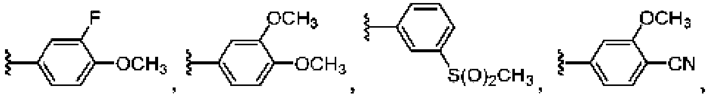

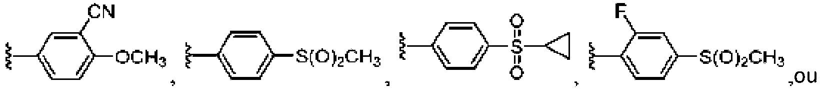

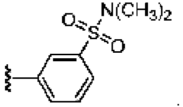

4. Composé selon la revendication 1 ou stéréoisomères, tautomère, solvates ou sels de ceux-ci, dans lesquels G est: 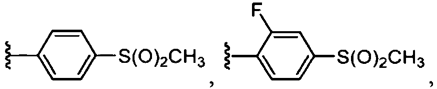 ou

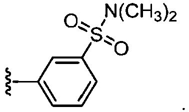

5. Composé selon la revendication 1 ou stéréoisomères, tautomère, solvates ou sels de ceux-ci, dans lesquels :
R₁ est hydrogène ou -CH₃ ;
chaque R₅ est hydrogène ;
G est phényle substitué par 1 ou 2 substituants choisis indépendamment parmi F, -CN, -OCH₃, -S(O)₂CH₃, -S(O)₂(cyclopropyle) ou -S(O)₂N(CH₃)₂ ;
A est pipéridinyle, phényle ou pyridinyle, chacun substitué par -L-R₄ ;
L est une liaison, -CH₂- ou -C(O)- ;
R₃ est hydrogène ;
R₄ est :
(i) pipéridinyle, pipérazinyle, pyridinyle, azabicyclo[3.2.1]octanyl ou diazabicyclo[3.2.1]octanyl, chacun substitué par zéro à 1 R₄ₐ et zéro à 2 -CH₃ ; ou
(ii) 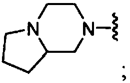
R₄ₐ est -OH, -CH₃, -CH₂CH₃, -CH(CH₃)₂, -CH₂CH(CH₃)₂, -CH₂C(CH₃)₂OH, - CH₂CH₂C(CH₃)₂OH, -CH₂(cyclopropyle) ou cyclopropyle ; et
chaque R₅ est hydrogène ou -CH₃.

6. Composé selon la revendication 1 ou stéréoisomères, tautomère, solvates ou sels de ceux-ci, dans lesquels :
R₁ est hydrogène ou -CH₃ ;
chaque R₅ est hydrogène ;
G est phényle substitué par 1 ou 2 substituants choisis indépendamment parmi F, -CN, -OCH₃, -S(O)₂CH₃, -S(O)₂(cyclopropyle) ou -S(O)₂N(CH₃)₂ ;
A est pipéridinyle ou phényle, chacun substitué par -L-R₄;
L est une liaison ou -CH₂- ;
R₃ est hydrogène ;
R₄ est pipérazinyle ou azabicyclo[3.2.1]octanyl, chacun substitué par zéro à 1 R₄ₐ et zéro à 2 -CH₃ ;
R₄ₐ est -CH₂CH₃, -CH(CH₃)₂, -CH₂CH(CH₃)₂, -CH₂C(CH₃)₂OH, -CH₂(cyclopropyle) ou cyclopropyle ; et
chaque R₅ est hydrogène ou -CH₃.

7. Composé selon la revendication 1 présentant la structure : 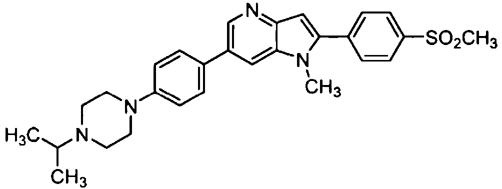 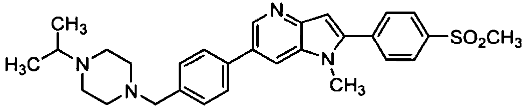 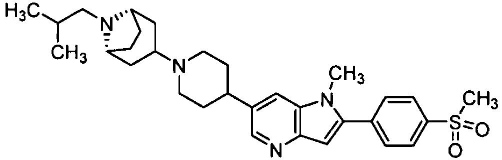 ou 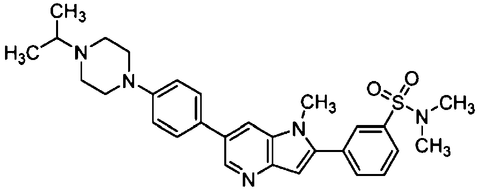 ou sel pharmaceutiquement acceptable de celui-ci.

8. Composé selon la revendication 1 présentant la structure : 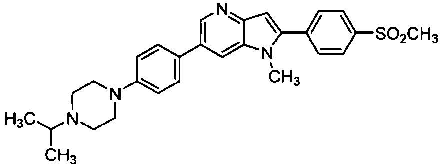 ou sel pharmaceutiquement acceptable de celui-ci.

9. Composé selon la revendication 1 présentant la structure : 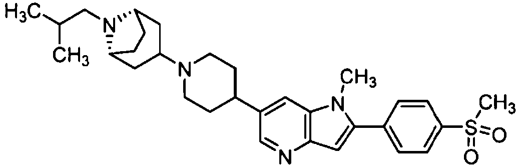 ou sel pharmaceutiquement acceptable de celui-ci.

10. Composé selon la revendication 1 présentant la structure : 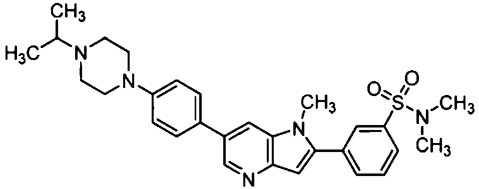 ou sel pharmaceutiquement acceptable de celui-ci.

11. Composé selon la revendication 1 ou stéréoisomères, tautomère, solvates ou sels de ceux-ci, dans lesquels ledit composé est :
2-(3,4-diméthoxyphényle)-6-(4-(4-isopropylpipérazine-1-yl)phényle)-1-méthyle-1H-pyrrolo[3,2-b]pyridine (1) ;
6-(4-(4-isopropylpipérazine-1-yl)phényle)-1-méthyle-2-(4-(méthylsulfonyle)phényle)-1H-pyrrolo[3,2-b]pyridine (2) ;
1-(4-(4-(2-(3,4-diméthoxyphényle)-1-méthyle-1H-pyrrolo[3,2-b]pyridine-6-yl)phényle)pipérazine-1-yl)-2-méthylpropane-2-ol (4) ;
(4-(2-(3,4-diméthoxyphényle)-1-méthyle-1H-pyrrolo[3,2-b]pyridine-6-yl)phényle)(4-isopropylpipérazine-1-yl)méthanone (5) ;
6-(4-(4-isobutylpipérazine-1-yl)phényle)-1-méthyle-2-(4-(méthylsulfonyle)phényle)-1H-pyrrolo[3,2-b]pyridine (6) ;
6-(4-(4-(cyclopropylméthyle)pipérazine-1-yl)phényle)-2-(3,4-diméthoxyphényle)-1-méthyle-1H-pyrrolo[3,2-b]pyridine (8) ;
2-(3,4-diméthoxyphényle)-6-(6-(4-isopropylpipérazine-1-yl)pyridine-3-yl)-1-méthyle-1H-pyrrolo[3,2-b]pyridine (9) ;
2-(3-fluoro-4-méthoxyphényle)-6-(4-(4-isopropylpipérazine-1-yl)phényle)-1-méthyle-1H-pyrrolo[3,2-b]pyridine (10) ;
6-(4-(4-isopropylpipérazine-1-yl)phényle)-2-(4-(méthylsulfonyle)phényle)-1H-pyrrolo[3,2-b]pyridine (11) ;
4-(4-(4-(2-(3,4-diméthoxyphényle)-1-méthyle-1H-pyrrolo[3,2-b]pyridine-6-yl)phényle)pipérazine-1-yl)-2-méthylbutane-2-ol (12) ;
2-(3,4-diméthoxyphényle)-1-méthyle-6-(4-(pipérazine-1-yl)phényle)-1H-pyrrolo[3,2-b]pyridine (13) ;
6-(4-(4-isobutylpipérazine-1-yl)phényle)-2-(4-(méthylsulfonyle)phényle)-1H-pyrrolo[3,2-b]pyridine (14) ;
2-méthyle-1-(4-(4-(1-méthyle-2-(4-(méthylsulfonyle)phényle)-1H-pyrrolo[3,2-b]pyridine-6-yl)phényle)pipérazine-1-yl)propane-2-ol (15) ;
2-méthyle-4-(4-(4-(1-méthyle-2-(4-(méthylsulfonyle)phényle)-1H-pyrrolo[3,2-b]pyridine-6-yl)phényle)pipérazine-1-yl)butane-2-ol (16) ; ou
6-(4-(4-isobutylpipérazine-1-yl)phényle)-4-méthyle-2-(4-(méthylsulfonyle)phényle)-4H-pyrrolo[3,2-b]pyridine (18) ;
6-(4-(4-isopropylpipérazine-1-yl)phényle)-1-méthyle-2-(3-(méthylsulfonyle)phényle)-1H-pyrrolo[3,2-b]pyridine (19) ;
1-méthyle-2-(4-(méthylsulfonyle)phényle)-6-(4-(pipérazine-1-yl)phényle)-1H-pyrrolo[3,2-b]pyridine (20) ;
2-méthyle-1-(4-(4-(1-méthyle-2-(4-(méthylsulfonyle)phényle)-1H-pyrrolo[3,2-b]pyridine-6-yl)benzyle)pipérazine-1-yl)propane-2-ol (21) ;
4-méthyle-1-(4-(1-méthyle-2-(4-(méthylsulfonyle)phényle)-1H-pyrrolo[3,2-b]pyridine-6-yl)benzyle)pipéridine-4-ol (22) ;
6-(4-((4-isopropylpipérazine-1-yl)méthyle)phényle)-1-méthyle-2-(4-(méthylsulfonyle)phényle)-1H-pyrrolo[3,2-b]pyridine (23) ;
6-(4-((4-éthylpipérazine-1-yl)méthyle)phényle)-1-méthyle-2-(4-(méthylsulfonyle)phényle)-1H-pyrrolo[3,2-b]pyridine (25) ;
6-(4-(8-isopropyle-3,8-diazabicyclo[3.2.1]octane-3-yl)phényle)-1-méthyle-2-(4-(méthylsulfonyle)phényle)-1H-pyrrolo[3,2-b]pyridine (26) ;
6-(4-(hexahydropyrrolo[1,2-a]pyrazine-2(1H)-yl)phényle)-1-méthyle-2-(4-(méthylsulfonyle)phényle)-1H-pyrrolo[3,2-b]pyridine (27) ;
(R)-6-(4-(4-isopropyle-2-méthylpipérazine-1-yl)phényle)-1-méthyle-2-(4-(méthylsulfonyle)phényle)-1H-pyrrolo[3,2-b]pyridine (28) ;
(S)-6-(4-(4-isopropyle-3-méthylpipérazine-1-yl)phényle)-1-méthyle-2-(4-(méthylsulfonyle)phényle)-1H-pyrrolo[3,2-b]pyridine (29) ;
(S)-6-(4-(4-isopropyle-2-méthylpipérazine-1-yl)phényle)-1-méthyle-2-(4-(méthylsulfonyle)phényle)-1H-pyrrolo[3,2-b]pyridine (30) ;
6-(4-((2S,6S)-4-isopropyle-2,6-diméthylpipérazine-1-yl)phényle)-1-méthyle-2-(4-(méthylsulfonyle)phényle)-1H-pyrrolo[3,2-b]pyridine (31) ;
(R)-6-(4-(4-isopropyle-3-méthylpipérazine-1-yl)phényle)-1-méthyle-2-(4-(méthylsulfonyle)phényle)-1H-pyrrolo[3,2-b]pyridine (32) ;
6-(4-((2R,6S)-4-isopropyle-2,6-diméthylpipérazine-1-yl)phényle)-1-méthyle-2-(4-(méthylsulfonyle)phényle)-1H-pyrrolo[3,2-b]pyridine (33) ;
6-(4-((3S,5R)-4-isopropyle-3,5-diméthylpipérazine-1-yl)phényle)-1-méthyle-2-(4-(méthylsulfonyle)phényle)-1H-pyrrolo[3,2-b]pyridine (34) ;
6-(4-((3R,5R)-4-isopropyle-3,5-diméthylpipérazine-1-yl)phényle)-1-méthyle-2-(4-(méthylsulfonyle)phényle)-1H-pyrrolo[3,2-b]pyridine (35) ;
6-(1-((1R,5S)-8-isobutyle-8-azabicyclo[3.2.1]octane-3-yl)pipéridine-4-yl)-1-méthyle-2-(4-(méthylsulfonyle)phényle)-1H-pyrrolo[3,2-b]pyridine (36) ;
2-(4-cyclopropylsulfonylphényle)-1-méthyle-6-[1-[rac-(1S,5R)-8-isobutyle-8-azabicyclo[3.2.1]octane-3-yl]-4-pipéridyle]pyrrolo[3,2-b]pyridine (37) ;
4-[6-[4-(4-isopropylpipérazine-1-yl)phényle]-1-méthyle-pyrrolo[3,2-b]pyridine-2-yl]-2-méthoxy-benzonitrile (38) ;
5-(6-(4-(4-isopropylpipérazine-1-yl)phényle)-1-méthyle-1H-pyrrolo[3,2-b]pyridine-2-yl)-2-méthoxybenzonitrile (39) ;
3-(6-(4-(4-isopropylpipérazine-1-yl)phényle)-1-méthyle-1H-pyrrolo[3,2-b]pyridine-2-yl)-N,N-diméthylbenzènesulfonamide (40) ;
2-(2-fluoro-4-(méthylsulfonyle)phényle)-6-(4-(4-isopropylpipérazine-1-yl)phényle)-1-méthyle-1H-pyrrolo[3,2-b]pyridine (41) ;
2-(2-fluoro-4-(méthylsulfonyle)phényle)-6-(4-(hexahydropyrrolo[1,2-a]pyrazine-2(1H)-yl)phényle)-1-méthyle-1H-pyrrolo[3,2-b]pyridine (42) ;
2-(2-fluoro-4-(méthylsulfonyle)phényle)-6-(4-(8-isopropyle-3,8-diazabicyclo[3.2.1]octane-3-yl)phényle)-1-méthyle-1H-pyrrolo[3,2-b]pyridine (43) ;
3-(6-(4-(4-cyclopropylpipérazine-1-yl)phényle)-1-méthyle-1H-pyrrolo[3,2-b]pyridine-2-yl)-N,N-diméthylbenzènesulfonamide (44) ; ou
6-(4-(4-cyclopropylpipérazine-1-yl)phényle)-2-(2-fluoro-4-(méthylsulfonyle)phényle)-1-méthyle-1H-pyrrolo[3,2-b]pyridine (45).

12. Composé selon la revendication 1 ou sel de celui-ci, dans lesquels ledit composé est :
6-(4-(4-isopropylpipérazine-1-yl)phényle)-4-méthyle-2-(4-(méthylsulfonyle)phényle)-4H-pyrrolo[3,2-b]pyridine (3) ; ou
6-(4-(4-isobutylpipérazine-1-yl)phényle)-4-méthyle-2-(4-(méthylsulfonyle)phényle)-4H-pyrrolo[3,2-b]pyridine (17).

13. Composition pharmaceutique comprenant un ou plusieurs composés selon l'une quelconque des revendications 1 à 12 et un vecteur ou un diluant pharmaceutiquement acceptable.

14. Composé selon l'une quelconque des revendications 1 à 12 ou stéréoisomères, tautomères, solvates ou sels pharmaceutiquement acceptables de ceux-ci, ou composition pharmaceutique selon la revendication 13, pour une utilisation dans le traitement de la fibrose pathologique.

15. Composé ou stéréoisomères, tautomères, solvates ou sel pharmaceutiquement acceptable de ceux-ci, ou sels pharmaceutiquement acceptables de ceux-ci, ou composition pharmaceutique, pour une utilisation selon la revendication 14 dans lesquels ladite fibrose pathologique est une fibrose hépatique, une fibrose rénale, une fibrose biliaire ou une fibrose pancréatique.

16. Composé selon l'une quelconque des revendications 1 à 12 ou stéréoisomères, tautomères, solvates ou sels pharmaceutiquement acceptables de ceux-ci, ou composition pharmaceutique selon la revendication 13, pour une utilisation dans le traitement de la stéatohépatite non alcoolique (NASH), de la stéatose hépatique non alcoolique (NAFLD), de l'insuffisance rénale chronique, de la néphropathie diabétique, de la cholangite sclérosante primitive (PSC) ou de la cirrhose biliaire primitive (PBC).

17. Composé selon l'une quelconque des revendications 1 à 12 ou stéréoisomères, tautomères, solvates ou sels pharmaceutiquement acceptables de ceux-ci, ou composition pharmaceutique selon la revendication 13, pour une utilisation dans le traitement de la fibrose pulmonaire idiopathique (IPF).
